(19)

**Europäisches Patentamt**
**European Patent Office**
**Office européen des brevets**

(11) **EP 4 495 263 A1**

(12) **EUROPEAN PATENT APPLICATION**

(43) Date of publication:
22.01.2025   Bulletin 2025/04

(21) Application number: 23382740.1

(22) Date of filing: **19.07.2023**

(51) International Patent Classification (IPC):
**C12Q 1/6883** (2018.01)

(52) Cooperative Patent Classification (CPC):
**C12Q 1/6883; C12Q 1/6886;** C12Q 2600/154

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fundación Privada Instituto De Salud Global**
**Barcelona**
**08036 Barcelona (ES)**

(72) Inventors:
• **CÁCERES DOMÍNGUEZ, Alejandro**
**08018 BARCELONA (ES)**
• **GONZÁLEZ RUIZ, Juan Ramón**
**08917 BADALONA (ES)**

(74) Representative: **ZBM Patents - Zea, Barlocci & Markvardsen**
**Rambla de Catalunya, 123**
**08008 Barcelona (ES)**

(54) **METHOD FOR DETERMINING THE X CHROMOSOME REACTIVATION LEVEL OF A FEMALE SUBJECT**

(57)   The present invention provides an *in vitro* method for determining the X chromosome reactivation (X-Ra) level of a female subject, the method comprising the steps of (a) determining the methylation status of at least 10 CpG sites of Table 1 in a DNA-containing sample from the female subject; and (b) calculating the fraction of the CpG sites analyzed in step (a) that are hypomethylated. The invention also provides in vitro methods for determining the biological age of a female subject, diagnosis and/or prognosis of an age-related disease or disorder or an infection, and for determining the gestational age of a fetus.

**EP 4 495 263 A1**

**Description**

**Technical Field**

The invention relates to methods for quantifying the global X chromosome reactivation level of a female subject, in particular to methods based on determining the DNA methylation status. The invention also provides to the use of X chromosome reactivation levels as a biomarker of several aspects of female health and disease.

**Background Art**

The X chromosome in the cells of therian mammals can inactivate. During female development, for instance, one of the two X chromosomes in female cells is randomly inactivated, leading to the formation of Barr bodies, which are visible in cell nuclei.

Chromosome X inactivation (XCI) thus achieves gene dosage compensation with males, who carry only one X chromosome per cell. It is estimated that up to 23% of genes on chromosome X naturally escape inactivation in the inactivated X chromosome (Xi).

XCI is an epigenetic process that occurs early in development and it is thought to be stably maintained throughout the lifespan of females. However, some genes subject to XCI have been described to reactivate under some circumstances. Notably, XCI and its reverse process, chromosome X reactivation (X-Ra), have been mostly measured as an individual property of genes, but not as a global property of the X chromosome, let alone as a global parameter a female subject.

Some attempts have been made in the prior art to provide global values of XCI in particular cell types, for example, by measuring Xist mRNA levels or visualizing Barr bodies. However, these approaches provide poor quantitative values and are generally not reliable, which has hindered the assessment of XCI or X-Ra as a potential marker in women's health. More recently, it was described that chromosome X to autosome (X:A) allelic ratios could be used to measure the XCI status of individual cells. However, this value is not useful for measuring XCI in complex samples including different cell types, such as whole blood or tumor biopsies.

Thus, in spite of the efforts made so far, there is still a need for methods for quantitatively determining the global X-Ra level of a female subject.

**Summary of Invention**

The present inventors have developed a novel method for calculating a biomarker value that provides accurate information regarding numerous aspects of women's health and disease.

As shown in the Examples below, the inventors have devised a simple method for accurately and reliable calculating a quantitative value representing the global level of X chromosome reactivation (X-Ra) in a female subject, the method being based on determining the fraction of hypomethylated CpGs in a particular set of genes of the X chromosome. The method of the invention allows calculating the global X-Ra level in a female subject using complex samples containing different cell types, such as whole blood or tumor biopsies, which is a noteworthy advantage when it comes to the design of practical diagnostics and for testing blood samples collected from other studies.

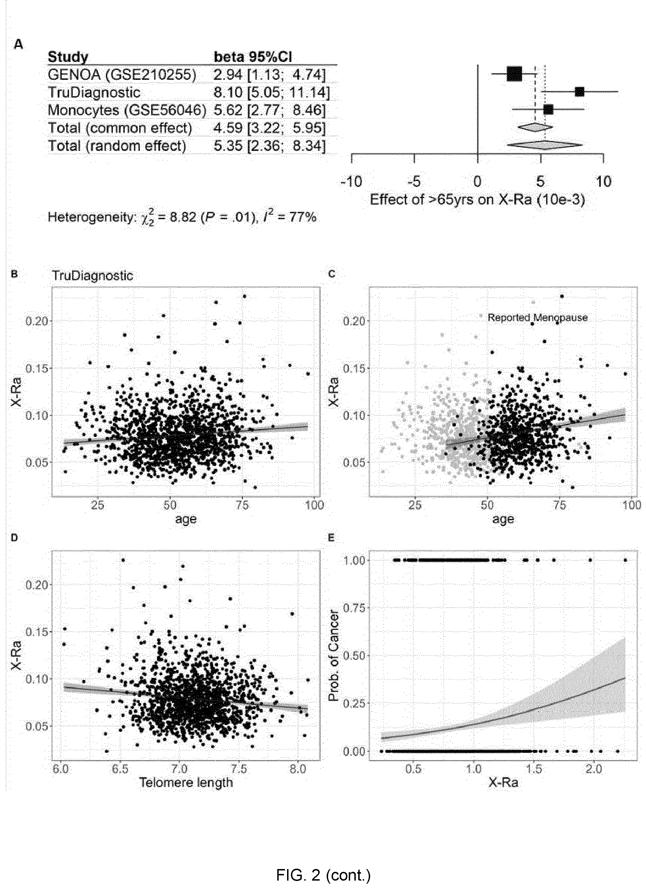

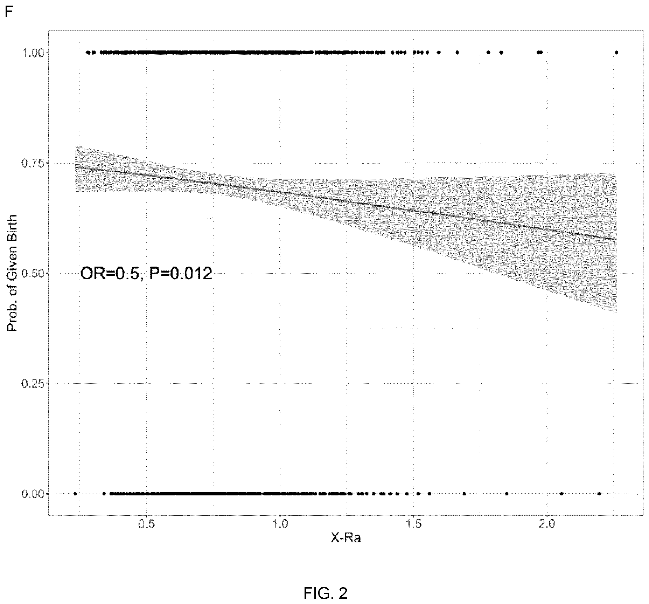

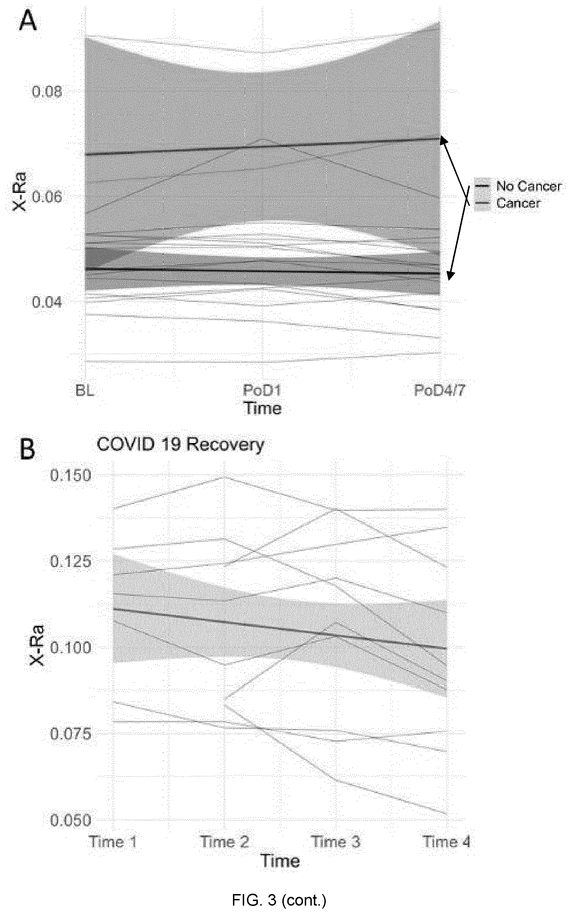

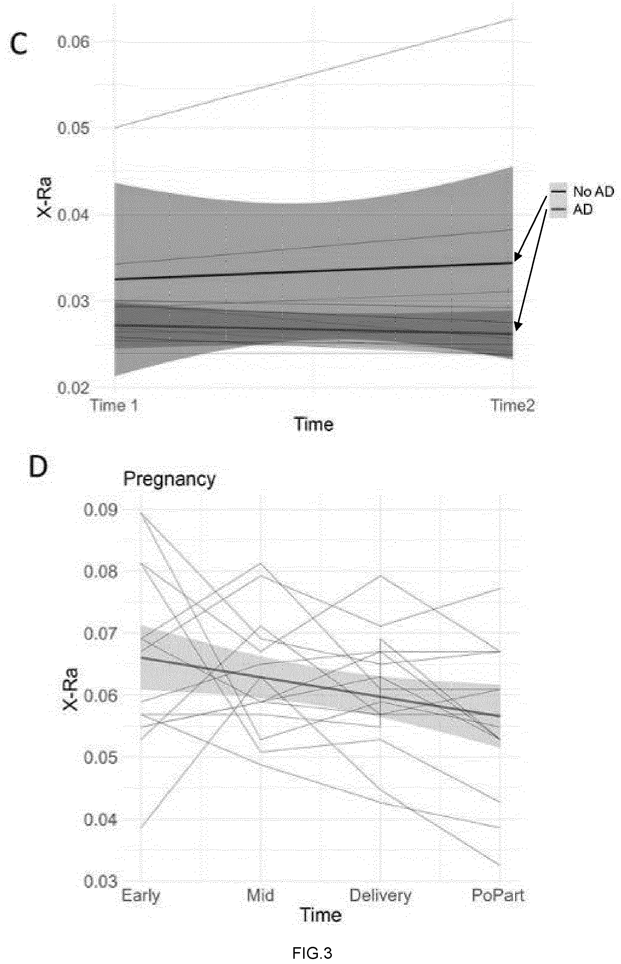

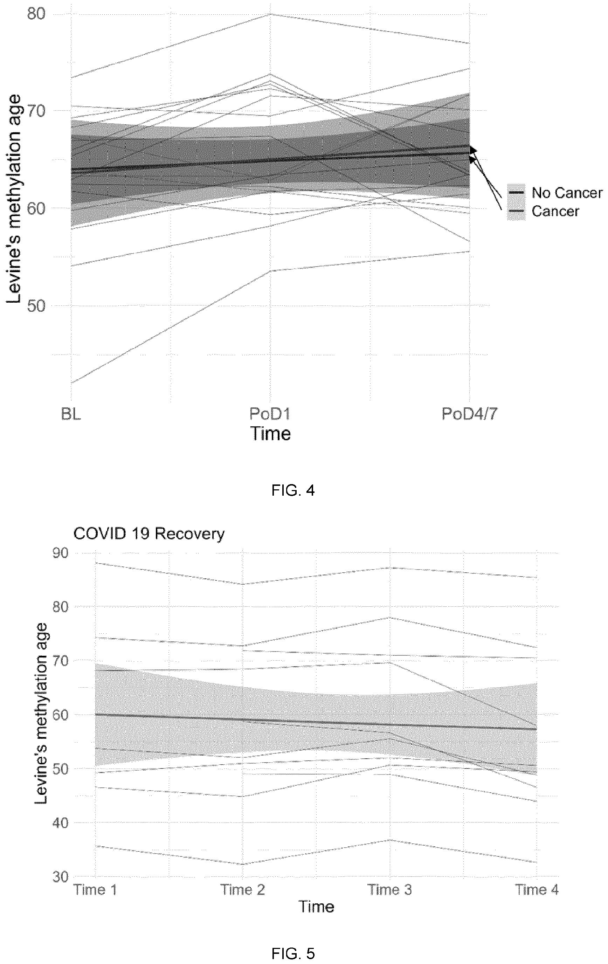

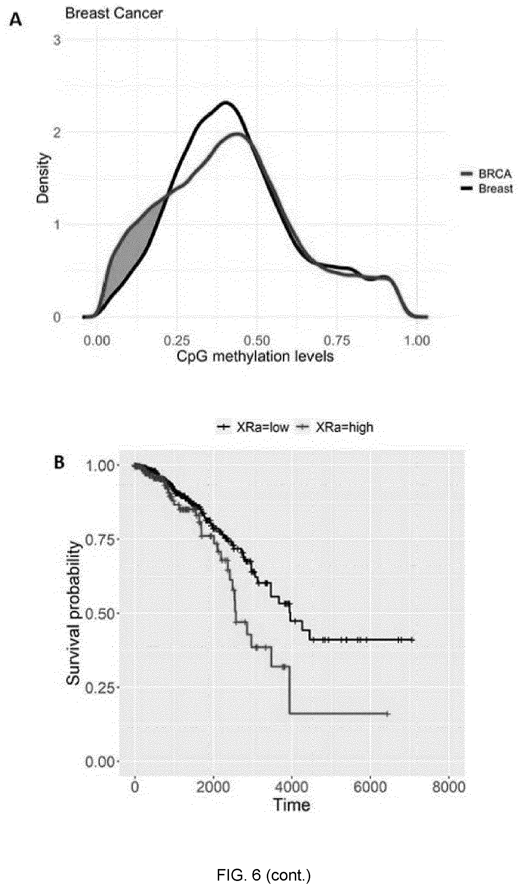

Furthermore, the present inventors have surprisingly found that the global X-Ra biomarker calculated by the method of the invention is strongly associated with female age (Figure 2A-2C) and shorter telomere length (Figure 2D). They have also found that the X-Ra biomarker provides more information than other known aging biomarkers based on general methylation levels (e.g. those disclosed in M. E. Levine, et al. 2018). In particular, X-Ra level is strongly associated with age-associated diseases in women, such as cancer (Figure 2E, 3A, 7A, 7B) or Alzheimer's disease (Figure 3C), while global methylation levels are not (Figure 4-5). X-Ra levels were also found to correlate with low survival of breast cancer patients (Figure 6B), particularly in triple-negative breast cancer patients. The inventors also found that X-Ra levels correlate with recovery from infection, in particular COVID 19 infections (Figure 3B), while global methylation levels did not. In addition to the above, the inventors found that the X-Ra levels correlate with the stage of pregnancy (Figure 3D) and female fertility (Figure 2F).

In conclusion, the results herein provided demonstrate that the global X-Ra level of a female subject calculated by the method of the invention represents a multipurpose biomarker of female health which is highly robust, reliable and informative for a range of applications, including the determination of aging, pregnancy, fertility, and various types of diseases.

Thus, in a first aspect, the invention provides an *in vitro* method for determining the X chromosome reactivation (X-Ra) level of a female subject, the method comprising the steps of: (a) determining the methylation status of at least 10 CpG sites of Table 1 in a DNA-containing sample from the female subject; and (b) calculating the fraction of the CpG sites analyzed in step (a) (i.e., whose methylation status is determined in step (a)) that are hypomethylated.

In a second aspect, the invention provides an *in vitro* method for determining the biological age of a female subject, the method comprising the steps of determining the X chromosome reactivation (X-Ra) level in a DNA-containing sample from the female subject with a method as defined in the first aspect and comparing the determined X-Ra level with a reference value or a range of reference values.

In a third aspect, the invention provides an *in vitro* method for the diagnosis and/or prognosis of an age-related disease or disorder in a female subject, the method comprising the steps of determining the X chromosome reactivation (X-Ra) level in a DNA-containing sample from the female subject with a method as defined in the first aspect and comparing the determined X-Ra level with a reference value or a range of reference values; optionally, wherein the age-related disease or disorder is selected from the group consisting of cancer, neurodegenerative disease, sarcopenia, osteopenia, osteoporosis, arthritis, atherosclerosis, cardiovascular disease, hypertension, cataracts, presbyopia, glaucoma, type 2 diabetes, metabolic syndrome, chronic inflammation, immunosenescence, menopausal symptoms, and combinations thereof.

In a fourth aspect, the invention provides an *in vitro* method for the diagnosis and/or prognosis of an infection in a female subject, the method comprising the steps of determining the X chromosome reactivation (X-Ra) level in a DNA-containing sample from the female subject with a method as defined in the first aspect and comparing the determined X-Ra level with a reference value or a range of reference values.

In a fifth aspect, the invention provides an *in vitro* method for deciding or recommending whether to initiate a therapeutic intervention in a female subject suspicious of suffering an age-related disease or disorder, or an infection, wherein the method comprises the steps of determining the X chromosome reactivation (X-Ra) level in a DNA-containing sample from the female subject with a method as defined in the first aspect and comparing the determined X-Ra level with a reference value or a range of reference values.

In a sixth aspect, the invention provides an *in vitro* method for determining the efficacy of a therapeutic intervention in a female subject already diagnosed of suffering an age-related disease or disorder, or an infection, the method comprising determining the X chromosome reactivation (X-Ra) level in a DNA-containing sample from the female subject with a method as defined in the first aspect before and after starting the therapeutic intervention.

In a seventh aspect, the invention provides an *in vitro* method for determining the ER- status, PR- status, and/or HER2-status of a breast cancer in a female subject, the method comprising the steps of determining the X chromosome reactivation (X-Ra) level in a DNA-containing sample from the female subject with a method as defined in the first aspect and comparing the determined X-Ra level with a reference value or a range of reference values.

In an eighth aspect, the invention provides an *in vitro* method for determining the gestational age of a fetus, the method comprising the steps of determining the X chromosome reactivation (X-Ra) level in a DNA-containing sample from a female subject pregnant with the fetus with a method as defined in the first aspect and comparing the determined X-Ra level with a reference value or a range of reference values.

In a nineth aspect, the invention provides an *in vitro* method for determining the fertility status of a female subject, the method comprising the steps of determining the X chromosome reactivation (X-Ra) level in a DNA-containing sample from the female subject with a method as defined in the first aspect and comparing the determined X-Ra level with a reference value or a range of reference values.

In a tenth aspect, the invention provides the use of means for determining, in a DNA-containing sample from a female subject, the methylation status of at least 10 CpG sites of Table 1 in a method for determining the X chromosome reactivation (X-Ra) level as defined in the first aspect; optionally, wherein the means are a methylation chip array.

In an eleventh aspect, the invention provides the use of the methylation status of at least 10 CpG sites of Table 1 as a biomarker for: determining the X chromosome reactivation (X-Ra) level of a female subject; determining the biological age of a female subject (i.e., as an aging biomarker); determining the gestational age of a fetus; diagnosing and/or prognosing an age-related disease or disorder, or an infection, in a female subject; deciding or recommending whether to initiate a therapeutic intervention in a female subject suspicious of suffering an age-related disease or disorder, or an infection; determining the efficacy of a therapeutic intervention in a female subject already diagnosed of suffering an age-related disease or disorder, or an infection; or determining the fertility status of a female subject.

In a twelfth aspect, the invention provides a computer-implemented method comprising the steps of: (a) receiving information corresponding to the methylation status of at least 10 CpG sites of Table 1 from a DNA-containing sample from a female subject; (b) calculating the fraction of the CpG sites of step (a) that are hypomethylated, which corresponds to the X-Ra level of the female subject.

In a thirteenth aspect, the invention provides a data processing apparatus comprising means for carrying out the method of the twelfth aspect.

In a fourteenth aspect, the invention provides a computer program comprising instructions which, when the program is executed by a computer, cause the computer to carry out the method of the twelfth aspect.

In a fifteenth aspect, the invention provides a computer-readable medium comprising instructions which, when executed by a computer, cause the computer to carry out the method of the twelfth aspect.

In a sixteenth aspect, the invention provides a method of identifying a candidate molecule that modulates the X chromosome reactivation (X-Ra) level, the method comprising the steps of contacting the candidate molecule with a cell from a female subject and determining the X chromosome reactivation (X-Ra) level with a method as defined in the first aspect.

**Brief Description of Drawings**

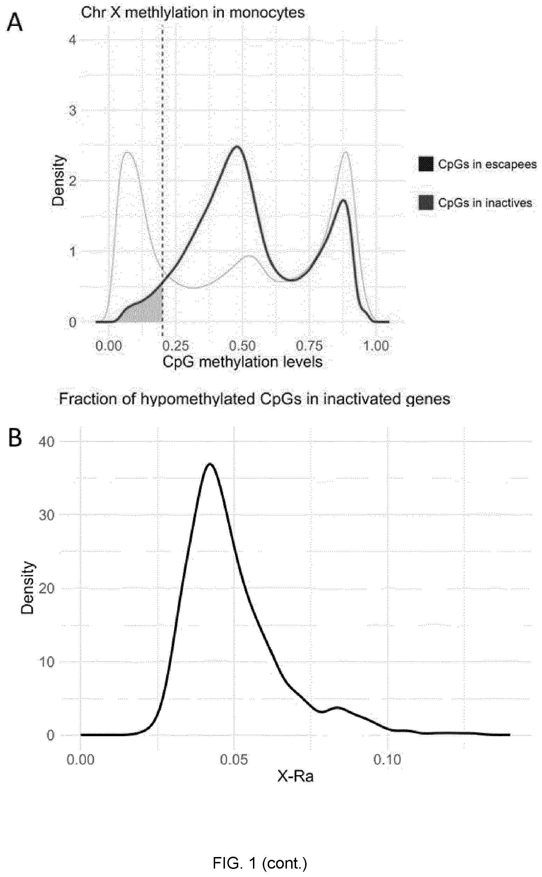

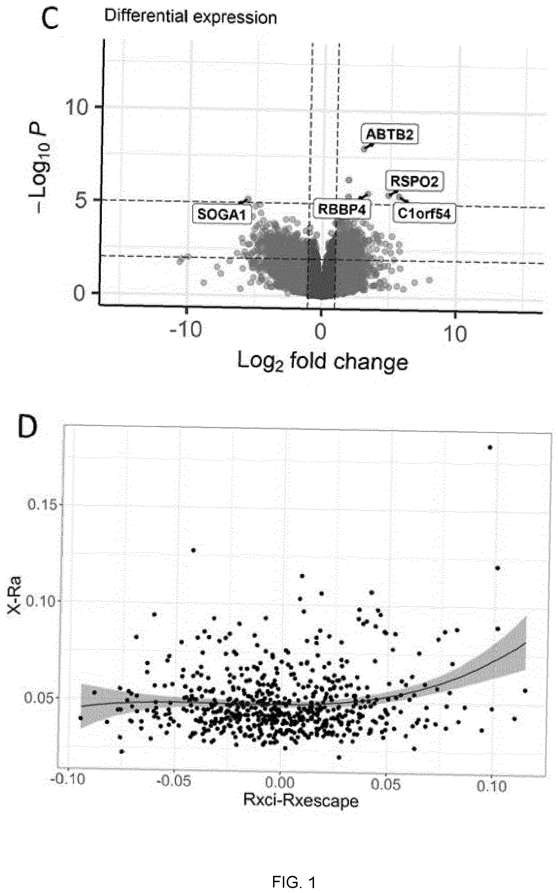

Figure 1. X-Ra in monocytes of adult women. (A) The red line corresponds to methylation beta levels of CpGs from genes inactivated across several tissues. The blue line is of the CpGs from genes that escape XCI. Below 0.2 there is a number of hypomethylated CpGs that defines X-Ra. (B) Distribution of X-Ra across women in the study. X-Ra is the individual's fraction of CpGs in inactivated genes with methylation levels below 0.2, corresponding to the shaded area of a figure similar to (A) for each individual. (C) Differential expression analysis for X-Ra revealing significant genes involved in cancer and histone modification. (D) Grade-3 polynomial association of X-Ra with the difference in RNA transcription output between inactivated genes and escapees (Rxci-Rxescape).

Figure 2. Association of X-Ra in blood with aging and cancer. (A) Meta-analysis for the association between X-Ra and age (>0.65yrs) across three large methylation studies in adult women. X-Ra significantly increases in elder women in each of the studies. (B) In TruDiagnostic DNA biobank, association between X-Ra and continuous age, which increases in women with reported menopause (C). Also, in TruDiagnostic, associations of X-Ra in blood (D) and telomere length and cancer (E). (F) Association between probability of having given birth and X-Ra, showing increasing levels of X-Ra with lower fertility.

Figure 3. Association of X-Ra in blood with aging diseases and transient states of immune upregulation. (A) Longitudinal study (GSE142536) of methylation changes in blood through major surgical intervention. Methylation was assessed at one day before intervention (BL), one day post-operative intervention (PoD1) and 4-7 days post-operative intervention (PoD4/7). X-Ra was significant higher in cancer patients than others. Overall effect of cancer is shown with 95% confidence bands from a mixed model with random effect on subject and adjusted by age. (B) Longitudinal study (GSE62003) of methylation changes in blood of patients with severe COVID19 infection admitted to the UCI (Time 1). All patients recovered from infection and were tested at two following times (Time 2 and Time 3), one just before remission. Additional follow-up (Time 4) was performed after remission. X-Ra was significantly reduced with time of visit. (C) Longitudinal study (GSE62003) of methylation changes in blood in an elderly population. Methylation was assessed two time points and Alzheimer's disease (AD) was diagnosed at base line. X-Ra was significant lower in AD patients than others. (D) Longitudinal study (GSE37722) of pregnancy. Methylation in blood was assayed at early, mid and late pregnancy, and at post post-partum. X-Ra was significantly reduced with progression of pregnancy.

Fig. 4. Longitudinal study (GSE142536) of global methylation changes in blood through major surgical intervention. Methylation was assessed at one day before intervention (BL), one day post-operative intervention (PoD1) and 4-7 days post-operative intervention (PoD4/7). Levine's methylation age (i.e., age determined by global DNA methylation) was not significantly higher in cancer patients than others.

Fig. 5. Longitudinal study (GSE62003) of global methylation changes in blood of patients with severe COVID19 infection admitted to the UCI. Levine's methylation age (i.e., age determined by global DNA methylation) was not significantly associated with time of visit.

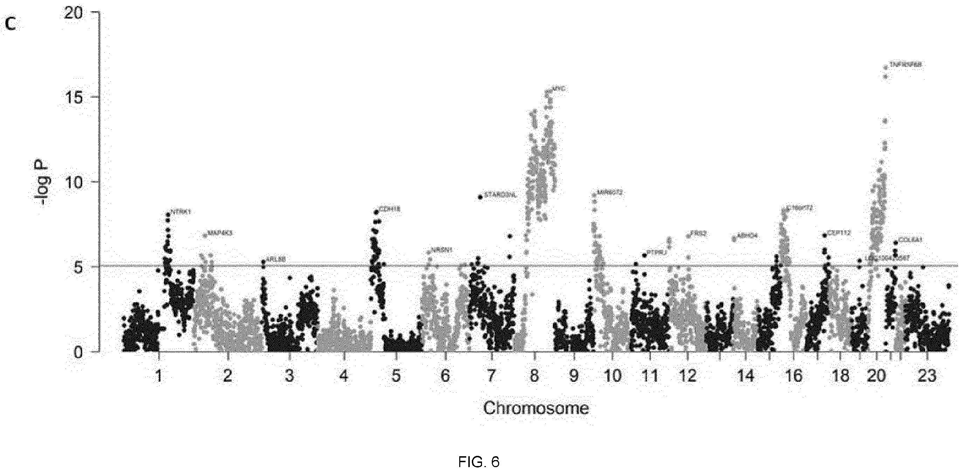

Figure 6. X-Ra as a marker of breast cancer (BRCA). (A) Distribution of CpG methylation levels in inactive and escapee genes in normal breast tissue and breast cancer from TCGA samples. An increased frequency of CpG hypomethylation is observed in inactive genes from tumor relative to normal tissue (shaded area). (B) X-Ra association with BRCA survival. The figure shows the trajectories for the top quartile of X-Ra (red) and the rest (black). (C) Genome-wide association for the frequency of copy number gains in BRCA with X-Ra, in sliding windows of 0.05MB and duplication frequency >0.01. The highest peaks are observed in the *TNFRSF68* and *MYC* loci.

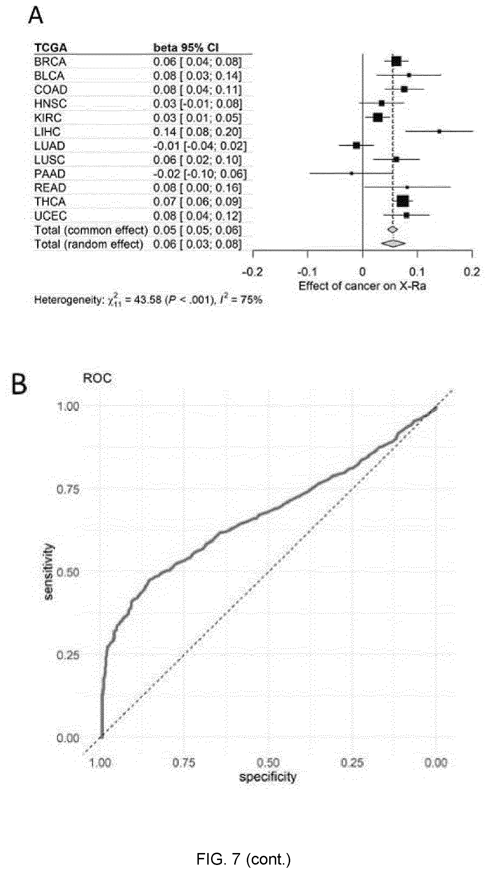

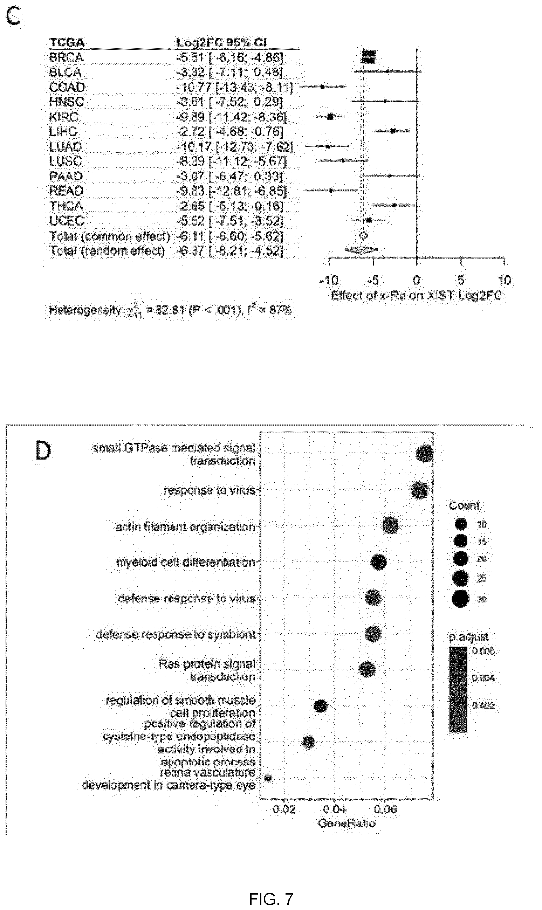

Figure 7. X-Ra as a marker of multiple cancer types. (A) Meta-analysis across 12 different cancer types from the TCGA for the effect of cancer status of samples on the values of X-Ra. Cancer sites included: breast (BRCA), bladder (BLCA), colon (COAD) head and neck (HNSC), kidney (KIRC), liver (LIHC), lung adenocarcinoma (LUAD), lung squamous cell carcinoma (LUSC), pancreas (PAAD), rectum (READ), thyroid (THCA) and uterus (UCEC). (B) Receiver operating curve (ROC) for X-Ra on the cancer status of the TCGA samples. Youden value (red) for X-Ra was at 0.11 with 85.4% specificity. (C) Meta-analysis for the differential expression of *XIST* by X-Ra across the 12 TCGA cancer types. (D) Enrichment analysis for 537 significant genes from the transcriptome-wide meta-analysis of differential expression by X-Ra. Numerous pathways related to immune response and tissue migration were observed.

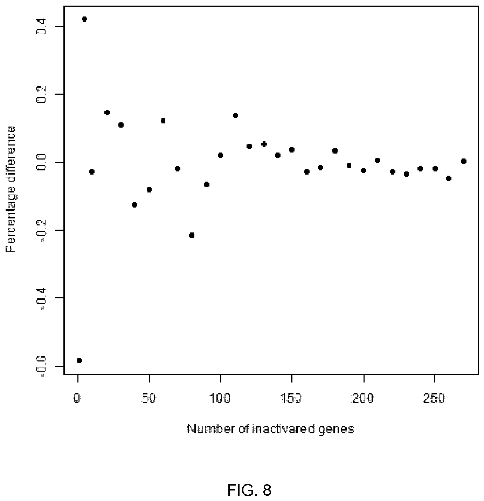

Figure 8. X-Ra calling using reduced numbers of CpGs (genes). The y-axis represents the percentage deviation (average in the whole sample) of the X-Ra value, and the x-axis represents the number of genes tested in each case.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

For purposes of the present invention, any ranges given include both the lower and the upper end-points of the range. Ranges given should be considered approximate, unless specifically stated. The term "about" refers to a deviation of plus/minus 10 %, particularly plus/minus 5 %.

As above indicated, the present invention provides an *in vitro* method for determining the X chromosome reactivation (X-Ra) level of a female subject comprising (a) determining the methylation status of at least 10 CpG sites of Table 1 in a DNA-containing sample from the female subject; and (b) calculating the fraction of the CpG sites analyzed in step (a) that are hypomethylated. The calculated fraction of CpGs that are hypomethylated represents the X-Ra level of the female subject. While increasing the number of CpG sites analyzed increases the accuracy of the method, as shown in the examples below and Figure 8, it is sufficient to only determine the methylation status of a low number of CpGs of Table 1 -e.g. as low as 10 CpGs-to obtain a reliable X-Ra level.

As used herein, "X chromosome inactivation" or "XCI" refers to the transcriptional silencing of one of the two X chromosomes in female somatic cells. "X chromosome reactivation" or "X-Ra" refers to the reverse process of XCI, where the transcription of the inactivated X chromosome is reactivated, for example, by an external process or by XCI deficiency. Unless specifically indicated, both the terms XCI and X-Ra refer to global (intra individual and chromosome-wide) XCI and X-Ra levels, respectively, that is, to the individual's tendency to inactivate or reactivate of the whole X chromosome, and not a single gene, across the cells in a given biological sample. Therefore, the first aspect can also be formulated as an *in vitro* method for determining the global X chromosome reactivation (X-Ra) level of a female subject. As used herein, "X-Ra level" and "X-Ra value" are used interchangeably in the context of X-Ra determination.

As used herein, the "methylation status" of a CpG site refers to the presence or absence of a methylated cytosine, or the ratio of methylated cytosine to unmethylated cytosine, in said CpG site. The term "CpG site" refers to a region of a DNA molecule where a cytosine nucleotide is followed by a guanine nucleotide in the linear sequence of bases along its 5' to 3' direction. "CpG" is a shorthand for 5'-C-phosphate-G-3' that is cytosine and guanine separated by only one phosphate group; phosphate links any two nucleotides together in DNA. The CpG site may be present in a CpG island, a CpG doublet, a promoter, an intron, or an exon of gene. In a particular embodiment, the CpG sites whose methylation status is determined in step (a) are located in promoters. The CpG sites are well-defined genomic regions and they are indexed in database giving to said regions an identification number (ID) as cg_number, according to Illumina®'s CpG Loci Identification wherein flanking sequences regions around the CpG dinucleotide are used to generate unique CpG cluster IDs (cg_number). The location of all CpG sites in human chromosome X provided herein are according to the chromosome map and sequence entries of database UCSC Genome Browser on Human February 2009, GRCh37/hg19 assembly of the University of California Santa Cruz (UCSC).The methylation status of DNA, and in particular of any CpG site, can optionally be represented or indicated by a "methylation value" or "methylation level." A methylation value, score or level can be generated, for example, by quantifying the methylation in a cytosine using, for example a beta value ranging from 0 to 1, as explained below.

The term "polynucleotide" is interchangeably used with "nucleic acid" and refers to a polymer of nucleotides, either ribonucleotides or deoxyribonucleotides. A polynucleotide formed by ribonucleotides may be referred to as "RNA polynucleotide", "ribonucleic acid" or simply "RNA"; and a polynucleotide formed by deoxyribonucleotides may be referred to as "DNA polynucleotide", "deoxyribonucleic acid" or simply "DNA". As used herein, the term "genomic DNA" refers to the chromosomal DNA, particularly to human chromosomal DNA.

In a particular embodiment of the first aspect, the method consists of the steps of:

(a) determining the methylation status of at least 10 CpG sites of Table 1 in a DNA-containing sample from the female subject; and

(b) calculating the fraction of the CpG sites analyzed in step (a) that are hypomethylated.

In a particular embodiment of the above aspects, the female subject is a mammal, particularly a human or a non-human mammal. In another embodiment, the female subject is a non-human primate. In some embodiments, the female subject is a rodent. In some embodiments, the female subject is a sheep, a goat, cattle, a cat, or a dog. The female subject may be at any stage of development.

In a particular embodiment of the first aspect, the method comprises determining the methylation status of at least 10 CpG sites of Table 1, optionally wherein the CpG sites are in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1. In a more particular embodiment, the method comprises determining the methylation status of at least 20, at least 30, at least 40, at least 50, at least 100, at least 150, at least 200, at least 250, at least 300, at least 350, at least 400, at least 450, or 498 CpG sites of Table 1, optionally wherein the CpG sites are in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1.

In a particular embodiment of the first aspect, the method comprises determining the methylation status of at least one CpG site in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1.

In a particular embodiment of the first aspect, the method comprises determining the methylation status of a least 10 CpG sites of Table 2, optionally wherein the CpG sites are in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2. In a more particular embodiment, the method comprises determining the methylation status of a least 10, at least 20, at least 30, at least 40, at least 50, at least 100, at least 150, at least 200, at least 250, at least 300, at least 350, at least 400, at least 450, at least 500, at least 1000, at least 1500, at least 2000, at least 2500, at least 3000, at least 3500, at least 4000, at least 4500, or 4758 CpG sites of Table 2, optionally wherein the CpG sites are in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2.

In a particular embodiment of the first aspect, the method comprises determining the methylation status of at least one CpG site in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2.

In a particular embodiment of the first aspect, in step (b) the fraction of the CpG sites is the weighted fraction of the CpG sites analyzed in step (a) that are hypomethylated.

The skilled in the art would appreciate that there are several methods known in the art, that belong to the common general knowledge, to determine the methylation status of CpG sites, which range from commercial array platforms (e.g. from Illumina) to sequencing approaches of individual genes, which can all be used in the methods of the invention.

Thus, in a particular embodiment of the first aspect, the methylation status of the CpGs is determined by a method selected from the group consisting of methylation chip array (e.g. Illumina's Infimum HumanMethylation27 BeadChip array, Illumina's Infimum HumanMethylation450 BeadChip array, or Illumina's Infimum MethylationEPIC), bisulfite sequencing (e.g. bisulfite pyrosequencing), next-generation bisulfite sequencing (NGBS), methylated DNA immunoprecipitation (MeDIP), enzymatic methyl sequencing (EM-Seq), HELP assay, mass spectrometry, HPLC, ChIP-on-chip assay, High Resolution Melt Analysis (HRM or HRMA), molecular break light assay for DNA adenine methyltransferase activity, methyl sensitive Southern blotting, and Methylation Specific PCR (MSP).

In general, methods for the detection of DNA methylation first involve converting the DNA to be analyzed so that the unmethylated cytosine is converted to uracil. In one embodiment, a chemical reagent that selectively modifies either the methylated or non-methylated form of CpG dinucleotide motifs may be used. Suitable chemical reagents include hydrazine and bisulphite ions and the like. For example, isolated DNA can be treated with sodium bisulfite (NaHS03) which converts unmethylated cytosine to uracil, while methylated cytosines are maintained. Without wishing to be bound by a theory, it is understood that sodium bisulfite reacts readily with the 5,6-double bond of cytosine, but poorly with methylated cytosine. Cytosine reacts with the bisulfite ion to form a sulfonated cytosine reaction intermediate that is susceptible to deamination, giving rise to a sulfonated uracil. The sulfonated group can be removed under alkaline conditions, resulting in the formation of uracil. The nucleotide conversion results in a change in the sequence of the original DNA. It is general knowledge that the resulting uracil has the base pairing behavior of thymine, which differs from cytosine base pairing behavior. To that end, uracil is recognized as a thymine by DNA polymerase. Therefore, after PCR or sequencing, the resultant product contains cytosine only at the position where 5-methylcytosine occurs in the starting template DNA. This makes the discrimination between unmethylated and methylated cytosine possible by multiple methods.

There are numerous commercial methylation chip arrays that can be used in the methods of the invention, for example Illumina's Infimum HumanMethylation450 BeadChip array or Illumina's Infimum HumanMethylation27 Bead-Chip array. These arrays generate fluorescent signals that are scanned, and the intensities of the signals produced by the unmethylated and methylated bead types are measured. Software is generally used to record DNA methylation values, described as "beta values," for each locus in each sample. DNA methylation beta values are continuous variables between 0 and 1, representing the ratio of the intensity of the methylated bead type to the combined locus intensity. Further details describing the Illumina's Infimum HumanMethylation BeadChip arrays and assay platform are described in, e.g., Morris TJ et al., " Analysis pipelines and packages for Infinium HumanMethylation450 BeadChip (450k) data", Methods. 2015 Jan 15; 72: 3-8.

Methylation-Specific PCR (MSP), is based on a chemical reaction of sodium bisulfite with DNA, converting unmethylated cytosines of CpG dinucleotides to uracil (UpG), followed by traditional PCR. Methylated cytosines will not be converted by the sodium bisulfite, and specific nucleotide primers designed to overlap with the CpG site of interest will allow determining the methylation status as methylated or unmethylated, based on the amount of PCR product formed. The HELP assay is based on the differential ability of restriction enzymes to recognize and cleave methylated and unmethylated CpG DNA sites. Furthermore, ChIP-on-chip assays, based on the ability of commercially prepared antibodies to bind to DNA methylation-associated proteins like MCP2, can be used to determine the methylation status. Also, restriction landmark genomic scanning, also based upon differential recognition of methylated and unmethylated CpG sites by restriction enzymes can be used. Methylated DNA immunoprecipitation (MeDIP), analogous to chromatin immunoprecipitation, can be used to isolate methylated DNA fragments for input into DNA detection methods such as DNA microarrays (MeDIP-chip) or DNA sequencing (MeDIP-seq). The unmethylated DNA is not precipitated. Alternatively, molecular break light assay for DNA adenine methyltransferase activity can be used. This is an assay that uses the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase. Pyrosequencing of bisulfite treated DNA is a sequencing of an amplicon made by a normal forward primer but a biotinylated reverse primer to PCR the gene of choice. The Pyrosequencer then analyses the sample by denaturing the DNA and adding one nucleotide at a time to the mix according to a sequence given by the user. If there is a mismatch, it is recorded and the percentage of DNA for which the mismatch is present is noted. This gives the user a percentage methylation per CpG island. Next generation genome bisulfite sequencing, also known as NGBS, is a genome-wide analysis of DNA methylation. It is based on the sodium bisulfite conversion of genomic DNA, which is then sequencing on a Next-Generation Sequencing (NGS) platform. The sequences obtained are then re-aligned to the reference genome to determine methylation states of CpG dinucleotides based on mismatches resulting from the conversion of unmethylated cytosines into uracil.

In some embodiments, the hypomethylated CpG sites are the CpG sites with a beta value equal to or lower than 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, or 0.2, particularly where the methylation status is determined by a methylation chip array (such as the Illumina's Infimum HumanMethylation450 BeadChip array or Infimum HumanMethylation27 BeadChip array). In a more particular embodiment, the hypomethylated CpG sites are the CpG sites with a beta value equal to or lower than about 0.2, where the methylation status is determined by a methylation chip array.

In some embodiments, the hypomethylated CpG sites are the CpG sites with a methylation equal to or lower than 20%, 19%, 18%, 17%, 16%, 15%, 14%, 13%, 12%, 11%, 10%, 9%, 8%, 7%, 6%, 5%, 4%, 3%, 2%, or, 1%, particularly where the methylation status is determined by bisulfite sequencing. In a more particular embodiment, the hypomethylated CpG sites are the CpG sites with a methylation equal to or lower than about 20%, where the methylation status is determined by bisulfite sequencing.

In some embodiments, the hypomethylated CpG sites are the CpG sites with a methylation equal to or lower than 5%, 4%, 3%, 2%, or, 1%, particularly where the methylation status is determined by next-generation bisulfite sequencing. In a more particular embodiment, the hypomethylated CpG sites are the CpG sites with a methylation equal to or lower than about 5%, where the methylation status is determined by next-generation bisulfite sequencing.

The skilled in the art would know how to calculate the beta value for each CpG site from the data obtained with a methylation chip array following routinary methods known in the art, for example those disclosed in the Examples below. In a particular embodiment, the beta value of each CpG site may be calculated with the formula (I):

$$Beta = 2^M/(2^M+1) \quad (I)$$

where M represents methylation data provided by a methylation chip array.

In a particular embodiment of the first aspect, the DNA-containing sample is a genomic DNA-containing sample, optionally selected from the group consisting of whole blood, plasma, serum, urine, saliva, sputum, nasal mucous, respiratory lavage, tears, chorionic villi, amniotic fluid, cerebrospinal fluid, spinal fluid, lavage fluid, stool, lymphatic fluid, bile, sweat, breast milk, breast fluid, cultured cells, tissue biopsy (e.g. tumor biopsy), tissue swabs, cervical cytology, synovial fluid, and combinations thereof. In a more particular embodiment, the whole blood is peripheral whole blood.

In one embodiment of the first aspect, the method further comprises determining the biological age of the female subject by comparing the determined X-Ra level with a reference value or a range of reference values.

In one embodiment of the first aspect, the method further comprises diagnosing and/or prognosing an age-related disease or disorder in the female subject by comparing the determined X-Ra level with a reference value or a range of reference values.

In one embodiment of the first aspect, the method further comprises diagnosing and/or prognosing an infection in the female subject by comparing the determined X-Ra level with a reference value or a range of reference values.

In one embodiment of the first aspect, the method further comprises determining the gestational age of a fetus by comparing the determined X-Ra level with a reference value or a range of reference values.

In one embodiment of the first aspect, the method further comprises determining the fertility status of the female subject by comparing the determined X-Ra level with a reference value or a range of reference values.

In one embodiment of the first aspect, the method further comprises reporting the X-Ra level of the female subject, optionally wherein said reporting comprises preparing a written or electronic report

In one embodiment of the first aspect, the method is with the proviso that the method does not comprise determining the methylation status of any CpG site in an autosome (non-sexual chromosome) and/or a Y chromosome. In other words, the method is with the proviso that it does not comprise determining the methylation status of any CpG site outside the X chromosome.

As above indicated, in a second aspect the invention provides an *in vitro* method for determining the biological age of a female subject, the method comprising the steps of determining the X chromosome reactivation (X-Ra) level in a DNA-containing sample from the female subject with a method as defined in the first aspect and comparing the determined X-Ra level with a reference value or a range of reference values. The skilled person would appreciate that the determination of the biological age of the female subject is based on the determined X-Ra level.

As used herein, "biological age" refers to the physiological state of a female subject, or of a tissue from said subject, relative to the physiological changes that occur throughout the subject's lifespan. Therefore, the skilled person would appreciate that to determine the biological age of a subject means to determine the degree of aging of said subject. In contrast, "chronological age" refers to the age of a subject as measured by a time scale, such as months and years. If the subject has premature aging, the subject's biological age is greater than their chronological age. If a subject has delayed aging, the subject's biological age is less than their chronological age.

In the present invention, the term "reference value or a range of reference values" referred to in the methods of the invention is to be understood as a predefined value or range of values of X-Ra derived from a control sample or group of samples. The control samples are taken from a subject or group of subjects wherein the biological age; gestational age of a fetus; fertility; or presence, absence, stage, histological subtype or grade, or course of a disease; has been properly performed previously. This value is used as a threshold to discriminate the female subjects assayed. In the case of disease prognosis or diagnosis, the subject or subjects from whom the reference value is derived may include subject/s wherein the condition is absent, subject/s wherein the condition is present, or both. The skilled person in the art, making use of the general knowledge, is able to choose the subject or group of subjects more adequate for obtaining the reference value for each of the methods of the present invention. Methods for obtaining the reference value from the group of subjects selected are well-known in the state of the art (Burtis C. A. et al., 2008, Chapter 14, section "Statistical Treatment of Reference Values"). In a particular case, "reference value" is a cut-off value defined by means of a conventional ROC analysis (Receiver Operating Characteristic analysis). As the skilled person will appreciate, optimal cut-off value will be defined according to the particular applications of the method - e.g., purpose, target population for the diagnosis or prognosis, balance between specificity and sensibility, etc. The term "reference value", as used herein, can be an absolute value; a relative value; a value that has an upper or a lower limit; a range of values; an average value; a median value, a mean value, or a value as compared to a particular control or baseline value.

In a particular embodiment of the second aspect, the method further comprises the step of determining the age of the female subject based on the determined X-Ra level, optionally by applying a statistical prediction algorithm.

In a particular embodiment of the second aspect, the reference value or a range of reference values are derived from one or more female subjects of known ages.

In a particular embodiment of the second aspect, the method further comprises reporting the biological age of the female subject, optionally wherein said reporting comprises preparing a written or electronic report.

As above explained, in a third aspect the invention provides an *in vitro* method for the diagnosis and/or prognosis of an age-related disease or disorder in a female subject, the method comprising the steps of determining the X chromosome reactivation (X-Ra) level in a DNA-containing sample from the female subject with a method as defined in the first aspect and comparing the determined X-Ra level with a reference value or a range of reference values.

In a particular embodiment of the second aspect, the method further comprises the step of diagnosing and/or prognosing the age-related disease or disorder based on the determined X-Ra level, optionally by applying a statistical prediction algorithm.

In a particular embodiment of the second aspect, the reference value or a range of reference values are derived from one or more female subjects where the age-related disease or disorder is absent. In another embodiment, the reference value or a range of reference values are derived from one or more female subjects where the age-related disease or disorder is present.

As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification. "Prognosis" as used herein refers to the prediction of the probable progression and outcome of a disease, including the death of the subject.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

As used herein, "age-related disease or disorder" refers to diseases and disorders often associated with aging, that is, diseases or disorders in which aging is a major risk factor, for example cancers (e.g., gliomas, leukemia, lymphoma, breast cancer, prostate cancer, lung cancer, etc.) and neurodegenerative diseases (e.g., Parkinson's disease, Alzheimer's disease, Huntington's disease, dementia, etc.).

Thus, in a particular embodiment of the third aspect, the age-related disease or disorder is selected from the group consisting of cancer, neurodegenerative disease (e.g. Alzheimer's disease, dementia), sarcopenia, osteopenia, osteoporosis, arthritis, atherosclerosis, cardiovascular disease, hypertension, cataracts, presbyopia, glaucoma, type 2 diabetes, metabolic syndrome, chronic inflammation, immunosenescence, menopausal symptoms, and combinations thereof.

In a particular embodiment of the third aspect, if the determined X-Ra level is upper or lower than the reference value, this is indicative that the female subject suffers from an age-related disease or disorder. In a more particular embodiment, if the determined X-Ra level is upper than the reference value derived from one or more female subjects where the age-related disease or disorder is absent, this is indicative that the female subject suffers from an age-related disease or disorder, particularly cancer. In another embodiment, wherein the determined X-Ra level is lower than the reference value derived from one or more female subjects where the age-related disease or disorder is present, this is indicative that the female subject suffers from an age-related disease or disorder, particularly Alzheimer's disease.

In a particular embodiment of the third aspect, if the determined X-Ra level is upper or lower than the reference value, this is indicative that the female subject has a bad prognosis. In a more particular embodiment, if the determined X-Ra level is upper than the reference value derived from one or more female subjects where the age-related disease or disorder is absent, this is indicative that the female subject has a bad prognosis. In another embodiment, if the determined X-Ra level is lower than the reference value derived from one or more female subjects where the age-related disease or disorder is present, this is indicative that the female subject has a bad prognosis.

In a particular embodiment of the third aspect, the cancer is breast cancer; particularly triple-negative breast cancer (TNBC). As used herein, "triple-negative breast cancer" or "TNBC" refers to a specific subtype of breast cancer that does not express estrogen receptor (ER), progesterone receptor (PR), and human epidermal growth factor receptor 2 (HER-2).

In a particular embodiment of the third aspect, the method further comprises reporting the diagnosis or prognosis of the age-related disease or disorder, optionally wherein said reporting comprises preparing a written or electronic report.

In a particular embodiment of the third aspect, the method further comprises treating the female subject with an anticancer drug.

As above mentioned, in a fourth aspect the invention provides an *in vitro* method for the diagnosis and/or prognosis of an infection in a female subject, the method comprising the steps of determining the X chromosome reactivation (X-Ra) level in a DNA-containing sample from the female subject with a method as defined in the first aspect and comparing the determined X-Ra level with a reference value or a range of reference values.

In a particular embodiment of the fourth aspect, the method further comprises the step of diagnosing and/or prognosing the infection based on the determined X-Ra level, optionally by applying a statistical prediction algorithm.

In a particular embodiment of the fourth aspect, the reference value or a range of reference values are derived from one or more female subjects where the infection is absent. In another embodiment, the reference value or a range of reference values are derived from one or more female subjects where the infection is present.

In a particular embodiment of the fourth aspect, the method further comprises reporting the diagnosis or prognosis of the infection, optionally wherein said reporting comprises preparing a written or electronic report.

In a particular embodiment of the fourth aspect, if the determined X-Ra level is upper or lower than the reference value, this is indicative that the female subject suffers from an infection. In a particular embodiment of the fourth aspect, if the determined X-Ra level is upper or lower than the reference value, this is indicative that the female subject has a bad prognosis.

As above indicated, the invention also provides in a fifth and sixth aspects, a method for deciding or recommending whether to initiate a therapeutic intervention, and a method for determining the efficacy of a therapeutic intervention, respectively. All embodiments above disclosed for the third and fourth aspects of the invention are also meant to apply to the fifth and sixth aspects of the invention.

In a particular embodiment of the fifth aspect, if the determined X-Ra level is the same or different from the reference value, this is indicative that the female subject has to start a therapeutic intervention.

In a particular embodiment of the sixth aspect, if the X-Ra level determined once started the intervention is different than the level determined before starting the intervention, and optionally the same or different than the reference value, it is indicative that the therapeutic intervention is effective.

As above indicated, in a nineth aspect, the invention provides an *in vitro* method for determining the fertility status of a female subject. In a particular embodiment, if the determined X-Ra level is lower than the reference value, this is indicative that the female subject has a low fertility. In a particular embodiment, if the determined X-Ra level is upper than the reference value, this is indicative that the female subject has a high fertility.

The invention also provides, in a tenth aspect, the use of means for determining, in a DNA-containing sample from a female subject, the methylation status of at least 10 CpG sites of Table 1 in a method for determining the X chromosome reactivation (X-Ra) level as defined in the first aspect.

In a particular embodiment of the tenth aspect, the means are for determining the methylation status of:

- at least 10, at least 20, at least 30, at least 40, at least 50, at least 100, at least 150, at least 200, at least 250, at least 300, at least 350, at least 400, at least 450, or 498 CpG sites of Table 1, optionally wherein the CpG sites are in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1;
- at least one CpG site in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1;
- a least 10, at least 20, at least 30, at least 40, at least 50, at least 100, at least 150, at least 200, at least 250, at least 300, at least 350, at least 400, at least 450, at least 500, at least 1000, at least 1500, at least 2000, at least 2500, at least 3000, at least 3500, at least 4000, at least 4500, or 4758 CpG sites of Table 2; optionally wherein the CpG sites are in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2; or
- at least one CpG site in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2.

These means may be, in a particular embodiment, DNA oligonucleotides that are complementary to a sequence comprising the cytosine of each CpG and producing a differential signal if the cytosine in a determined position is methylated or unmethylated. In a more particular embodiment, the differential signal is selected from fluorescence signal, chemiluminescence signal and combinations thereof. This signal is mainly the result of the emission of either fluorescence or chemiluminescence by a compound associated, in particular, covalently bonded, to the oligonucleotides complementary to the sequences to be detected. Alternatively, in another embodiment, the means comprise one or more DNA oligonucleotides that are complementary to a sequence comprising the methylated cytosine of each CpG site, and one or more DNA oligonucleotides that are complementary to a sequence comprising the unmethylated cytosine of each CpG site.

In another particular embodiment of the use of these means, the DNA oligonucleotides are provided together with other reagents, such as buffers, fluorescent or chemiluminescent labels, and instructions to use them in the determination of the methylation status of the one or more CpG sites of interest.

In a more particular embodiment of the tenth aspect, the means are a methylation chip array, in particular Illumina's Infimum HumanMethylation27 BeadChip array or Illumina's Infimum HumanMethylation450 BeadChip array.

In one embodiment, the means form part of a kit which further comprises instructions for its use. The term "kit", as used herein, refers to a product containing the different reagents (or reagent means) necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes. In a particular embodiment, the instructions in the kit are for the simultaneous, sequential or separate use of the different components which are in the kit. Said instructions can be in the form of printed material or in the form of an electronic support capable of storing instructions susceptible of being read or understood, such as, for example, electronic storage media (e.g. pendrive), or optical media (e.g. CD-ROM, DVD), or audio materials. Additionally, or alternatively, the media can contain internet addresses that provide said instructions.

As above explained, in an eleventh aspect the invention provides the use of the methylation status of at least 10 CpG sites of Table 1 as a biomarker for several purposes.

All the embodiments of the first aspect, in particular those related to the different subgroups of CpG sites, are also meant to apply to the eleventh aspect.

In a particular embodiment, the eleventh aspect provides the use of the methylation status of:

- at least 10, at least 20, at least 30, at least 40, at least 50, at least 100, at least 150, at least 200, at least 250, at least 300, at least 350, at least 400, at least 450, or 498 CpG sites of Table 1, optionally wherein the CpG sites are in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1;
- at least one CpG site in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1;
- a least 10, at least 20, at least 30, at least 40, at least 50, at least 100, at least 150, at least 200, at least 250, at least 300, at least 350, at least 400, at least 450, at least 500, at least 1000, at least 1500, at least 2000, at least 2500, at least 3000, at least 3500, at least 4000, at least 4500, or 4758 CpG sites of Table 2; optionally wherein the CpG sites are in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2; or
- at least one CpG site in at least 2, at least 5, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, at least 300, at least 350, or 362 genes of Table 2; for any one of the purposes above indicated.

As indicated above, the invention provides in a twelfth aspect a computer-implemented method comprising the steps of (a) receiving information corresponding to the methylation status of at least 10 CpG sites of Table 1 in a DNA-containing sample from the female subject; (b) calculating the fraction of the CpG sites analyzed in step (a) that are hypomethylated, which corresponds to the X-Ra level of the female subject.

In a particular embodiment of the twelfth aspect, the method further comprises:

- determining the biological age of the female subject by comparing the determined X-Ra level with a reference value or a range of reference values, and, optionally, applying a statistical prediction algorithm;
- diagnosing and/or prognosing an age-related disease or disorder, or an infection, in the female subject by comparing the determined X-Ra level with a reference value or a range of reference values and, optionally, applying a statistical prediction algorithm;
- determining the gestational age of a fetus by comparing the determined X-Ra level with a reference value or a range of reference values and, optionally, applying a statistical prediction algorithm; and/or

- determining the fertility status of the female subject by comparing the determined X-Ra level with a reference value or a range of reference values and, optionally, applying a statistical prediction algorithm.

In a particular embodiment of the twelfth aspect, the method further comprising collecting the information regarding the X-Ra level of the female subject and saving the information in a data carrier and/or reporting the information.

As above disclosed, in a sixteenth aspect the invention provides a method of identifying a candidate molecule that modulates the X chromosome reactivation (X-Ra) level, the method comprising the steps of contacting the candidate molecule with a cell from a female subject and determining the X chromosome reactivation (X-Ra) level with a method as defined in the first aspect. The skilled person would understand that the incubation time and compound concentration required will vary depending on several factors and these can be adjusted by routine experimentation.

In a particular embodiment of the sixteenth aspect, the method is an *in vitro* method. I another embodiment, the candidate molecule increases X-Ra level. In yet another embodiment, the candidate molecule decreases X-Ra level.

In a particular embodiment of the sixteenth aspect, the method further comprises comparing the determined X-Ra level with a reference value or a range of reference values. In a more particular embodiment, the reference value corresponds to the X-Ra level of the cells before contacting the candidate molecule.

In a particular embodiment of the sixteenth aspect, an increased X-Ra level identifies the candidate molecule as a molecule that increases X-Ra (i.e., inhibits XCI), and a decreased X-Ra level identifies the candidate molecule as a molecule that decreases X-Ra (i.e., promotes XCI).

In a particular embodiment of the sixteenth aspect, the cell is a somatic cell.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

**Examples**

**1. Materials**

Monocytes study

The data was obtained from the Gene Expression Omnibus (https://www.ncbi.nlm.nih.gov/geo/) with the reference GSE56046. It is a large population study with gene expression (Illumina HumanHT-12 v4 Expression) and methylation (Illumina HumanMethylation450) data in purified human monocytes from blood. The data included covariates such as race, gender, study site, and sample contamination with B-cells, T-cells, natural killer cells, and neutrophils. As race, gender, and study site were encoded in a categorical variable with 18 different categories, sex was inferred from the mean expression of chromosome Y divided by the mean expression from the whole genome (Ry). Individuals from categories with Ry values less than -0.35 were classified as females. The classification was validated by the average methylation from inactive genes from chromosome X, where categories with values less than -0.5 were classified as females, thus completely matching those classified by Ry. Data was then analyzed for 605 females with a mean age of 60 years (SD=9.47).

The methylation data were obtained in M values and transformed into Beta values using the formula:

$$Beta = 2^M/(2^M+1)$$

A total of 11,648 CpG sites from chromosome X were analyzed. The association between X-Ra level and age was assessed adjusting by leukocyte content and well. For gene expression data, 47,308 transcripts were analyzed and detected surrogate variables with SVA, adjusting by age, cohort and contamination with B-cells, T-cells, natural killer cells, and neutrophils.

GENOA study

The data was obtained from Gene Expression Omnibus (https://www.ncbi.nlm.nih.gov/geo/) with reference GSE210255. The Genetic Epidemiology Network of Arteriopathy (GENOA) study is a large community-based study of hypertensive siblings, including African Americans from Jackson, USA. Genomic DNA was extracted from stored peripheral blood leukocytes collected using AutoGen FlexStar (AutoGen, Holliston, MA). Bisulfite conversion was performed with the EZ DNA Methylation Kit (Zymo Research, Irvine, CA), and methylation was assessed using the Illumina Infinium HumanMethylationEPIC BeadChip. After quality control and filtering, a total of 1,394 samples were included in the analysis. The analysis was focused on female samples (n=986) with a mean age of 57.2 years (SD=10.3). A total of 10,125 CpG sites on chromosome X were analyzed and the association between X-Ra and age, adjusting for plate, was assessed.

TruDiagnostic DNA biobank

The TruDiagnostic DNA biobank is a USA population-based cohort aged between 13 and 97 years old. The biobank includes 3,590 individuals recruited between October 2020 and February 2022, who have chosen TruDiagnostic for DNA methylation analysis for biological aging. Clinical data was collected, including anthropometric measures and diagnosis of common diseases. Methylation data was analyzed from 1,414 females from 3,259 individuals who passed quality control.

For the DNA methylation analysis, DNA was extracted from peripheral whole blood. The Infinium HumanMethylationEPIC BeadChip was used for DNA methylation assessment following the manufacturer's protocol. Several quality controls and functional normalizations were performed using the meffil package, resulting in 745,150 probes, as described in detail elsewhere (Carreras-Gallo, A. et al, "The early-life exposome modulates the effect of polymorphic inversions on DNA methylation". Commun. Biol. 2022 51 5, 1-13 (2022)). CpG sites were annotated to genes using the EPIC Illumina annotation ilm10b4.hg19. Blood cell types were estimated using the blood gse35069 reference panel from the meffil package. A total of 4,471 CpG sites from chromosome X were analyzed. The association of X-Ra) with age adjusting for leukocyte content and slide was then assessed. Associations between cancer diagnosis and X-Ra, and the interaction of X-Ra with height, were adjusted for age, leukocyte content, slide, and in the case of the interaction, also X-Ra and height.

Longitudinal data

Data from four studies were obtained from the Gene Expression Omnibus with accession numbers GSE142536, GSE62003, GSE62003 and GSE37722. These are studies with longitudinal methylation data, at different time points, that allowed us testing the effect age-related diseases (cancer and Alzheimer's disease) and of transient states (COVID 19 and pregnancy) on X-Ra. In the first study GSE142536, 17 elderly women were assayed immediately before major surgery, in the morning of postoperative day and between 4 and 7 days after surgery. Methylation data in blood was collected at each visit. Patients had a mean age of 79 years and three of them had cancer. In the second study GSE62003, blood methylation data were collected on11 elderly women (mean age 74.18), at two time points. Five patients were diagnosed with AD before the first visit. In the third study GSE62003, methylation data was collected in buffy coat from individuals who recovered from severe COVID 19 infection and were admitted to the UCI. Methylation was assayed at admission, during and previous discharge from UCI and at follow-up after discharge. In the fourth study GSE37722, methylation data was collected in the blood of 14 pregnant women at early, midterm, delivery and post-partum.

TCGA data

The Cancer Genome Atlas (TCGA) consortium has mapped DNA methylation in thousands of cancer samples using Illumina Infinium Human Methylation 450K BeadChip (Illumina 450K array). Methylation data for 15 different cancer sites and normal tissue were obtained. The data was downloaded from *ExperimentHub* version 2.0.1 using the R package *curatedTCGAData* version 1.16.0. The cancer sites included breast (BRCA), bladder (BLCA), colon (COAD), head and neck (HNSC), kidney (KIRC), liver (LIHC), lung adenocarcinoma (LUAD), lung squamous cell carcinoma (LUSC), pancreas (PAAD), rectum (READ), thyroid (THCA), uterus (UCEC), skin (SKCM) and ovary (OV). The last two were discarded because they did not have methylation data on more than 5 samples of healthy tissue. A total of 11,232 CpGs on chromosome X for 3,741 samples were analyzed.

Clinical, RNA-seq and CNV data for the 12 selected cancer sites was downloaded to perform associations with X-Ra. For clinical associations, data was downloaded using *TCGAbiolinks* version 2.22.4 and fitted proportional hazards regression models for survival data adjusting by age for each independent cancer site. The association between X-Ra and hormonal status (ER, PR, HER2) of breast cancer, adjusting by age was also tested. For RNA-seq data, surrogate variables were inferred with *svaseq* from the SVA R library for each individual cancer. The association of transcription levels of 20,532 genes with FIGO.2 was tested adjusting by age and surrogate variables, transforming the expression levels with *voom* and fitting regression models with *limma.* In each cancer site, genes with less than 15 counts in more than 75% of the samples were removed from the analysis.

**2. Methods**

X-Ra definition

To differentiate between women with different levels of X-Ra (or XCI), the list of CpGs in X-linked genes that are consistently inactivated across several tissues was compiled. Then, it was used the CpGs from TCGA that underwent a methylation harmonization workflow for Human Methylation 27 (HM27), HumanMethylation 450 (HM450), and EPIC platforms

(https://docs.gdc.cancer.gov/Data/Bioinformatics_Pipelines/Methylation_Pipeline/) to create the final list of 499 CpGs for data derived from HM27 (Table 1) and 4,757 CpGs for data derived from HM450 (Table 2) hypothesized to have methylation levels of 0.5 or higher due to XCI, which was considered as the reference panel.

Table 1 shows the panel of 468 CpGs used to calculate X-Ra levels in data derived from HM27 platform, and their corresponding position in the chromosome X and the associated gene.

**Table 1**

| Probe ID | Associated gene | Position in ChrX (hg19) | Probe ID | Associated gene | Position in ChrX (hg19) |
|---|---|---|---|---|---|
| cg00029931 | RPL36A | 100645741 | cg13286902 | TRMT2B | 100306780 |
| cg00032666 | MAMLD1 | 149613398 | cg13340269 | TMEM35 | 100333778 |
| cg00121904 | MAOB | 43741826 | cg13370916 | STARD8 | 67867403 |
| cg00126698 | BTK | 100640503 | cg13482233 | HEPH | 65382529 |
| cg00128197 | HSD17B10 | 53461455 | cg13658777 | PDHA1 | 19361740 |
| cg00141845 | UPRT | 74493352 | cg13674559 | WDR44 | 117479752 |
| cg00145348 | APEX2 | 55026862 | cg13839778 | SYN1 | 47479048 |
| cg00151234 | PGK1 | 77359363 | cg13897449 | PFKFB1 | 55021841 |
| cg00179446 | UPF3B | 118986947 | cg13915726 | DUSP9 | 152908309 |
| cg00180631 | MTM1 | 149736640 | cg14106263 | RBMX2 | 129535683 |
| cg00191052 | CHST7 | 46432770 | cg14132995 | SLC35A2 | 48769370 |
| cg00230685 | SCML1 | 17755839 | cg14168975 | ALAS2 | 55057838 |
| cg00238052 | TCEAL2 | 101380291 | cg14179628 | TCEAL7 | 102584953 |
| cg00279797 | AMMECR1 | 109561918 | cg14230133 | IDS | 148587055 |
| cg00404599 | TSC22D3 | 107018431 | cg14321399 | RRAGB | 55744191 |
| cg00536175 | GATA1 | 48645024 | cg14368286 | PRAF2 | 48931953 |
| cg00547789 | RAB39B | 154493750 | cg14372520 | EDA2R | 65835804 |
| cg00618396 | VBP1 | 154444551 | cg14457691 | ARMCX4 | 100740937 |
| cg00673020 | MOSPD1 | 134049678 | cg14506668 | FHL1 | 135228853 |
| cg00690049 | MAGED2 | 54834244 | cg14520892 | POLA1 | 24711633 |
| cg00725777 | NGFRAP1 | 102630411 | cg14562990 | LDOC1 | 140271626 |
| cg00862041 | GPRASP2 | 101967017 | cg14570389 | RAP2C | 131351543 |
| cg00874863 | LHFPL1 | 111924370 | cg14600040 | GPC4 | 132549764 |
| cg00920960 | XIAP | 122993419 | cg14625604 | UBE2A | 118708625 |
| cg00932276 | EGFL6 | 13587976 | cg14642832 | RBMX | 135963137 |
| cg00939965 | PRRG1 | 37245294 | cg14688956 | PAK3 | 110339862 |
| cg00941549 | AKAP4 | 49966273 | cg14708847 | PAK3 | 110339313 |
| cg00947275 | LASLL | 64754953 | cg14743649 | CENPI | 100353353 |
| cg00962799 | SSX8 | 52651887 | cg14755341 | CDKL5 | 18444361 |

(continued)

| Probe ID | Associated gene | Position in ChrX (hg19) | Probe ID | Associated gene | Position in ChrX (hg19) |
|---|---|---|---|---|---|
| cg00968475 | BHLHB9 | 101975738 | cg14766682 | CCDC120 | 48916658 |
| cg00981643 | MECP2 | 153363708 | cg14807303 | AR | 66763445 |
| cg01079126 | MTMR1 | 149861641 | cg14815778 | FAM58A | 152865295 |
| cg01080862 | IL13RA1 | 117861389 | cg14841098 | SLC9A7 | 46618968 |
| cg01086868 | AR | 66764531 | cg14854487 | LAMP2 | 119603098 |
| cg01140782 | FAM58A | 152864413 | cg14892037 | SNX12 | 70288026 |
| cg01172484 | MAGEH1 | 55478539 | cg14986420 | YIPF6 | 67718299 |
| cg01195014 | MAGED1 | 51546668 | cg15200096 | KCND1 | 48829104 |
| cg01241836 | TFE3 | 48901346 | cg15231886 | UTP14A | 129039863 |
| cg01257202 | DKC1 | 153991830 | cg15241084 | TLR7 | 12886121 |
| cg01309671 | FAM122C | 133941225 | cg15309236 | BHLHB9 | 101975597 |
| cg01353347 | IRAKI | 153285158 | cg15454483 | SNX12 | 70288604 |
| cg01408383 | PIGA | 15353254 | cg15480941 | PIM2 | 48776125 |
| cg01632517 | SSX8 | 52652796 | cg15536552 | ACSL4 | 108976035 |
| cg01851385 | CLCN4 | 10125135 | cg15681351 | UPF3B | 118987163 |
| cg01885202 | CDKL5 | 18443457 | cg15895359 | EFNB1 | 68048613 |
| cg01887353 | ZNF41 | 47342480 | cg15977272 | EFNB1 | 68048909 |
| cg02004156 | TFE3 | 48900769 | cg16022279 | ZNF185 | 152086723 |
| cg02148711 | ATRX | 77041559 | cg16082125 | USP11 | 47092560 |
| cg02232922 | SLC25A43 | 118532809 | cg16204757 | MSN | 64887739 |
| cg02264284 | HPRT1 | 133594951 | cg16227684 | GD11 | 153664862 |
| cg02326006 | ARHGEF9 | 62974433 | cg16227775 | ABCB7 | 74376251 |
| cg02345317 | NLGN3 | 70364543 | cg16243644 | STAG2 | 123095766 |
| cg02457752 | CDR1 | 139868078 | cg16269097 | CASK | 41783155 |
| cg02492740 | UBQLN2 | 56590362 | cg16343842 | CD99L2 | 150066722 |
| cg02549418 | MBNL3 | 131573634 | cg16367232 | UTP14A | 129040481 |
| cg02649608 | ZBTB33 | 119384792 | cg16429439 | MAGT1 | 77150966 |
| cg02690554 | CITED1 | 71527606 | cg16440909 | MAMLD1 | 149613745 |
| cg02725692 | PORCN | 48367969 | cg16452396 | PHF6 | 133507394 |
| cg02804166 | PHF6 | 133506996 | cg16510010 | FAM122C | 133941163 |
| cg03020597 | SLITRK2 | 144898810 | cg16510657 | TMEM35 | 100333950 |
| cg03021892 | SLC38A5 | 48329278 | cg16561743 | CXorf23 | 19984992 |
| cg03057808 | CXorf57 | 105855296 | cg16679837 | AR | 66764000 |
| cg03122511 | NKAP | 119077820 | cg16716983 | DRP2 | 100474247 |
| cg03157806 | TMEM185A | 148713279 | cg16948369 | KLHL4 | 86772436 |
| cg03161453 | BCOR | 39962049 | cg17001703 | LASLL | 64754863 |
| cg03164831 | BRWD3 | 80065461 | cg17051440 | CLDN2 | 106163689 |
| cg03221436 | CCDC120 | 48916091 | cg17119559 | GPR64 | 19100990 |
| cg03273615 | RBM41 | 106362373 | cg17196716 | CXorf36 | 45059906 |

(continued)

| Probe ID | Associated gene | Position in ChrX (hg19) | Probe ID | Associated gene | Position in ChrX (hg19) |
|---|---|---|---|---|---|
| cg03445896 | LPAR4 | 78010565 | cg17354708 | HDAC8 | 71793175 |
| cg03455762 | XPNPEP2 | 128873475 | cg17398312 | FRMPD4 | 12157040 |
| cg03464416 | RPL10 | 153626979 | cg17439480 | SLC25A43 | 118533042 |
| cg03575468 | PDHA1 | 19362613 | cg17552650 | WDR45 | 48957963 |
| cg03672021 | FLNA | 153602658 | cg17571782 | CXorf40A | 148623024 |
| cg03735049 | TSR2 | 54466618 | cg17626563 | RS1 | 18691141 |
| cg03791917 | BTK | 100641287 | cg17662177 | CFP | 47490087 |
| cg04037732 | NLGN3 | 70364775 | cg17694877 | DIAPH2 | 95939631 |
| cg04058675 | HUWE1 | 53680779 | cg17705081 | GPKOW | 48980694 |
| cg04238548 | TBC1D8B | 106045825 | cg17718322 | OPHN1 | 67653749 |
| cg04241572 | HSD17B10 | 53461213 | cg17843048 | PHF16 | 46771580 |
| cg04283377 | ARAF | 47420348 | cg17991347 | MAGED1 | 51546132 |
| cg04297329 | NAP1L2 | 72434720 | cg18049750 | SCML1 | 17755305 |
| cg04336572 | ATP1B4 | 119496002 | cg18091964 | SASH3 | 128913980 |
| cg04368919 | EFNB1 | 68048131 | cg18230216 | NYX | 41306271 |
| cg04493740 | SLC25A14 | 129474199 | cg18233735 | PDZD4 | 153095721 |
| cg04499381 | SASH3 | 128913671 | cg18289259 | TIMM8A | 100604065 |
| cg04533591 | RPL39 | 118926128 | cg18307604 | LAMP2 | 119603221 |
| cg04544154 | ELF4 | 129245509 | cg18389752 | AMOT | 112084448 |
| cg04574723 | P2RY4 | 69479214 | cg18414950 | PDK3 | 24483896 |
| cg04702045 | OPHN1 | 67653598 | cg18433694 | ATP11C | 138914571 |
| cg04755662 | AR | 66763359 | cg18468467 | CITED1 | 71526790 |
| cg04872051 | CHST7 | 46433447 | cg18503052 | PLP2 | 49028039 |
| cg04882894 | IGSF1 | 130423289 | cg18507125 | RPL36A | 100646106 |
| cg04907664 | EFNB1 | 68049805 | cg18535534 | BCORL1 | 129115458 |
| cg04920616 | FOXP3 | 49121288 | cg18564727 | PLP1 | 103030978 |
| cg04922020 | FAM127A | 134166009 | cg18624866 | SMS | 21958505 |
| cg04929865 | BGN | 152760078 | cg18653991 | PIM2 | 48776735 |
| cg04944936 | FAM120C | 54209594 | cg18665563 | HMGB3 | 150151128 |
| cg05019001 | AR | 66766334 | cg18695429 | CSTF2 | 100074974 |
| cg05039054 | DMD | 33357782 | cg18731813 | ARMCX1 | 100805683 |
| cg05105069 | TCEAL7 | 102585354 | cg18742441 | ATP6AP1 | 153657222 |
| cg05206587 | GLRA2 | 14547880 | cg18780401 | OTUD5 | 48815125 |
| cg05254049 | SYN1 | 47479662 | cg18799866 | SLC9A7 | 46618164 |
| cg05327750 | RBM41 | 106361572 | cg18869368 | MAGEH1 | 55478180 |
| cg05373692 | ZDHHC15 | 74743375 | cg18923230 | RPL10 | 153626413 |
| cg05397738 | PGRMC1 | 118369571 | cg19002579 | SMPX | 21776218 |
| cg05437059 | KCNE1L | 108868775 | cg19011603 | UPRT | 74494066 |
| cg05494459 | FTSJ1 | 48334337 | cg19055639 | ARMCX2 | 100914138 |

(continued)

| Probe ID | Associated gene | Position in ChrX (hg19) | Probe ID | Associated gene | Position in ChrX (hg19) |
|---|---|---|---|---|---|
| cg05511752 | MED12 | 70338656 | cg19062189 | TMSB15A | 101771625 |
| cg05587158 | ARR3 | 69487748 | cg19138060 | WDR13 | 48455227 |
| cg05592527 | ARMCX6 | 100872988 | cg19194595 | PHF16 | 46771884 |
| cg05782975 | NGFRAP1 | 102631408 | cg19223616 | CLCN5 | 49834454 |
| cg05786601 | AR | 66764126 | cg19407886 | SLC38A5 | 48328287 |
| cg05856884 | SMPX | 21776372 | cg19410841 | NKAP | 119077663 |
| cg05921207 | CHRDL1 | 110038886 | cg19481052 | PLP1 | 103032137 |
| cg05935584 | HMGB3 | 150151823 | cg19523692 | ATP1B4 | 119495705 |
| cg05961595 | APEX2 | 55026485 | cg19618706 | BGN | 152760906 |
| cg06041240 | NXT2 | 108778793 | cg19688503 | CAPN6 | 110513941 |
| cg06051662 | PDZD4 | 153096786 | cg19696622 | RPS6KA3 | 20285908 |
| cg06208111 | ITM2A | 78624108 | cg19928247 | TCEAL1 | 102883794 |
| cg06220755 | RAI2 | 17821058 | cg19949137 | HTATSF1 | 135579980 |
| cg06273903 | UBE2A | 118707913 | cg20051589 | DLG3 | 69664482 |
| cg06277838 | SLC16A2 | 73641370 | cg20062122 | CLCN5 | 49833742 |
| cg06306751 | F8 | 154251005 | cg20085077 | ARMCX4 | 100740421 |
| cg06340713 | CHM | 85303427 | cg20104776 | LDOC1 | 140271176 |
| cg06363801 | MOSPD1 | 134049271 | cg20119635 | DYNLT3 | 37707074 |
| cg06384491 | SPIN2A | 57163888 | cg20154346 | RAI2 | 17821097 |
| cg06428055 | ELF4 | 129243872 | cg20244073 | MID1 | 10851627 |
| cg06457357 | KLF8 | 56259243 | cg20275344 | RAB9B | 103087153 |
| cg06489418 | FTSJ1 | 48334722 | cg20308511 | SAT1 | 23801056 |
| cg06494770 | KLHL13 | 117107675 | cg20371650 | PRAF2 | 48931388 |
| cg06526829 | KCND1 | 48828070 | cg20450764 | TSPAN7 | 38420326 |
| cg06655100 | KLF8 | 56259373 | cg20504202 | TSR2 | 54467052 |
| cg06657741 | SLC9A6 | 135067456 | cg20540913 | EDA | 68836108 |
| cg06659128 | TMLHE | 154842535 | cg20641280 | LAGE3 | 153707793 |
| cg06700462 | PHKA2 | 19002006 | cg20816612 | PHKA1 | 71933629 |
| cg06731599 | ATP6AP2 | 40440704 | cg20825323 | IGBP1 | 69353047 |
| cg06758848 | FLNA | 153603268 | cg20832009 | STARD8 | 67867718 |
| cg06822229 | ACSL4 | 108976856 | cg20842040 | PRPS2 | 12809608 |
| cg06900776 | ARMCX3 | 100878107 | cg20855303 | REPS2 | 16965405 |
| cg06942445 | TRO | 54947373 | cg20869203 | LHFPL1 | 111923211 |
| cg06944050 | LONRF3 | 118109068 | cg20878850 | AR | 66763685 |
| cg06959635 | MCF2 | 138724627 | cg20922422 | PCYT1B | 24665669 |
| cg07177300 | FRMPD4 | 12156499 | cg20931907 | CXorf67 | 51149742 |
| cg07242375 | PRICKLE3 | 49042556 | cg20964758 | ENOX2 | 130037157 |
| cg07297906 | GPC3 | 133119578 | cg21030483 | TMEM185A | 148713678 |
| cg07311845 | TMLHE | 154842736 | cg21045917 | ALAS2 | 55057395 |

(continued)

| Probe ID | Associated gene | Position in ChrX (hg19) | Probe ID | Associated gene | Position in ChrX (hg19) |
|---|---|---|---|---|---|
| cg07314984 | PHKA2 | 19003243 | rq21046413 | AIFM1 | 129299367 |
| cg07342016 | RAB33A | 129306129 | cg21067846 | MORC4 | 106228404 |
| cg07428182 | PLXNA3 | 153686926 | cg21080294 | PRPS1 | 106871478 |
| cg07473550 | TSC22D3 | 107019191 | rq21183872 | LONRF3 | 118108506 |
| cg07581973 | PRDX4 | 23685729 | rq21195120 | MBTPS2 | 21858304 |
| cg07654896 | RGN | 46937829 | cg21224759 | PIN4 | 71401335 |
| cg07688234 | CFP | 47489663 | cg21232685 | GPRASP1 | 101909064 |
| cg07780118 | AR | 66766506 | cg21248478 | CLIC2 | 154563968 |
| cg07824317 | RPGR | 38187006 | cg21258987 | GSPT2 | 51485834 |
| cg07874284 | PRPS2 | 12809156 | cg21284636 | PIN4 | 71401009 |
| cg07876586 | PGRMC1 | 118370356 | cg21365235 | OCRL | 128674765 |
| cg07897414 | KLHL4 | 86772895 | cg21395967 | TBC1D8B | 106046174 |
| cg07911663 | TMEM164 | 109245325 | cg21516819 | TSPAN7 | 38421078 |
| cg07917796 | DIAPH2 | 95939710 | cg21557231 | MCF2 | 138725077 |
| cg08021299 | HEPH | 65383076 | cg21693321 | MED12 | 70338331 |
| cg08076508 | PHF8 | 54070176 | cg21697779 | FUNDC2 | 154255534 |
| cg08093211 | CSTF2 | 100075588 | cg21836062 | DMD | 33357661 |
| cg08214957 | CDR1 | 139866495 | cg21860846 | ELK1 | 47510507 |
| cg08248532 | PRDX4 | 23685453 | cg21888438 | MID11P1 | 38663117 |
| cg08262933 | MORF4L2 | 102941498 | cg21932800 | MMGT1 | 135056858 |
| cg08300622 | SCML2 | 18372963 | cg21966410 | AR | 66765882 |
| cg08324796 | GPKOW | 48979771 | cg21978299 | MCTS1 | 119737955 |
| cg08575950 | MAP7 D2 | 20135450 | cg22010317 | CXorf36 | 45060544 |
| cg08591489 | CXorf56 | 118699483 | cg22016571 | MST4 | 131157426 |
| cg08680085 | MST4 | 131157316 | cg22343001 | ARMCX2 | 100914848 |
| cg08695223 | SLC9A6 | 135067793 | cg22543648 | GATA1 | 48644610 |
| cg08759036 | OCRL | 128674134 | cg22601215 | GSPT2 | 51486845 |
| cg08785133 | PORCN | 48367193 | cg22617367 | NKRF | 118740614 |
| cg08798116 | GPC4 | 132548882 | cg22731373 | PCYT1B | 24665296 |
| cg08846002 | ZNF75D | 134477026 | cg22858728 | MPP1 | 154034149 |
| cg08884343 | FOXP3 | 49122423 | cg22873668 | CXorf56 | 118699347 |
| cg08905118 | RP2 | 46696426 | cg22983460 | SUV39H1 | 48554902 |
| cg08965337 | RAP2C | 131352534 | cg23026995 | XPNPEP2 | 128872940 |
| cg09018810 | IDS | 148586931 | cg23066860 | GPRASP2 | 101967557 |
| cg09144786 | WDR44 | 117479692 | cg23083672 | EGFL6 | 13587603 |
| cg09275137 | TMEM187 | 153238579 | cg23116589 | ARMCX1 | 100805503 |
| cg09406238 | MTMR8 | 63615383 | cg23137494 | SLC16A2 | 73639809 |
| cg09517019 | ARHGEF6 | 135863323 | cg23163644 | TCEAL2 | 101381018 |
| cg09625066 | RGN | 46937571 | cg23232084 | UBL4A | 153715609 |

(continued)

| Probe ID | Associated gene | Position in ChrX (hg19) | Probe ID | Associated gene | Position in ChrX (hg19) |
|---|---|---|---|---|---|
| cg09778422 | MSN | 64887118 | cg23269489 | BCORL1 | 129117241 |
| cg09920632 | PHF8 | 54070741 | cg23279136 | ABCB7 | 74375966 |
| cg09923855 | HTATSF1 | 135579466 | cg23493704 | SLC10A3 | 153719780 |
| cg09950034 | XIAP | 122994069 | cg23545272 | EFNB1 | 68049613 |
| cg10009003 | UBQLN2 | 56589792 | cg23571457 | GPRASP1 | 101907558 |
| cg10009830 | MAP7 D2 | 20134904 | cg23698956 | FAM3A | 153744915 |
| cg10076009 | SMARCA1 | 128656725 | cg23709838 | SLC35A2 | 48768883 |
| cg10090585 | ENOX2 | 130037302 | cg23756219 | DRP2 | 100475124 |
| cg10246296 | CLCN4 | 10124977 | cg23926715 | ZC4H2 | 64196278 |
| cg10290730 | ATP2B3 | 152801187 | cg23947872 | MTMR1 | 149862363 |
| cg10318351 | GNL3L | 54556330 | cg24034992 | YIPF6 | 67719066 |
| cg10417559 | PRICKLE3 | 49043355 | cg24054653 | C1GALT1C 1 | 119763829 |
| cg10455133 | ARAF | 47420508 | cg24139739 | EFNB1 | 68048818 |
| cg10526888 | MAGT1 | 77151237 | cg24183173 | BCOR | 39962195 |
| cg10530733 | GPR173 | 53078631 | cg24249486 | ARHGAP6 | 11684119 |
| cg10536534 | MCTS1 | 119737767 | cg24340926 | RAB33A | 129305036 |
| cg10560144 | PGK1 | 77359355 | cg24376537 | DLG3 | 69665103 |
| cg10584819 | HPRT1 | 133593670 | cg24443136 | ATP2B3 | 152801587 |
| cg10591659 | NYX | 41306768 | cg24447042 | SMARCA1 | 128657893 |
| cg10783042 | HDAC6 | 48660813 | cg24511534 | RP2 | 46696351 |
| cg10818284 | SYP | 49056605 | cg24616736 | HDAC6 | 48659713 |
| cg10894453 | ITM2A | 78622859 | cg24765005 | BEX2 | 102565608 |
| cg10950297 | EFNB1 | 68049085 | cg24774956 | SH3KBP1 | 19907122 |
| cg11049305 | ZC4H2 | 64196751 | cg24779040 | TSPYL2 | 53111607 |
| cg11132751 | CXorf67 | 51149970 | cg25053301 | WDR13 | 48456550 |
| cg11233153 | SLC10A3 | 153718691 | cg25063710 | BRWD3 | 80064750 |
| cg11272332 | ZDHHC15 | 74742810 | cg25130333 | CD99L2 | 150067942 |
| cg11291009 | ARHGEF9 | 62975067 | cg25257360 | CXorf23 | 19983591 |
| cg11371160 | UBL4A | 153714615 | cg25270201 | WWC3 | 9984045 |
| cg11472424 | PFKFB1 | 55020450 | cg25316166 | USP11 | 47092288 |
| cg11479591 | MECP2 | 153362176 | cg25317260 | AIFM1 | 129300090 |
| cg11494656 | ARR3 | 69487197 | cg25359581 | DYNLT3 | 37706819 |
| cg11565248 | RPS6KA3 | 20284128 | cg25410279 | LAGE3 | 153707517 |
| cg11598929 | FAAH2 | 57313075 | cg25456959 | ASB12 | 63446044 |
| cg11609760 | FGD1 | 54522363 | cg25488547 | CHM | 85303309 |
| cg11653864 | ELK1 | 47509884 | cg25710140 | MIDI | 10852037 |
| cg11661234 | RLIM | 73834596 | cg25714610 | FAM3A | 153744216 |
| cg11681617 | GAB3 | 153979649 | cg25812668 | MID11P1 | 38662926 |
| cg11764747 | EFNB1 | 68050216 | cg25813820 | MPP1 | 154033598 |

(continued)

| Probe ID | Associated gene | Position in ChrX (hg19) | Probe ID | Associated gene | Position in ChrX (hg19) |
|---|---|---|---|---|---|
| cg11806565 | PHKA1 | 71934695 | cg25933726 | RPGR | 38186399 |
| cg11824111 | DKC1 | 153990676 | cg25941716 | SH3KBP1 | 19905525 |
| cg11839979 | TMEM164 | 109246372 | cg26116400 | FUNDC2 | 154254947 |
| cg12064213 | WWC3 | 9982520 | cg26222407 | STAG2 | 123094884 |
| cg12324831 | VBP1 | 154444470 | cg26246138 | SCML2 | 18372612 |
| cg12434779 | ARHGAP6 | 11684104 | cg26309951 | MORF4L2 | 102941669 |
| cg12485020 | CHRDL1 | 110039269 | cg26328611 | EDA2R | 65835992 |
| cg12549513 | BEX2 | 102565951 | cg26461352 | TIMM8A | 100603561 |
| cg12576145 | AMMECR1 | 109560751 | cg26545968 | P2RY4 | 69480213 |
| cg12589433 | AR | 66765978 | cg26606552 | CCDC22 | 49092437 |
| cg12653105 | PLP2 | 49028299 | cg26617508 | EDA | 68835489 |
| cg12654845 | CLDN2 | 106163009 | cg26644504 | KCNE1L | 108869644 |
| cg12687215 | FAM50A | 153672898 | cg26655138 | RBMX2 | 129536087 |
| cg12691219 | ARMCX3 | 100878134 | cg26745032 | REPS2 | 16964743 |
| cg12707233 | WNK3 | 54384964 | cg26751631 | RLIM | 73834295 |
| cg12724827 | CENPI | 100355703 | cg26822601 | GNL3L | 54556833 |
| cg12731488 | SYTL5 | 37893211 | cg26848126 | CYSLTR1 | 77582913 |
| cg12753358 | EFNB1 | 68047716 | cg26872564 | ZNF185 | 152086636 |
| cg12799835 | FAAH2 | 57313034 | cg26955512 | HDAC8 | 71792545 |
| cg12900739 | RRAGB | 55744069 | cg27198071 | FAM127A | 134166610 |
| cg12938998 | GAB3 | 153978709 | cg27271368 | AR | 66764411 |
| cg13128531 | ZBTB33 | 119384384 | cg27278294 | MTM1 | 149736594 |
| cg13174077 | CUL4B | 119709371 | cg27465531 | MBTPS2 | 21857712 |
| cg13183496 | FAM120C | 54209920 | cg27581839 | AMOT | 112083581 |
| cg13257485 | NXT2 | 108778854 | cg27584469 | SLITRK2 | 144899412 |

Probe ID: unique identifier from the Illumina CG database. Chromosomal position (hg19): chromosomal coordinates of the CpG in the X chromosome (build hg19). Associated gene: target gene name from the UCSC database.

Table 2 shows the panel of 468 CpGs used to calculate X-Ra levels in data derived from HM450 platform, and their corresponding position in the chromosome X and the associated gene

**Table 2**

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00005617 | EFNB1 | 68059898 | cg08065271 | PDZD4 | 153090978 | cg17703220 | XIAP | 122993978 |
| cg00011891 | UBL4A | 153714660 | cg08070147 | SCML1 | 17756762 | cg17705081 | GPKOW | 48980694 |
| cg00014152 | WDR13 | 48457128 | cg08076508 | PHF8 | 54070176 | cg17710066 | SCML2 | 18373455 |
| cg00016522 | MTM1 | 149737876 | cg08076861 | ZNF275 | 152599398 | cg17715113 | TBC1D25 | 48402339 |
| cg00016934 | BCOR | 40035961 | cg08087512 | DKC1 | 153990886 | cg17715469 | PDZD4 | 153068923 |
| cg00026186 | PORCN | 48367230 | cg08087911 | TRMT2B | 100306877 | cg17718322 | OPHN1 | 67653749 |
| cg00027454 | RP2 | 46740285 | cg08093211 | CSTF2 | 100075588 | cg17720011 | MOSPD1 | 134049345 |
| cg00029931 | RPL36A | 100645741 | cg08097522 | GRIPAP1 | 48833607 | cg17725109 | GPC3 | 133093850 |
| cg00031235 | PPP1R3F | 49124819 | cg08099387 | CSTF2 | 100075179 | cg17725595 | BGN | 152772036 |
| cg00032666 | MAMLD1 | 149613398 | cg08100265 | THOC2 | 122867073 | cg17727916 | SCML2 | 18373022 |
| cg00040923 | ARMCX1 | 100806880 | cg08104711 | CCDC22 | 49091769 | cg17729873 | FAM127B | 134186456 |
| cg00046099 | GNL3L | 54556443 | cg08113756 | SPIN3 | 57022079 | cg17731486 | ARMCX3 | 100878793 |
| cg00112256 | DOCK11 | 117629928 | cg08117103 | TSC22D3 | 106957583 | cg17734959 | SUV39H1 | 48567293 |
| cg00114625 | PDK3 | 24483478 | cg08118341 | PRAF2 | 48931823 | cg17736560 | ZNF185 | 152110426 |
| cg00114913 | LONRF3 | 118107540 | cg08119295 | TCEAL7 | 102586466 | cg17758583 | FUNDC2 | 154254916 |
| cg00116709 | MAGIX | 49020091 | cg08122495 | HMGB3 | 150150670 | cg17775340 | DMD | 31284448 |
| cg00117663 | MAGEB16 | 35816347 | cg08126551 | PDZD4 | 153069729 | cg17775518 | SLC35A2 | 48768764 |
| cg00120172 | ATRX | 77042539 | cg08130682 | STAG2 | 123094001 | cg17792806 | TCEAL7 | 102585107 |
| cg00121904 | MAOB | 43741826 | cg08143197 | SLC35A2 | 48761518 | cg17794813 | RPS6KA3 | 20285083 |
| cg00124708 | ARMCX3 | 100877463 | cg08147391 | CXorf56 | 118699975 | cg17797857 | KLHL15 | 24005356 |
| cg00126698 | BTK | 100640503 | cg08156775 | BCOR | 40009864 | cg17800714 | ATP6AP1 | 153656771 |
| cg00128197 | HSD17B10 | 53461455 | cg08195538 | STARD8 | 67914130 | cg17808639 | EGFL6 | 13586377 |
| cg00139317 | PCYT1B | 24665544 | cg08199506 | FAM120C | 54209916 | cg17811760 | CLCN5 | 49687610 |
| cg00139547 | LAMP2 | 119603329 | cg08209483 | LONRF3 | 118110092 | cg17814335 | KIAA2022 | 74143945 |
| cg00140189 | CLCN4 | 10128888 | cg08214610 | NKRF | 118739824 | cg17824401 | STARD8 | 67906181 |
| cg00141845 | UPRT | 74493352 | cg08214957 | CDR1 | 139866495 | cg17831869 | SAT1 | 23801244 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00142683 | UBE2A | 118708623 | cg08221357 | OTUD5 | 48815790 | cg17832062 | GPR64 | 19139978 |
| cg00145348 | APEX2 | 55026862 | cg08239458 | HTATSF1 | 135579472 | cg17833022 | AMMECR1 | 109452788 |
| cg00147819 | MED12 | 70349036 | cg08248532 | PRDX4 | 23685453 | cg17843048 | PHF16 | 46771580 |
| cg00150874 | BCOR | 40003284 | cg08255147 | HSD17B10 | 53460775 | cg17847781 | IGBP1 | 69353185 |
| cg00151234 | PGK1 | 77359363 | cg08262933 | MORF4L2 | 102941498 | cg17849117 | HUWE1 | 53713883 |
| cg00179446 | UPF3B | 118986947 | cg08266027 | NKRF | 118736960 | cg17852104 | BHLHB9 | 102000694 |
| cg00180631 | MTM1 | 149736640 | cg08275242 | IGBP1 | 69353171 | cg17853057 | PHF8 | 54070497 |
| cg00188971 | FTSJ1 | 48343409 | cg08282969 | XIAP | 122993891 | cg17856536 | SCML2 | 18257977 |
| cg00191052 | CHST7 | 46432770 | cg08300622 | SCML2 | 18372963 | cg17865482 | POLA1 | 24711703 |
| cg00191758 | MAGEH1 | 55478814 | cg08311689 | FAM3A | 153735091 | cg17868752 | CITED1 | 71527142 |
| cg00191903 | MUM1L1 | 105451070 | cg08318837 | KCND1 | 48827880 | cg17874273 | SCML1 | 17771386 |
| cg00200463 | STARD8 | 67913573 | cg08323957 | PAK3 | 110187600 | cg17875227 | MORF4L2 | 102941928 |
| cg00203568 | ARHGEF6 | 135850876 | cg08324796 | GPKOW | 48979771 | cg17877174 | SLC25A14 | 129473999 |
| cg00205905 | PRRG1 | 37208111 | cg08327779 | PDZD4 | 153070157 | cg17878135 | PLP2 | 49028673 |
| cg00206414 | BCOR | 39949510 | cg08327960 | MID11P1 | 38663047 | cg17914143 | TSR2 | 54466747 |
| cg00213805 | AIFM1 | 129300008 | cg08328842 | TCEAL4 | 102841681 | cg17914927 | ARHGEF6 | 135749399 |
| cg00213853 | MPP1 | 154034091 | cg08333400 | CLCN4 | 10126881 | cg17917970 | DUSP9 | 152908205 |
| cg00216997 | GPKOW | 48980153 | cg08355317 | BTK | 100642128 | cg17920241 | IDS | 148586978 |
| cg00230685 | SCML1 | 17755839 | cg08358587 | TIMM8A | 100603709 | cg17933493 | TKTL1 | 153532656 |
| cg00238052 | TCEAL2 | 101380291 | cg08384999 | BCOR | 40004346 | cg17934320 | PJA1 | 68385435 |
| cg00238131 | CITED1 | 71527298 | cg08395108 | MAGED2 | 54835372 | cg17959735 | AMMECR1 | 109561515 |
| cg00242233 | ARHGEF9 | 62954565 | cg08395966 | BHLHB9 | 102001268 | cg17962105 | CUL4B | 119658858 |
| cg00250879 | ARHGEF6 | 135847852 | cg08401365 | IRAK1 | 153276960 | cg17964359 | AR | 66766254 |
| cg00253811 | PIGA | 15353923 | cg08405463 | LONRF3 | 118108651 | cg17968718 | ARHGAP6 | 11161466 |
| cg00258480 | PIM2 | 48776587 | cg08408091 | FAM50A | 153672393 | cg17973147 | PHF16 | 46772394 |
| cg00261039 | BCOR | 40006123 | cg08437035 | RAB9B | 103078413 | cg17976175 | UBE2A | 118708089 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00263759 | TMEM187 | 153245522 | cg08447449 | KLHL13 | 117250943 | cg17979616 | NGFRAP1 | 102632793 |
| cg00264378 | CLCN5 | 49687306 | cg08456124 | ARHGAP6 | 11442350 | cg17991347 | MAGED1 | 51546132 |
| cg00266918 | SCML1 | 17755541 | cg08456555 | ARHGAP6 | 11282604 | cg17994214 | BCOR | 40036916 |
| cg00267352 | MAMLD1 | 149613734 | cg08457610 | MTMR1 | 149930840 | cg17995340 | MBNL3 | 131624394 |
| cg00272286 | ARL13A | 100224722 | cg08460331 | ARMCX4 | 100673802 | cg18001119 | ARAF | 47424678 |
| cg00276426 | MORC4 | 106243545 | cg08464305 | ARMCX6 | 100872602 | cg18003012 | APOOL | 84258777 |
| cg00279797 | AMMECR1 | 109561918 | cg08468443 | CLIC2 | 154563852 | cg18015035 | PHKA2 | 19002577 |
| cg00291164 | ZDHHC9 | 128939277 | cg08470131 | DGKK | 50213842 | cg18015266 | GATA1 | 48644759 |
| cg00291222 | ARMCX2 | 100910662 | cg08470157 | GK | 30671302 | cg18016370 | PIGA | 15354150 |
| cg00303108 | RBM41 | 106360881 | cg08476890 | MCF2 | 138774601 | cg18019621 | DMD | 33229689 |
| cg00323457 | RAI2 | 17879951 | cg08479532 | AMMECR1 | 109560744 | cg18031151 | CLDN2 | 106142458 |
| cg00328965 | ELF4 | 129243062 | cg08479711 | UBL4A | 153715068 | cg18031773 | ZNF81 | 47696239 |
| cg00329040 | FRMPD4 | 12600990 | cg08498653 | CDKL5 | 18459007 | cg18036967 | ARHGAP6 | 11683625 |
| cg00347850 | RP2 | 46696907 | cg08513688 | BCORL1 | 129115932 | cg18049750 | SCML1 | 17755305 |
| cg00355945 | HCCS | 11129241 | cg08539065 | WDR45 | 48957699 | cg18064897 | WWC3 | 10085184 |
| cg00357087 | MCTS1 | 119738550 | cg08573069 | NRK | 105066049 | cg18077778 | MAGEB16 | 35816333 |
| cg00360298 | CLCN5 | 49779544 | cg08575950 | MAP7D2 | 20135450 | cg18086582 | WDR13 | 48459799 |
| cg00360365 | GNL3L | 54556536 | cg08577442 | UBL4A | 153712753 | cg18087559 | PAK3 | 110187501 |
| cg00363318 | ZDHHC15 | 74743636 | cg08578454 | KCND1 | 48826167 | cg18091964 | SASH3 | 128913980 |
| cg00366674 | CLCN5 | 49686431 | cg08587717 | UBE2A | 118709261 | cg18093711 | RPL10 | 153626478 |
| cg00368230 | FAM58A | 152865160 | cg08590378 | SYTL5 | 37892347 | cg18096738 | MECP2 | 153363765 |
| cg00369058 | ARHGEF9 | 62972602 | cg08591489 | CXorf56 | 118699483 | cg18099006 | MID11P1 | 38662943 |
| cg00373606 | EFNB1 | 68048660 | cg08605656 | SLC16A2 | 73642885 | cg18102950 | THOC2 | 122866938 |
| cg00375105 | RPL10 | 153625785 | cg08609270 | SLITRK2 | 144903125 | cg18103394 | SYN1 | 47433374 |
| cg00379371 | PIM2 | 48774742 | cg08609845 | CXorf36 | 45060113 | cg18105467 | ATP6AP2 | 40440021 |
| cg00379983 | UTP14A | 129042628 | cg08610278 | APLN | 128788071 | cg18110168 | SHROOM4 | 50557055 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00382651 | MORF4L2 | 102941915 | cg08614574 | BCOR | 39961501 | cg18112782 | OTUD5 | 48814914 |
| cg00387056 | MAP7D3 | 135332628 | cg08615635 | FAM104B | 55188047 | cg18112785 | SLITRK2 | 144903000 |
| cg00387940 | MTM1 | 149742400 | cg08617833 | SMARCA1 | 128657697 | cg18124907 | MAGEH1 | 55478610 |
| cg00389552 | BCOR | 40028487 | cg08622041 | GATA1 | 48644252 | cg18144073 | MAGED2 | 54835328 |
| cg00390049 | PDZD4 | 153082274 | cg08623129 | TLR7 | 12884470 | cg18145088 | KCNE1L | 108869721 |
| cg00399450 | RLIM | 73834086 | cg08623691 | SNX12 | 70288356 | cg18147067 | ARHGAP6 | 11445818 |
| cg00401265 | LPAR4 | 78003113 | cg08625693 | DLG3 | 69674126 | cg18154457 | RBM10 | 47039371 |
| cg00402910 | AMMECR1 | 109561221 | cg08637771 | YIPF6 | 67718940 | cg18154784 | SAT1 | 23804100 |
| cg00404599 | TSC22D3 | 107018431 | cg08638354 | BHLHB9 | 101975281 | cg18155062 | PCYT1B | 24665469 |
| cg00406460 | SYN1 | 47433415 | cg08642787 | RPS6KA6 | 83443104 | cg18156601 | AR | 66766050 |
| cg00408231 | ARMCX1 | 100807848 | cg08648877 | SLC10A3 | 153719071 | cg18225658 | PDZD4 | 153084485 |
| cg00412010 | PAK3 | 110339837 | cg08656747 | ZNF81 | 47696169 | cg18230216 | NYX | 41306271 |
| cg00412554 | KLHL13 | 117107719 | cg08664054 | ARMCX2 | 100916283 | cg18230356 | MMGT1 | 135045624 |
| cg00416689 | NGFRAP1 | 102631887 | cg08680085 | MST4 | 131157316 | cg18232738 | SLC35A2 | 48769019 |
| cg00420717 | DLG3 | 69663586 | cg08695223 | SLC9A6 | 135067793 | cg18233735 | PDZD4 | 153095721 |
| cg00422452 | XPNPEP2 | 128872061 | cg08697944 | MAGED1 | 51637845 | cg18240390 | NXT2 | 108780378 |
| cg00426668 | CAPN6 | 110514231 | cg08700291 | MORC4 | 106240034 | cg18266647 | BHLHB9 | 102000811 |
| cg00428314 | UBE2A | 118708830 | cg08703114 | PGRMC1 | 118371210 | cg18273575 | PRRG1 | 37208602 |
| cg00431602 | SAT1 | 23799775 | cg08704320 | NSDHL | 152037892 | cg18274682 | ATP6AP2 | 40439842 |
| cg00433220 | RP2 | 46697120 | cg08707617 | BCOR | 39944352 | cg18275846 | CCDC22 | 49094825 |
| cg00433890 | RPS6KA3 | 20280171 | cg08711530 | DMD | 31282451 | cg18289259 | TIMM8A | 100604065 |
| cg00457389 | SCML2 | 18372736 | cg08722366 | MECP2 | 153295804 | cg18307604 | LAMP2 | 119603221 |
| cg00473051 | BCOR | 39949232 | cg08725455 | GPR173 | 53077301 | cg18336502 | ARHGAP6 | 11446209 |
| cg00473354 | FTSJ1 | 48334413 | cg08728127 | STARD8 | 67913300 | cg18337321 | SLITRK2 | 144899069 |
| cg00479885 | FAM127B | 134185269 | cg08744017 | GPR64 | 19139038 | cg18362897 | PSMD10 | 107331058 |
| cg00481999 | ZNF81 | 47696603 | cg08750061 | IL13RA1 | 117860789 | cg18383984 | HAUS7 | 152736387 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00491350 | CXorf36 | 45015865 | cg08758558 | CXorf36 | 45046434 | cg18389510 | RPS6KA3 | 20168552 |
| cg00510327 | RLIM | 73835412 | cg08759036 | OCRL | 128674134 | cg18389752 | AMOT | 112084448 |
| cg00524424 | CACNALF | 49089903 | cg08761350 | GPRASP2 | 101967081 | cg18394995 | TFE3 | 48901652 |
| cg00536175 | GATA1 | 48645024 | cg08762253 | PLP1 | 103031565 | cg18395382 | PLXNA3 | 153686199 |
| cg00547789 | RAB39B | 154493750 | cg08774483 | MID11P1 | 38663490 | cg18396637 | TSC22D3 | 107016227 |
| cg00550141 | PCYT1B | 24666268 | cg08777147 | BCOR | 39953550 | cg18396806 | FAM122C | 133941123 |
| cg00551957 | CXorf36 | 45017137 | cg08778681 | RBMX2 | 129535611 | cg18397310 | PRDX4 | 23685473 |
| cg00581583 | SYP | 49047791 | cg08783735 | WDR13 | 48458274 | cg18406314 | BHLHB9 | 102004767 |
| cg00583618 | MAGIX | 49020114 | cg08784874 | CUL4B | 119695210 | cg18406472 | GPRASP2 | 101967643 |
| cg00594844 | ATRX | 77037960 | cg08785133 | PORCN | 48367193 | cg18414950 | PDK3 | 24483896 |
| cg00597858 | MAGED1 | 51636568 | cg08786213 | RBMX | 135953040 | cg18419476 | EGFL6 | 13587506 |
| cg00603890 | CXorf56 | 118699184 | cg08786860 | MPP1 | 154034184 | cg18422972 | BCOR | 40029227 |
| cg00604086 | YIPF6 | 67718648 | cg08796898 | HUWE1 | 53713664 | cg18433694 | ATP11C | 138914571 |
| cg00611101 | TCEAL2 | 101380608 | cg08798116 | GPC4 | 132548882 | cg18440824 | AP00 | 23851466 |
| cg00615738 | BCOR | 40012582 | cg08800499 | HDX | 83757894 | cg18445504 | ACSL4 | 108913553 |
| cg00615794 | MORF4L2 | 102943966 | cg08812897 | RPS6KA3 | 20286243 | cg18450499 | HUWE1 | 53707967 |
| cg00618396 | VBP1 | 154444551 | cg08817443 | HTATSF1 | 135579746 | cg18452672 | IGSF1 | 130423431 |
| cg00619443 | CHRDL1 | 110039536 | cg08818092 | PCYT1B | 24665074 | cg18452799 | TRMT2B | 100307174 |
| cg00622384 | RPL39 | 118925635 | cg08822676 | LONRF3 | 118107997 | cg18453389 | RAB41 | 69502490 |
| cg00622389 | ARMCX2 | 100914950 | cg08823898 | IDS | 148587784 | cg18453720 | TMEM35 | 100332757 |
| cg00623388 | BCORL1 | 129116224 | cg08836298 | MID1 | 10588200 | cg18457106 | CXorf57 | 105876926 |
| cg00632130 | ZNF630 | 47931806 | cg08846002 | ZNF75D | 134477026 | cg18468467 | CITED1 | 71526790 |
| cg00632374 | CHST7 | 46432929 | cg08847773 | FUNDC2 | 154255265 | cg18470455 | BHLHB9 | 102001134 |
| cg00634642 | UTP14A | 129040194 | cg08855111 | ACSL4 | 108976340 | cg18480129 | PAK3 | 110187465 |
| cg00645049 | ATP6AP1 | 153656930 | cg08860608 | MAP7D3 | 135332975 | cg18480706 | ZDHHC15 | 74742458 |
| cg00646659 | PIM2 | 48776613 | cg08867267 | ENOX2 | 130033963 | cg18492059 | SLC38A5 | 48329985 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00648125 | NUDT10 | 51075046 | cg08867430 | WWC3 | 10092255 | cg18497064 | DDX26B | 134715899 |
| cg00650640 | EGFL6 | 13587678 | cg08871010 | CLCN5 | 49855003 | cg18502642 | NYX | 41328787 |
| cg00651446 | TCEAL2 | 101380386 | cg08872581 | MBTPS2 | 21857475 | cg18503052 | PLP2 | 49028039 |
| cg00661180 | DMD | 31285029 | cg08881528 | USP11 | 47092529 | cg18507125 | RPL36A | 100646106 |
| cg00666173 | CHST7 | 46433828 | cg08882363 | ZNF81 | 47695789 | cg18510064 | PAK3 | 110342214 |
| cg00667781 | HUWE1 | 53713885 | cg08884343 | FOXP3 | 49122423 | cg18515307 | DLG3 | 69664611 |
| cg00673020 | MOSPD1 | 134049678 | cg08905118 | RP2 | 46696426 | cg18515545 | ASB9 | 15288312 |
| cg00679603 | PCYT1B | 24691104 | cg08951164 | ATP11C | 138915416 | cg18516687 | FUNDC2 | 154254859 |
| cg00680673 | OTUD5 | 48815747 | cg08952424 | MED12 | 70338432 | cg18520280 | TREX2 | 152712923 |
| cg00685925 | TBC1D25 | 48398048 | cg08955276 | YIPF6 | 67719027 | cg18524099 | ARHGAP6 | 11456323 |
| cg00690049 | MAGED2 | 54834244 | cg08955859 | PGK1 | 77360510 | cg18535534 | BCORL1 | 129115458 |
| cg00697812 | RAB39B | 154488728 | cg08964402 | ZBTB33 | 119384409 | cg18536496 | GPRASP1 | 101906035 |
| cg00705055 | KCND1 | 48824499 | cg08965337 | RAP2C | 131352534 | cg18542573 | CITED1 | 71526995 |
| cg00713642 | IGBP1 | 69356087 | cg08966380 | EDA | 68835729 | cg18564727 | PLP1 | 103030978 |
| cg00718129 | WWC3 | 10084289 | cg08973369 | ASB9 | 15288572 | cg18585581 | WDR13 | 48454810 |
| cg00723973 | RP2 | 46696265 | cg08973646 | FAM3A | 153744488 | cg18610025 | MECP2 | 153363350 |
| cg00724728 | STARD8 | 67867238 | cg08975046 | SLC6A14 | 115566412 | cg18610223 | BCOR | 39942648 |
| cg00725777 | NGFRAP1 | 102630411 | cg08983668 | SCML2 | 18371906 | cg18617394 | ATRX | 76832207 |
| cg00725912 | PRPS2 | 12810331 | cg08991015 | MOSPD1 | 134049419 | cg18624866 | SMS | 21958505 |
| cg00727483 | ARHGEF9 | 62975065 | cg08994891 | UPF3B | 118987153 | cg18632412 | TIMM8A | 100603351 |
| cg00735876 | MAP7 D3 | 135334923 | cg08998989 | SCML1 | 17755531 | cg18636716 | WDR13 | 48455835 |
| cg00735885 | LHFPL1 | 111874418 | cg09005457 | MAOB | 43626076 | cg18653991 | PIM2 | 48776735 |
| cg00739787 | MST4 | 131157326 | cg09013091 | UBQLN2 | 56589946 | cg18665563 | HMGB3 | 150151128 |
| cg00742240 | MECP2 | 153287830 | cg09016243 | GLUD2 | 120181455 | cg18679504 | ATP6AP2 | 40439984 |
| cg00743068 | ATRX | 77041832 | cg09018810 | IDS | 148586931 | cg18689730 | PGRMC1 | 118370302 |
| cg00763725 | TMLHE | 154842689 | cg09033783 | HAUS7 | 152737555 | cg18695429 | CSTF2 | 100074974 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00765885 | TMSB15A | 101771142 | cg09034929 | SNX12 | 70288225 | cg18700197 | ZC3H12B | 64723481 |
| cg00768158 | SYP | 49054690 | cg09035532 | ARHGAP6 | 11398388 | cg18700741 | RLIM | 73834821 |
| cg00769799 | HPRT1 | 133594079 | cg09038535 | FAM120C | 54209715 | cg18701656 | SLITRK2 | 144898803 |
| cg00772918 | PDK3 | 24487341 | cg09045008 | PHF8 | 54043133 | cg18702239 | DLG3 | 69663606 |
| cg00775115 | ZNF41 | 47343198 | cg09049140 | GPR64 | 19011128 | cg18721956 | TKTL1 | 153532354 |
| cg00776430 | TSPYL2 | 53111427 | cg09049751 | DKC1 | 153993495 | cg18731813 | ARMCX1 | 100805683 |
| cg00779763 | SYN1 | 47479114 | cg09055214 | DUSP9 | 152915087 | cg18742441 | ATP6AP1 | 153657222 |
| cg00785877 | CAPN6 | 110513957 | cg09055253 | DKC1 | 153990874 | cg18748527 | BCOR | 40036021 |
| cg00790086 | RP2 | 46696474 | cg09056691 | MPP1 | 154033836 | cg18751919 | IL13RA1 | 117927862 |
| cg00799224 | PHKA2 | 19002639 | cg09061583 | ACOT9 | 23761412 | cg18765710 | BCOR | 39970419 |
| cg00804510 | BRCC3 | 154302208 | cg09063705 | MIDI | 10535248 | cg18769420 | RLIM | 73834329 |
| cg00810519 | SLC35A2 | 48769107 | cg09070095 | NUDT11 | 51240021 | cg18771512 | TKTL1 | 153532633 |
| cg00812293 | CLCN5 | 49831097 | cg09070199 | SPIN4 | 62570990 | cg18773835 | KLHL15 | 24046475 |
| cg00813142 | PRDX4 | 23685984 | cg09072865 | MORC4 | 106243506 | cg18778986 | TMLHE | 154842064 |
| cg00813999 | CYSLTR1 | 77582839 | cg09074544 | RAB33A | 129307515 | cg18779595 | TSR2 | 54466831 |
| cg00817749 | DYNLT3 | 37699631 | cg09075063 | BCOR | 40036659 | cg18780401 | OTUD5 | 48815125 |
| cg00818649 | CASK | 41782297 | cg09080960 | UBE2A | 118708361 | cg18785414 | DOCK11 | 117631015 |
| cg00820131 | STARD8 | 67912078 | cg09091181 | ACSL4 | 108976749 | cg18787045 | SLC16A2 | 73644754 |
| cg00822942 | FGD1 | 54521170 | cg09099391 | TRMT2B | 100285384 | cg18796341 | GD11 | 153664903 |
| cg00823357 | EFNB1 | 68048563 | cg09099637 | COL4A5 | 107939995 | cg18799866 | SLC9A7 | 46618164 |
| cg00825441 | APLN | 128789389 | cg09106468 | BHLHB9 | 102000610 | cg18809883 | ARMCX3 | 100878346 |
| cg00829575 | MID11P1 | 38663062 | cg09107157 | FTSJ1 | 48336389 | cg18811601 | SH3KBP1 | 19906283 |
| cg00832270 | PGK1 | 77359535 | cg09109599 | EFNB1 | 68048454 | cg18812977 | PHF16 | 46919898 |
| cg00839572 | EFNB1 | 68047847 | cg09112156 | LDOC1 | 140271259 | cg18813010 | MORF4L2 | 102931660 |
| cg00839869 | MAGED1 | 51640699 | cg09119244 | TSPYL2 | 53111239 | cg18813691 | BCOR | 40009241 |
| cg00845806 | VBP1 | 154445068 | cg09134399 | EFNB1 | 68047854 | cg18816329 | PHF8 | 54070878 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00854963 | NLGN3 | 70364566 | cg09139762 | CXorf36 | 45013417 | cg18828303 | MBTPS2 | 21857598 |
| cg00855706 | PGK1 | 77358961 | cg09143059 | FLNA | 153585715 | cg18834878 | NRK | 105067311 |
| cg00855923 | SMPX | 21776346 | cg09144786 | WDR44 | 117479692 | cg18836258 | HDAC8 | 71793459 |
| cg00862041 | GPRASP2 | 101967017 | cg09146129 | DIAPH2 | 95940102 | cg18849630 | MTM1 | 149736753 |
| cg00869298 | NDUFA1 | 119004292 | cg09163457 | DUSP9 | 152909014 | cg18869368 | MAGEH1 | 55478180 |
| cg00874863 | LHFPL1 | 111924370 | cg09167861 | FHL1 | 135251741 | cg18878700 | MBNL3 | 131623878 |
| cg00877150 | BCOR | 39972039 | cg09185350 | SHROOM4 | 50556361 | cg18879010 | TREX2 | 152710501 |
| cg00881203 | MTMR1 | 149930926 | cg09186478 | ZBTB33 | 119385725 | cg18885073 | PGRMC1 | 118370148 |
| cg00883449 | ZNF630 | 47930854 | cg09192059 | DMD | 31285032 | cg18923230 | RPL10 | 153626413 |
| cg00895705 | WNK3 | 54384537 | cg09192294 | LASLL | 64732622 | cg18924561 | MAGED2 | 54833733 |
| cg00902308 | ATP6AP2 | 40439693 | cg09195491 | KLF8 | 56258900 | cg18929098 | MOSPD1 | 134022854 |
| cg00909097 | PDHA1 | 19366087 | cg09207137 | SMS | 21958629 | cg18932686 | LAGE3 | 153707447 |
| cg00909388 | WWC3 | 10085400 | cg09208571 | MAGED2 | 54834954 | cg18939543 | SYP | 49057013 |
| cg00910720 | NXT2 | 108780265 | cg09209584 | FHL1 | 135291355 | cg18959966 | SMARCA1 | 128657727 |
| cg00917018 | LDOC1 | 140271549 | cg09210933 | SLC16A2 | 73641881 | cg18973991 | BCOR | 39965138 |
| cg00920537 | PHF6 | 133507230 | cg09227616 | SYP | 49056886 | cg18974084 | KIAA2022 | 74146414 |
| cg00920960 | XIAP | 122993419 | cg09231482 | RAB39B | 154493497 | cg18976803 | PHKA1 | 71934394 |
| cg00926894 | RGN | 46938229 | cg09253663 | APLN | 128788913 | cg18983709 | MST4 | 131157486 |
| cg00932276 | EGFL6 | 13587976 | cg09271603 | PDK3 | 24482885 | cg18986576 | BGN | 152772180 |
| cg00934057 | BCAP31 | 152985801 | cg09275137 | TMEM187 | 153238579 | cg18989057 | HDAC6 | 48660440 |
| cg00935394 | HCCS | 11129115 | cg09277772 | AR | 66764422 | cg18989233 | PGK1 | 77381829 |
| cg00935924 | UBE2A | 118708353 | cg09278708 | GPRASP1 | 101905837 | cg18989810 | DUSP9 | 152907969 |
| cg00936342 | TRMT2B | 100306566 | cg09285672 | GPKOW | 48980142 | cg18990292 | UBE2A | 118708366 |
| cg00936435 | FAM104B | 55187903 | cg09294666 | BCAP31 | 152973882 | cg18992293 | CXorf36 | 45010205 |
| cg00937263 | KIAA2022 | 74145012 | cg09300348 | CCDC22 | 49092146 | cg18998000 | IRAK1 | 153284959 |
| cg00939965 | PRRG1 | 37245294 | cg09302010 | SPIN3 | 57021502 | cg19000468 | CHM | 85302549 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg00941549 | AKAP4 | 49966273 | cg09307104 | DLG3 | 69665233 | cg19002579 | SMPX | 21776218 |
| cg00947275 | LASLL | 64754953 | cg09307237 | HDAC8 | 71549845 | cg19002828 | ITM2A | 78623745 |
| cg00953793 | RPS6KA3 | 20283580 | cg09309899 | IDS | 148586967 | cg19005062 | SYP | 49056661 |
| cg00962799 | SSX8 | 52651887 | cg09329826 | PDZD4 | 153094594 | cg19011603 | UPRT | 74494066 |
| cg00963467 | HMGB3 | 150151737 | cg09331780 | SATL1 | 84364419 | cg19014767 | TIMM8A | 100604147 |
| cg00965330 | ZNF182 | 47861943 | cg09332981 | GPRASP1 | 101905359 | cg19022966 | HPRT1 | 133594210 |
| cg00968475 | BHLHB9 | 101975738 | cg09335829 | MAOB | 43738323 | cg19039586 | PLXNA3 | 153686026 |
| cg00969573 | APOOL | 84259298 | cg09340741 | CHST7 | 46432858 | cg19041137 | TBC1D8B | 106046437 |
| cg00975326 | CXorf23 | 19989847 | cg09342610 | MAP7 D3 | 135333415 | cg19045344 | SATL1 | 84364350 |
| cg00979376 | KCND1 | 48826530 | cg09357232 | MAOB | 43741814 | cg19050914 | ARMCX6 | 100871603 |
| cg00981643 | MECP2 | 153363708 | cg09364317 | TREX2 | 152710813 | cg19053358 | CHRDL1 | 110039078 |
| cg00994038 | ZDHHC9 | 128978322 | cg09368035 | BCOR | 39943369 | cg19055639 | ARMCX2 | 100914138 |
| cg00996177 | RPGR | 38186710 | cg09370051 | MBNL3 | 131622892 | cg19056008 | DMD | 32175072 |
| cg00997875 | MSN | 64886318 | cg09385630 | PRRG1 | 37209324 | cg19056391 | EFNB1 | 68047992 |
| cg01003197 | OPHN1 | 67653925 | cg09406238 | MTMR8 | 63615383 | cg19059427 | FGD1 | 54519056 |
| cg01005760 | RAB41 | 69501696 | cg09407693 | PRPS1 | 106871408 | cg19060387 | AIFM1 | 129299908 |
| cg01008919 | NLGN3 | 70364646 | cg09411587 | DKC1 | 153990928 | cg19061228 | GPC4 | 132539525 |
| cg01010618 | IGSF1 | 130423130 | cg09418035 | DGKK | 50213528 | cg19062189 | TMSB15A | 101771625 |
| cg01022618 | ZNF185 | 152110833 | cg09418475 | SLC10A3 | 153718920 | cg19063141 | FLNA | 153603079 |
| cg01024350 | HDX | 83758409 | cg09418623 | AR | 66765795 | cg19063343 | PORCN | 48366824 |
| cg01051089 | CXorf23 | 19989310 | cg09443073 | RAI2 | 17879604 | cg19069416 | PLXNA3 | 153701160 |
| cg01052065 | PAK3 | 110339654 | cg09483847 | SMS | 21960827 | cg19092293 | LONRF3 | 118110194 |
| cg01055931 | FLNA | 153583236 | cg09484077 | PLP1 | 103029964 | cg19138060 | WDR13 | 48455227 |
| cg01057599 | UPRT | 74493807 | cg09514431 | SLC9A6 | 135067539 | cg19153710 | PHF8 | 54070756 |
| cg01058932 | PFKFB1 | 55021626 | cg009517019 | ARHGEF6 | 135863323 | cg19155032 | RBMX2 | 129538534 |
| cg01062269 | TIMM8A | 100603843 | cg009520583 | NUDT10 | 51076119 | cg19162841 | DGKK | 50214473 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01079126 | MTMR1 | 149861641 | cg009523186 | BRWD3 | 80064924 | cg19168249 | ATP6AP1 | 153657001 |
| cg01080862 | IL13RA1 | 117861389 | cg009526164 | FUNDC2 | 154254823 | cg19169535 | HDAC8 | 71699559 |
| cg01081720 | EFNB1 | 68047734 | cg009536390 | BGN | 152760191 | cg19169838 | DNASE1L1 | 153631347 |
| cg01083397 | MECP2 | 153362990 | cg009536652 | ATP1B4 | 119514843 | cg19172265 | BHLHB9 | 102001935 |
| cg01086868 | AR | 66764531 | cg009553014 | HDX | 83757168 | cg19177332 | LPAR4 | 78001744 |
| cg01087775 | ATRX | 77042040 | cg009598844 | MORF4L2 | 102941788 | cg19189070 | PLXNA3 | 153696548 |
| cg01098871 | WDR45 | 48958509 | cg009610569 | SLITRK2 | 144899592 | cg19194595 | PHF16 | 46771884 |
| cg01099541 | FAM127A | 134166046 | cg009624684 | ZNF275 | 152599453 | cg19208477 | LDOC1 | 140271604 |
| cg01104810 | HUWE1 | 53714710 | cg009625066 | RGN | 46937571 | cg19210487 | EDA | 68834606 |
| cg01108554 | SLC25A14 | 129473637 | cg009641151 | PLXNA3 | 153693918 | cg19214916 | SLC16A2 | 73642734 |
| cg01109012 | DYNLT3 | 37707161 | cg009655497 | TMEM164 | 109246283 | cg19223616 | CLCN5 | 49834454 |
| cg01109660 | GPR64 | 19008190 | cg009662069 | PHKA1 | 71899800 | cg19227523 | XPNPEP2 | 128872002 |
| cg01110765 | BCOR | 40016611 | cg009662907 | C1GALT1C1 | 119764367 | cg19227561 | CCDC120 | 48915352 |
| cg01112232 | TCEAL2 | 101382549 | cg009666011 | UBQLN2 | 56590113 | cg19230952 | FGD1 | 54523105 |
| cg01112624 | SYN1 | 47478591 | cg009673206 | ENOX2 | 130037332 | cg19238394 | WDR45 | 48958236 |
| cg01112702 | DKC1 | 154005455 | cg009673331 | PLXNA3 | 153692567 | cg19247520 | STARD8 | 67903751 |
| cg01112930 | DMD | 31285489 | cg009687634 | TCEAL4 | 102839328 | cg19250658 | SMARCA1 | 128654456 |
| cg01115636 | MCTS1 | 119751976 | cg009720515 | HDAC8 | 71792701 | cg19261292 | CCDC120 | 48922313 |
| cg01118900 | ARMCX4 | 100672737 | cg009724624 | TSC22D3 | 107019195 | cg19284023 | CCNB3 | 50094756 |
| cg01119520 | DLG3 | 69670818 | cg009725439 | SAT1 | 23801440 | cg19321437 | HDX | 83757536 |
| cg01119763 | PRRG1 | 37209004 | cg009738064 | ZNF185 | 152141622 | cg19332075 | SLITRK2 | 144903691 |
| cg01120006 | SLC35A2 | 48768342 | cg009738386 | RPL36A | 100645872 | cg19334385 | DOCK11 | 117630565 |
| cg01120894 | RAB33A | 129305726 | cg009743287 | TCEAL2 | 101380601 | cg19341307 | LRCH2 | 114468127 |
| cg01121830 | RPGR | 38186700 | cg009760728 | GPC3 | 133118344 | cg19344520 | RS1 | 18690398 |
| cg01131699 | ARHGEF6 | 135863051 | cg009761247 | IDS | 148585951 | cg19350224 | ZNF75D | 134419896 |
| cg01131961 | DRP2 | 100507598 | cg09773769 | GPC4 | 132547387 | cg19355593 | FAM104B | 55187242 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01132485 | CXorf66 | 139040942 | cg00978422 | MSN | 64887118 | cg19354122 | DMD | 33357554 |
| cg01134144 | ARHGEF6 | 135863582 | cg00981635 | ARMCX6 | 100872594 | cg19355555 | LRCH2 | 114468681 |
| cg01140782 | FAM58A | 152864413 | cg00986233 | SPIN4 | 62571228 | cg19355911 | PRPS2 | 12809326 |
| cg01145939 | WWC3 | 10098219 | cg009789426 | BHLHB9 | 102000698 | cg19358195 | RPS6KA6 | 83443518 |
| cg01150083 | GPKOW | 48980043 | cg09789474 | BHLHB9 | 101998763 | cg19382362 | PFKFB1 | 54976910 |
| cg01162841 | SH3KBP1 | 19818905 | cg09790289 | PGK1 | 77359560 | cg19383360 | CD99L2 | 150067114 |
| cg01162900 | MTMR1 | 149930532 | cg009791535 | GPC4 | 132435532 | cg19384325 | CDR1 | 139866549 |
| cg01172484 | MAGEH1 | 55478539 | cg09796544 | DUSP9 | 152913595 | cg19398192 | CXorf57 | 105855052 |
| cg01173508 | DCX | 110544388 | cg09796695 | TIMM17B | 48750744 | cg19405200 | STAG2 | 123097016 |
| cg01186310 | RAB41 | 69502085 | cg09799350 | SCML2 | 18372126 | cg19406135 | OPHN1 | 67653533 |
| cg01191578 | PFKFB1 | 55020455 | cg009809869 | FHL1 | 135277616 | cg19407886 | SLC38A5 | 48328287 |
| cg01191902 | PRAF2 | 48931851 | cg09810925 | BCOR | 40035331 | cg19410841 | NKAP | 119077663 |
| cg01193954 | HEPH | 65486761 | cg09813276 | ARMCX1 | 100805521 | cg19412336 | EFNB1 | 68061160 |
| cg01195014 | MAGED1 | 51546668 | cg09826544 | CLCN5 | 49686184 | cg19415116 | DDX26B | 134654686 |
| cg01207755 | CLCN5 | 49687058 | cg09849772 | ZNF185 | 152109751 | cg19415339 | HUWE1 | 53713491 |
| cg01212429 | CD99L2 | 150066099 | cg09850561 | TSPAN7 | 38420581 | cg19474517 | BEX2 | 102566419 |
| cg01212677 | PDK3 | 24483290 | cg09863225 | FTSJ1 | 48335010 | cg19481052 | PLP1 | 103032137 |
| cg01218690 | AR | 66787995 | cg09867302 | EFNB1 | 68048137 | cg19491742 | APEX2 | 55030675 |
| cg01220513 | SH3KBP1 | 19902129 | cg09868337 | PLXNA3 | 153686720 | cg19493242 | AR | 66765122 |
| cg01241836 | TFE3 | 48901346 | cg09889532 | CLIC2 | 154564511 | cg19499945 | DRP2 | 100474598 |
| cg01244877 | FLNA | 153598902 | cg09890269 | NUDT11 | 51239014 | cg19505129 | KLF8 | 56258695 |
| cg01257202 | DKC1 | 153991830 | cg09891468 | CDR1 | 139866441 | cg19506937 | APEX2 | 55026774 |
| cg01280366 | BCORL1 | 129120073 | cg09893016 | CLCN4 | 10145109 | cg19513111 | DUSP9 | 152909540 |
| cg01282661 | MAGIX | 49018919 | cg09895223 | CHRDL1 | 110039197 | cg19514407 | NSDHL | 152023870 |
| cg01283227 | CDKL5 | 18446221 | cg09895626 | ZNF75D | 134476314 | cg19514725 | HDX | 83757504 |
| cg1286388 | MAGT1 | 77151065 | cg09909750 | PHKA2 | 19003035 | cg19522972 | PLXNA3 | 153692379 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01288367 | PJA1 | 68384992 | cg09912589 | AMOT | 112067795 | cg19523014 | TMEM185A | 148713713 |
| cg01289637 | LRCH2 | 114468879 | cg09920632 | PHF8 | 54070741 | cg19523692 | ATP1B4 | 119495705 |
| cg01301326 | BCOR | 40014433 | cg09923855 | HTATSF1 | 135579466 | cg19532356 | SHROOM4 | 50556710 |
| cg01306265 | ARMCX2 | 100914469 | cg09935073 | PAK3 | 110187555 | cg19532714 | RPGR | 38186839 |
| cg01307615 | CAPN6 | 110515005 | cg09946139 | SCML1 | 17754979 | cg19545998 | P2RY4 | 69478365 |
| cg01309671 | FAM122C | 133941225 | cg09950034 | XIAP | 122994069 | cg19548176 | HDAC8 | 71792488 |
| cg01314643 | SPIN4 | 62570863 | cg09953988 | NYX | 41334352 | cg19548593 | VBP1 | 154444605 |
| cg01318188 | STAG2 | 123094675 | cg09966745 | PHKA1 | 71934213 | cg19549023 | MCF2 | 138773906 |
| cg01321830 | BCOR | 40005822 | cg09968133 | F8 | 154117632 | cg19566414 | IGSF1 | 130423448 |
| cg01325935 | STARD8 | 67905522 | cg09974287 | WWC3 | 9987772 | cg19567683 | CXorf56 | 118679581 |
| cg01333849 | PGRMC1 | 118370171 | cg09975171 | TMLHE | 154842841 | cg19579913 | FAM127A | 134166344 |
| cg01336098 | MBTPS2 | 21901558 | cg09978401 | PRPS2 | 12809216 | cg19580826 | ZNF81 | 47695826 |
| cg01337817 | DUSP9 | 152912242 | cg09985072 | OTUD5 | 48814942 | cg19587616 | PRAF2 | 48931578 |
| cg01340520 | BCOR | 40015196 | cg09989237 | HAUS7 | 152736955 | cg19593957 | MID1 | 10588208 |
| cg01343084 | FGD1 | 54521658 | cg09990582 | MORF4L2 | 102941884 | cg19599472 | ZDHHC15 | 74739430 |
| cg01345087 | SYN1 | 47479175 | cg09996779 | HPRT1 | 133594039 | cg19602178 | LASLL | 64754651 |
| cg01348942 | RAB40A | 102755537 | cg10001778 | FAM127A | 134166175 | cg19605705 | RAB40A | 102766745 |
| cg01349336 | HSD17B10 | 53461709 | cg10001949 | FAM127B | 134186517 | cg19605909 | YIPF6 | 67718729 |
| cg01353347 | IRAKI | 153285158 | cg10008347 | BCOR | 39958040 | cg19613925 | TCEAL4 | 102842289 |
| cg01355242 | KLF8 | 56258387 | cg10009003 | UBQLN2 | 56589792 | cg19616372 | DLG3 | 69674824 |
| cg01368763 | PDZD4 | 153072706 | cg10009830 | MAP7D2 | 20134904 | cg19618706 | BGN | 152760906 |
| cg01370077 | VMA21 | 150565243 | cg10016783 | SLITRK2 | 144898901 | cg19629755 | IGBP1 | 69353175 |
| cg01372992 | ENOX2 | 130037175 | cg10018458 | HSD17B10 | 53461681 | cg19631766 | OCRL | 128674232 |
| cg01374431 | BEX4 | 102469950 | cg10021693 | PHF16 | 46776812 | cg19635884 | ACSL4 | 108976825 |
| cg01375263 | SPIN2A | 57163746 | cg10026671 | GAB3 | 153979491 | cg19638040 | CASK | 41782196 |
| cg01377936 | DMD | 32173560 | cg10028049 | AMOT | 112067706 | cg19639792 | RGN | 46936504 |

EP 4 495 263 A1

32

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01379263 | STARD8 | 67867130 | cg10039267 | BCOR | 39960821 | cg19658849 | SLC25A43 | 118533119 |
| cg01381545 | GPR64 | 19140538 | cg10047502 | WDR44 | 117479900 | cg19680277 | TIMM8A | 100601582 |
| cg01384686 | HTATSF1 | 135579487 | cg10055097 | MAGIX | 49020084 | cg19681107 | FAM122C | 133940383 |
| cg01397290 | PRRG1 | 37208295 | cg10055173 | PLP2 | 49028100 | cg19687016 | ARMCX1 | 100805492 |
| cg01408383 | PIGA | 15353254 | cg10055320 | BCOR | 40009982 | cg19688503 | CAPN6 | 110513941 |
| cg01409685 | RAP2C | 131347226 | cg10058598 | NDUFB11 | 47001685 | cg19696622 | RPS6KA3 | 20285908 |
| cg01410230 | RPL36A | 100645860 | cg10063227 | DGKK | 50211229 | cg19721057 | AR | 66788395 |
| cg01413240 | CHIC1 | 72880337 | cg10064814 | CXorf40B | 149101909 | cg19723705 | AR | 66799850 |
| cg01413291 | TMEM187 | 153247841 | cg10066684 | TBC1D8B | 106044844 | cg19731655 | MAP7D2 | 20134778 |
| cg01430241 | GDI1 | 153665714 | cg10070741 | IDH3G | 153056291 | cg19736647 | AIFM1 | 129299788 |
| cg01434723 | UBL4A | 153715087 | cg10072574 | MPP1 | 154033934 | cg19750917 | MMGT1 | 135052066 |
| cg01445307 | SMS | 21959520 | cg10076009 | SMARCA1 | 128656725 | cg19753067 | SLC25A43 | 118536722 |
| cg01448525 | DLG3 | 69673805 | cg10078687 | KIAA2022 | 74144469 | cg19754833 | CHRDL1 | 110039080 |
| cg01460974 | ARHGEF9 | 62975657 | cg10082114 | ATRX | 77041194 | cg19770547 | NKAP | 119077802 |
| cg01466576 | APLN | 128787126 | cg10088372 | PHF6 | 133507354 | cg19774450 | ACOT9 | 23721943 |
| cg01473525 | RAB9B | 103085819 | cg10090585 | ENOX2 | 130037302 | cg19777864 | CHRDL1 | 110039082 |
| cg01477427 | HSD17B10 | 53461421 | cg10091155 | CYSLTR1 | 77584550 | cg19778009 | SLC9A6 | 135068018 |
| cg01479413 | ARHGEF6 | 135849691 | cg10098570 | OTUD5 | 48794377 | cg19780684 | ASB9 | 15288115 |
| cg01483252 | CHST7 | 46433132 | cg10101479 | PRAF2 | 48931790 | cg19780807 | C1GALT1C1 | 119763998 |
| cg01488378 | SLC35A2 | 48769307 | cg10102167 | PGRMC1 | 118370063 | cg19786359 | SYP | 49049363 |
| cg01490258 | BCOR | 40030456 | cg10105723 | FAM120C | 54206411 | cg19787517 | PRRG1 | 37209139 |
| cg01494339 | THOC2 | 122864788 | cg10105844 | PAK3 | 110339372 | cg19790752 | MID1 | 10852973 |
| cg01498090 | TLR7 | 12885115 | cg10143752 | PJA1 | 68382925 | cg19791442 | GNL3L | 54556538 |
| cg01514943 | BCOR | 40034026 | cg10149996 | PGRMC1 | 118369686 | cg19792266 | ZDHHC15 | 74743023 |
| cg01522249 | BCOR | 39951640 | cg10153260 | EDA2R | 65818628 | cg19793470 | PHKA2 | 19000406 |
| cg01528616 | GPKOW | 48979067 | cg10163050 | DNASE1L1 | 153637650 | cg19800913 | FAM127B | 134186228 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01529679 | PDZD4 | 153074551 | cg10164191 | AR | 66763521 | cg19818925 | ARMCX4 | 100677490 |
| cg01540916 | ASB9 | 15288755 | cg10169869 | BCOR | 40033636 | cg19834421 | SNX12 | 70288180 |
| cg01542035 | DMD | 31285501 | cg10170893 | ZNF630 | 47931142 | cg19836906 | UPF3B | 118987328 |
| cg01556010 | UPRT | 74493847 | cg10201390 | DYNLT3 | 37706936 | cg19839614 | DGKK | 50212149 |
| cg01559064 | CUL4B | 119710388 | cg10208578 | SLC35A2 | 48764871 | cg19854896 | TSC22D3 | 106959898 |
| cg01564045 | IDS | 148583595 | cg10208654 | FHL1 | 135230613 | cg19855812 | ABCB7 | 74376046 |
| cg01564693 | PJA1 | 68381877 | cg10212199 | GPRASP2 | 101966585 | cg19860691 | SLC9A6 | 135068355 |
| cg01577745 | NGFRAP1 | 102631077 | cg10237576 | WDR13 | 48455705 | cg19867709 | ZNF41 | 47342478 |
| cg01579322 | FAM120C | 54209148 | cg10246296 | CLCN4 | 10124977 | cg19868577 | KLHL15 | 24045303 |
| cg01598357 | DLG3 | 69664317 | cg10256662 | HPRT1 | 133594827 | cg19885057 | FAM58A | 152863995 |
| cg01600123 | DLG3 | 69672435 | cg10258730 | CLDN2 | 106142861 | cg19888047 | CHM | 85302610 |
| cg01600263 | TCEAL4 | 102840724 | cg10274815 | EFNB1 | 68048015 | cg19899539 | CD99L2 | 150067102 |
| cg01632517 | SSX8 | 52652796 | cg10277365 | MAGIX | 49022793 | cg19908985 | NYX | 41305937 |
| cg01634761 | EFNB1 | 68048386 | cg10286829 | ARMCX2 | 100913838 | cg19911179 | MCTS1 | 119737695 |
| cg01647019 | GLRA2 | 14547618 | cg10288106 | CITED1 | 71526903 | cg19912147 | FAM122C | 133955434 |
| cg01663055 | OPHN1 | 67649784 | cg10290730 | ATP2B3 | 152801187 | cg19922860 | COL4A5 | 107799664 |
| cg01668271 | ZNF81 | 47696333 | cg10293293 | ACOT9 | 23761105 | cg19926635 | KCND1 | 48819020 |
| cg01669374 | PRPS2 | 12809328 | cg10301657 | ZNF185 | 152083021 | cg19928247 | TCEAL1 | 102883794 |
| cg01674874 | MAGIX | 49017903 | cg10306780 | TBC1D25 | 48398418 | cg19933709 | SLC6A14 | 115567849 |
| cg01698523 | FLNA | 153583936 | cg10308137 | SAT1 | 23801074 | cg19935097 | ARHGAP6 | 11476834 |
| cg01716666 | NKAP | 119077648 | cg10310824 | SAT1 | 23801995 | cg19937198 | FAM3A | 153745361 |
| cg01717812 | ABCB7 | 74376285 | cg10318351 | GNL3L | 54556330 | cg19937286 | BCOR | 40017092 |
| cg01725669 | SLC25A14 | 129507302 | cg10320061 | PDZD4 | 153094967 | cg19937618 | TFE3 | 48901183 |
| cg01730928 | KIAA2022 | 74143801 | cg10321928 | TIMM8A | 100604176 | cg19944582 | DKC1 | 153990954 |
| cg01733439 | PRICKLE3 | 49042949 | cg10347293 | ATP6AP2 | 40440170 | cg19948991 | RBMX | 135961208 |
| cg01737010 | TMSB15A | 101772014 | cg10349721 | ZNF275 | 152613463 | cg19949137 | HTATSF1 | 135579980 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01737421 | PORCN | 48367069 | cg10403109 | VMA21 | 150565864 | cg19953825 | DDX26B | 134682062 |
| cg01742164 | CD99L2 | 149935904 | cg10417559 | PRICKLE3 | 49043355 | cg19992190 | MBTPS2 | 21857624 |
| cg01742836 | FHL1 | 135229755 | cg10418630 | GPC4 | 132547691 | cg19995126 | OCRL | 128674157 |
| cg01747155 | TMLHE | 154842516 | cg10420746 | GLUD2 | 120181090 | cg19997062 | SLITRK2 | 144900177 |
| cg01752898 | RPGR | 38186917 | cg10421300 | RAB9B | 103087077 | cg19998137 | STARD8 | 67913607 |
| cg01755224 | MID1 | 10586775 | cg10423328 | SATL1 | 84364131 | cg20001978 | NAP1L2 | 72434661 |
| cg01755617 | RPL10 | 153626633 | cg10425032 | CUL4B | 119694959 | cg20010130 | HSD17B10 | 53461462 |
| cg01756638 | VBP1 | 154444356 | cg10426500 | MMGT1 | 135055672 | cg20013240 | ATP1B4 | 119495923 |
| cg01760027 | CLCN5 | 49832257 | cg10432310 | FUNDC2 | 154255950 | cg20015269 | CLCN5 | 49687166 |
| cg01771673 | RBM41 | 106361539 | cg10446009 | PRPS1 | 106871260 | cg20018767 | PAK3 | 110339485 |
| cg01773478 | CHST7 | 46434442 | cg10448608 | SLITRK2 | 144903748 | cg20020143 | SH3KBP1 | 19905497 |
| cg01780361 | UBQLN2 | 56590386 | cg10455133 | ARAF | 47420508 | cg20024850 | MCF2 | 138774368 |
| cg01796247 | SLITRK2 | 144901935 | cg10460864 | PDHA1 | 19362102 | cg20025403 | ARHGEF6 | 135850058 |
| cg01798163 | PLXNA3 | 153691946 | cg10462174 | PIN4 | 71462220 | cg20034238 | DMD | 33038967 |
| cg01798375 | CFP | 47489719 | cg10465607 | CXorf40A | 148626178 | cg20051589 | DLG3 | 69664482 |
| cg01804836 | BEX2 | 102565738 | cg10476451 | CDKL5 | 18517817 | cg20056133 | CSTF2 | 100075233 |
| cg01807728 | HMGB3 | 150151624 | cg10494806 | GPKOW | 48980610 | cg20062122 | CLCN5 | 49833742 |
| cg01809408 | TSPYL2 | 53112501 | cg10500026 | MTMR1 | 149861761 | cg20069969 | DRP2 | 100517496 |
| cg01813276 | GLA | 100659166 | cg10501443 | LRCH2 | 114468791 | cg20074774 | TSPAN7 | 38420780 |
| cg01814235 | ARAF | 47422454 | cg10503697 | KLHL13 | 117102168 | cg20077179 | FLNA | 153585922 |
| cg01816615 | AMOT | 112066190 | cg10509982 | RAB41 | 69502031 | cg20085077 | ARMCX4 | 100740421 |
| cg01817230 | FLNA | 153594995 | cg10512285 | RAB39B | 154493754 | cg20085561 | TSC22D3 | 106960348 |
| cg01818727 | GPR64 | 19137817 | cg10513418 | ARMCX1 | 100805297 | cg20104776 | LDOC1 | 140271176 |
| cg01821772 | RBM10 | 47007440 | cg10519228 | ATP6AP1 | 153656860 | cg20114929 | OPHN1 | 67353125 |
| cg01825872 | OPHN1 | 67653324 | cg10526888 | MAGT1 | 77151237 | cg20119635 | DYNLT3 | 37707074 |
| cg01828434 | TSC22D3 | 106960491 | cg10530128 | SLC35A2 | 48769550 | cg20134598 | TRO | 54947308 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01831199 | RBM41 | 106362045 | cg10530733 | GPR173 | 53078631 | cg20154346 | RAI2 | 17821097 |
| cg01831944 | DIAPH2 | 95939323 | cg10536534 | MCTS1 | 119737767 | cg20197277 | MBNL3 | 131508512 |
| cg01832419 | KLHL15 | 24043059 | cg10538243 | DUSP9 | 152910068 | cg20197861 | BCOR | 39964229 |
| cg01834528 | REPS2 | 17167719 | cg10542519 | PLP2 | 49029384 | cg20203466 | PGK1 | 77359924 |
| cg01850541 | STARD8 | 67867448 | cg10549497 | MAP7 D3 | 135300739 | cg20207108 | GPRASP1 | 101906181 |
| cg01851385 | CLCN4 | 10125135 | cg10552586 | CXorf56 | 118673612 | cg20207989 | TREX2 | 152712221 |
| cg01852992 | PLXNA3 | 153695658 | cg10559220 | ELF4 | 129246153 | cg20208052 | ABCB7 | 74376828 |
| cg01864032 | DLG3 | 69674332 | cg10560144 | PGK1 | 77359355 | cg20208613 | AMMECR1 | 109561147 |
| cg01868753 | RBM41 | 106361886 | cg10575236 | REPS2 | 16964741 | cg20216854 | SH3KBP1 | 19817872 |
| cg01873087 | TBC1D25 | 48397836 | cg10581449 | NUDT11 | 51238781 | cg20219360 | WDR13 | 48455861 |
| cg01881753 | HAUS7 | 152733671 | cg10584819 | HPRT1 | 133593670 | cg20220865 | AWAT1 | 69454387 |
| cg01882566 | RPGR | 38187196 | cg10587888 | HEPH | 65390189 | cg20244073 | MIDI | 10851627 |
| cg01884384 | LONRF3 | 118108832 | cg10591659 | NYX | 41306768 | cg20258741 | PDK3 | 24482846 |
| cg01885202 | CDKL5 | 18443457 | cg10645377 | ATP6AP1 | 153659893 | cg20259518 | EFNB1 | 68048749 |
| cg01886630 | REPS2 | 16964556 | cg10649042 | SASH3 | 128913786 | cg20262028 | USP11 | 47107481 |
| cg01887353 | ZNF41 | 47342480 | cg10659052 | NKRF | 118724537 | cg20262712 | KLHL13 | 117120608 |
| cg01905377 | FOXP3 | 49122718 | cg10670396 | GPRASP1 | 101906537 | cg20265337 | AR | 66770278 |
| cg01906946 | BCOR | 39951219 | cg10673213 | MORC4 | 106243477 | cg20274188 | WNK3 | 54300963 |
| cg01913024 | AIFM1 | 129300230 | cg10674511 | LPAR4 | 78003091 | cg20275344 | RAB9B | 103087153 |
| cg01916066 | KLHL13 | 117032390 | cg10694252 | FRMPD4 | 12601729 | cg20279598 | PDZD4 | 153096506 |
| cg01924074 | HUWE1 | 53711167 | cg10703213 | RBMX | 135963580 | cg20281601 | CDKL5 | 18443740 |
| cg01924982 | G6PD | 153760139 | cg10706590 | CITED1 | 71525891 | cg20287862 | ITM2A | 78623956 |
| cg01927625 | RAB41 | 69502017 | cg10712678 | BHLHB9 | 102005972 | cg20289741 | ZDHHC9 | 128977498 |
| cg01932223 | UTP14A | 129040950 | cg10717149 | SLC25A14 | 129473838 | cg20291091 | ARMCX1 | 100806034 |
| cg01938887 | HTATSF1 | 135579106 | cg10721440 | ACSL4 | 108976619 | cg20308511 | SAT1 | 23801056 |
| cg01948589 | RPS6KA3 | 20285432 | cg10723556 | PPP1R3F | 49125646 | cg20329959 | DNASE1L1 | 153637607 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg01951307 | FAM122C | 133940723 | cg10729169 | DLG3 | 69664722 | cg20333676 | GPKOW | 48980132 |
| cg01954531 | MID11P1 | 38660667 | cg10743239 | IGSF1 | 130423167 | cg20336701 | DUSP9 | 152907540 |
| cg01960534 | PHF16 | 46771154 | cg10775576 | CLCN4 | 10124333 | cg20338399 | BCOR | 39943770 |
| cg01970554 | DGKK | 50212323 | cg10783042 | HDAC6 | 48660813 | cg20340631 | PHKA1 | 71933682 |
| cg01991530 | MBTPS2 | 21857632 | cg10789170 | SMPX | 21762904 | cg20344426 | VBP1 | 154444619 |
| cg01992935 | KCNE1L | 108868271 | cg10811052 | FAAH2 | 57311872 | cg20346189 | CITED1 | 71524766 |
| cg01996818 | MST4 | 131157562 | cg10818284 | SYP | 49056605 | cg20348344 | BCOR | 39951468 |
| cg01999076 | RP2 | 46696420 | cg10840597 | ZNF81 | 47777671 | cg20359784 | FAM127B | 134186334 |
| cg01999212 | EFNB1 | 68047865 | cg10848980 | PRAF2 | 48931872 | cg20367266 | TCEAL7 | 102585123 |
| cg02004156 | TFE3 | 48900769 | cg10851587 | ATP2B3 | 152818579 | cg20371650 | PRAF2 | 48931388 |
| cg02007259 | GATA1 | 48645037 | cg10856819 | BCOR | 40028998 | cg20372775 | FGD1 | 54522709 |
| cg02012821 | DIAPH2 | 95940519 | cg10858077 | FOXP3 | 49121205 | cg20417134 | TCEAL2 | 101381880 |
| cg02014085 | FAM120C | 54097113 | cg10860619 | PHF16 | 46771853 | cg20420363 | TFE3 | 48901149 |
| cg02019068 | FTSJ1 | 48337472 | cg10861555 | ENOX2 | 130036945 | cg20421037 | MST4 | 131157627 |
| cg02023473 | HMGB3 | 150150939 | cg10867226 | OPHN1 | 67352731 | cg20430749 | NUDT10 | 51075273 |
| cg02026371 | NUDT10 | 51074224 | cg10869581 | BHLHB9 | 102000905 | cg20431201 | TREX2 | 152712110 |
| cg02031392 | PCYT1B | 24691135 | cg10873964 | XIAP | 122993706 | cg20435542 | IL13RA1 | 117862176 |
| cg02031980 | PIN4 | 71401203 | cg10894453 | ITM2A | 78622859 | cg20439892 | NGFRAP1 | 102631453 |
| cg02033323 | FOXP3 | 49118313 | cg10900417 | PAK3 | 110188397 | cg20442640 | DGKK | 50213745 |
| cg02035698 | ZNF185 | 152126884 | cg10902968 | PRRG1 | 37209388 | cg20445038 | VMA21 | 150565466 |
| cg02035866 | PHKA2 | 19002442 | cg10906138 | CHST7 | 46433744 | cg20448001 | TSPAN7 | 38424760 |
| cg02059755 | HEPH | 65383371 | cg10910730 | ARHGAP6 | 11497215 | cg20450764 | TSPAN7 | 38420326 |
| cg02062238 | RPGR | 38186830 | cg10912974 | TMEM185A | 148713660 | cg20453549 | FAM127A | 134166287 |
| cg02077027 | SH3KBP1 | 19817931 | cg10914789 | DUSP9 | 152909097 | cg20455959 | MTMR1 | 149861947 |
| cg02095003 | ZNF275 | 152599890 | cg10950266 | ARMCX4 | 100673192 | cg20456193 | ATP2B3 | 152801636 |
| cg02095995 | MCF2 | 138774239 | cg10950297 | EFNB1 | 68049085 | cg20457190 | BCOR | 40024696 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02112649 | RPS6KA6 | 83442333 | cg10950413 | UBQLN2 | 56590097 | cg20477705 | ARHGAP6 | 11682132 |
| cg02116333 | HSD17B10 | 53461433 | cg10956264 | ZDHHC9 | 128977299 | cg20494209 | IRAKI | 153282222 |
| cg02117996 | MID11P1 | 38660490 | cg10978544 | PIGA | 15339848 | cg20495737 | SLC38A5 | 48327768 |
| cg02120013 | DGKK | 50214405 | cg10978884 | AMMECR1 | 109561384 | cg20497504 | TMEM35 | 100334897 |
| cg02121672 | PCYT1B | 24665932 | cg10981178 | ZBTB33 | 119385541 | cg20502006 | ZNF185 | 152082354 |
| cg02124059 | ZNF41 | 47341898 | cg10982861 | TSC22D3 | 107018095 | cg20503915 | DUSP9 | 152912606 |
| cg02127807 | LRCH2 | 114468833 | cg10983111 | SYP | 49056686 | cg20504202 | TSR2 | 54467052 |
| cg02129889 | RPL39 | 118925789 | cg10987536 | HUWE1 | 53711084 | cg20512370 | NRK | 105066357 |
| cg02144050 | TBC1D25 | 48397520 | cg10990357 | SLC6A14 | 115592030 | cg20517444 | PIGA | 15353854 |
| cg02148711 | ATRX | 77041559 | cg10991108 | WDR45 | 48958414 | cg20521035 | MIDI | 10645846 |
| cg02151408 | RS1 | 18690814 | cg10995081 | GRIPAP1 | 48858587 | cg20522855 | DMD | 31889692 |
| cg02151779 | SLC38A5 | 48327875 | cg10999701 | DIAPH2 | 95940170 | cg20540913 | EDA | 68836108 |
| cg02155838 | SLC9A6 | 135067201 | cg11005845 | LDOC1 | 140271314 | cg20546893 | CXorf67 | 51149573 |
| cg02156782 | PPP1R3F | 49126099 | cg11014998 | ATP6AP2 | 40439740 | cg20556598 | RS1 | 18691243 |
| cg02161125 | CASK | 41782692 | cg11019890 | ZNF185 | 152113691 | cg20557008 | HEPH | 65384237 |
| cg02161824 | FAM127B | 134186388 | cg11021247 | SYN1 | 47479625 | cg20557017 | CITED1 | 71528050 |
| cg02161919 | PHKA2 | 19002980 | cg11022804 | BGN | 152760227 | cg20563277 | APOOL | 84258823 |
| cg02166601 | GPR64 | 19140966 | cg11028627 | VMA21 | 150564821 | cg20585410 | DCX | 110654569 |
| cg02168925 | CHM | 85226163 | cg11031647 | DCX | 110654011 | cg20589243 | TMEM164 | 109246229 |
| cg02169289 | BCOR | 39967202 | cg11036290 | EFNB1 | 68060375 | cg20590161 | FAAH2 | 57313197 |
| cg02177607 | SASH3 | 128924261 | cg11036643 | UPRT | 74493779 | cg20592837 | ZNF81 | 47695927 |
| cg02179029 | RBM41 | 106359455 | cg11049154 | ELF4 | 129244816 | cg20593767 | ARMCX1 | 100804036 |
| cg02179438 | CCDC22 | 49091760 | cg11049305 | ZC4H2 | 64196751 | cg20641280 | LAGE3 | 153707793 |
| cg02179880 | WDR13 | 48456308 | cg11049634 | BCOR | 40016121 | cg20651980 | BCAP31 | 152987922 |
| cg02183297 | KCNE1L | 108867379 | cg11049750 | ASB9 | 15289615 | cg20653018 | FAM127A | 134165954 |
| cg02184147 | AIFM1 | 129300964 | cg11049774 | BEX4 | 102469996 | cg20662859 | HMGB3 | 150151609 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02185190 | SLC25A43 | 118534462 | cg11072201 | ACSL4 | 108976163 | cg20662913 | PDZD4 | 153095300 |
| cg02187432 | SCML2 | 18371852 | cg11080085 | WDR44 | 117480243 | cg20664065 | PHKA1 | 71933616 |
| cg02193580 | FHL1 | 135251444 | cg11081453 | ZNF185 | 152081948 | cg20687573 | UXT | 47519898 |
| cg02198544 | TREX2 | 152711731 | cg11081925 | RBMX2 | 129536117 | cg20731167 | TBC1D25 | 48397760 |
| cg02200373 | SASH3 | 128913578 | cg11082424 | BEX4 | 102470047 | cg20746035 | PRICKLE3 | 49042873 |
| cg02201836 | UTP14A | 129039832 | cg11085564 | P2RY4 | 69479839 | cg20749047 | DUSP9 | 152906772 |
| cg02211869 | ZNF449 | 134481233 | cg11102619 | RPS6KA6 | 83443094 | cg20749341 | LONRF3 | 118108666 |
| cg02212061 | NUDT11 | 51240339 | cg11105907 | GPC4 | 132545123 | cg20757997 | TIMM17B | 48751693 |
| cg02212907 | FAM104B | 55188884 | cg11108456 | CLCN5 | 49685776 | cg20767433 | PRPS1 | 106871562 |
| cg02223323 | MAP7 D2 | 20134399 | cg11111083 | ELK1 | 47510589 | cg20767561 | ACSL4 | 108976811 |
| cg02225164 | GAB3 | 153979588 | cg11111131 | CXorf57 | 105855108 | cg20767845 | SASH3 | 128926467 |
| cg02232922 | SLC25A43 | 118532809 | cg11113650 | CCNB3 | 50028082 | cg20780119 | EDA | 68836036 |
| cg02239640 | GSPT2 | 51487529 | cg11116781 | STAG2 | 123094384 | cg20781383 | RPL10 | 153626527 |
| cg02264182 | IDS | 148586734 | cg11117255 | BCOR | 40008202 | cg20786173 | MAP7 D2 | 20135761 |
| cg02264284 | HPRT1 | 133594951 | cg11132751 | CXorf67 | 51149970 | cg20786418 | KLHL13 | 117251000 |
| cg02265919 | CSTF2 | 100075240 | cg11138004 | FUNDC2 | 154283466 | cg20787451 | GPR173 | 53093414 |
| cg02272668 | ATG4A | 107338894 | cg11139821 | HAUS7 | 152736746 | cg20789492 | CXorf67 | 51149435 |
| cg02274705 | C1GALT1C1 | 119763815 | cg11143827 | BCOR | 39961764 | cg20790618 | ZDHHC9 | 128978347 |
| cg02275016 | FRMPD4 | 12739263 | cg11152253 | WDR45 | 48958091 | cg20792978 | ZDHHC9 | 128977934 |
| cg02275924 | MIDI | 10851874 | cg11153610 | PDZD4 | 153069926 | cg20793193 | SYP | 49056861 |
| cg02285254 | FHL1 | 135229382 | cg11164993 | SNX12 | 70288706 | cg20816612 | PHKA1 | 71933629 |
| cg02286623 | RAI2 | 17879424 | cg11165443 | STARD8 | 67943585 | cg20825323 | IGBP1 | 69353047 |
| cg02291897 | ELK1 | 47509947 | cg11165479 | SYP | 49056505 | cg20832009 | STARD8 | 67867718 |
| cg02295504 | ARMCX3 | 100878362 | cg11179629 | MMGT1 | 135056311 | cg20842040 | PRPS2 | 12809608 |
| cg02301364 | PRPS1 | 106871826 | cg11184796 | CCDC22 | 49091986 | cg20847962 | HCCS | 11133043 |
| cg02318959 | RBMX2 | 129535800 | cg11185978 | PRDX4 | 23685722 | cg20855303 | REPS2 | 16965405 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02326006 | ARHGEF9 | 62974433 | cg11186637 | RPL39 | 118925725 | cg20857470 | MIDI | 10588738 |
| cg02327549 | NRK | 105066292 | cg11186981 | HMGB3 | 150151784 | cg20858239 | MAGED2 | 54834222 |
| cg02329520 | FAM3A | 153740317 | cg11189761 | AR | 66763113 | cg20859738 | SLC25A14 | 129473846 |
| cg02333283 | LAGE3 | 153707099 | cg11190637 | TSPYL2 | 53112003 | cg20860642 | TLR7 | 12883857 |
| cg02337670 | OTUD5 | 48815787 | cg11193681 | UPF3B | 118986581 | cg20869203 | LHFPL1 | 111923211 |
| cg02345317 | NLGN3 | 70364543 | cg11194545 | NRK | 105066793 | cg20878850 | AR | 66763685 |
| cg02352295 | SLITRK2 | 144901807 | cg11233153 | SLC10A3 | 153718691 | cg20878864 | HUWE1 | 53713844 |
| cg02354343 | RAP2C | 131351392 | cg11254749 | FAM3A | 153736252 | cg20881335 | GLRA2 | 14547172 |
| cg02360578 | CXorf57 | 105855130 | cg11264711 | OPHN1 | 67653315 | cg20887366 | ATP6AP2 | 40440209 |
| cg02366635 | MTM1 | 149840937 | cg11267664 | WDR13 | 48457256 | cg20922422 | PCYT1B | 24665669 |
| cg02370478 | MORC4 | 106241676 | cg11271203 | MAGED1 | 51636206 | cg20931907 | CXorf67 | 51149742 |
| cg02376524 | GPRASP2 | 101967145 | cg11271681 | APLN | 128788936 | cg20945035 | SPIN3 | 57022192 |
| cg02379437 | NAP1L2 | 72435117 | cg11272332 | ZDHHC15 | 74742810 | cg20947407 | IGSF1 | 130418544 |
| cg02386310 | PORCN | 48367223 | cg11280668 | SCML1 | 17754811 | cg20948364 | LASLL | 64754701 |
| cg02395364 | UPRT | 74493592 | cg11281291 | ARHGEF9 | 62975200 | cg20951645 | BGN | 152774831 |
| cg02396917 | CXorf23 | 19988369 | cg11281673 | LAMP2 | 119603053 | cg20958732 | MBNL3 | 131624084 |
| cg02417823 | SMS | 21958684 | cg11286196 | PRAF2 | 48931623 | cg20964758 | ENOX2 | 130037157 |
| cg02419467 | ZNF630 | 47929088 | cg11286294 | FAM127B | 134185321 | cg20969392 | PDZD4 | 153070144 |
| cg02424254 | SLC25A43 | 118587302 | cg11290168 | MAGT1 | 77151316 | cg20972834 | ABCB7 | 74375795 |
| cg02433927 | BCOR | 39969103 | cg11291009 | ARHGEF9 | 62975067 | cg20973408 | GNL3L | 54556365 |
| cg02437314 | CITED1 | 71521494 | cg11304846 | BCORL1 | 129189315 | cg20976488 | PORCN | 48370301 |
| cg02438576 | PLXNA3 | 153697531 | cg11308037 | FAM3A | 153744609 | cg20977592 | OTUD5 | 48779608 |
| cg02442693 | GPC3 | 133118132 | cg11311049 | SSX8 | 52651893 | cg20979081 | MOSPD2 | 14939309 |
| cg02456261 | WDR44 | 117479353 | cg11315455 | XIAP | 122997585 | cg20979765 | UBQLN2 | 56590537 |
| cg02457752 | CDR1 | 139868078 | cg11320369 | BCOR | 40026673 | cg20983329 | MED12 | 70338670 |
| cg02459704 | PDZD4 | 153084910 | cg11320810 | IL13RA1 | 117861215 | cg20999651 | FHL1 | 135230594 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02469604 | TKTL1 | 153533342 | cg11321526 | LASLL | 64751905 | cg21010298 | BCOR | 40007065 |
| cg02471578 | NLGN3 | 70364539 | cg11322406 | LRCH2 | 114468360 | cg21016188 | FAM50A | 153672702 |
| cg02480419 | OTUD5 | 48815856 | cg11331191 | ASB12 | 63446644 | cg21017771 | CLDN2 | 106143406 |
| cg02492740 | UBQLN2 | 56590362 | cg11337025 | RBM41 | 106361672 | cg21019788 | SUV39H1 | 48555509 |
| cg02496423 | SMS | 21958619 | cg11337462 | ABCB7 | 74377604 | cg21021766 | PCYT1B | 24691075 |
| cg02497062 | IGBP1 | 69352975 | cg11340371 | ABCB7 | 74376241 | cg21030483 | TMEM185A | 148713678 |
| cg02510708 | STAG2 | 123095692 | cg11344644 | TIMM8A | 100603699 | cg21039523 | BEX4 | 102469724 |
| cg02512611 | SHROOM4 | 50557800 | cg11371160 | UBL4A | 153714615 | cg21045917 | ALAS2 | 55057395 |
| cg02539591 | PDK3 | 24483092 | cg11390763 | RPL36A | 100648809 | cg21046413 | AIFM1 | 129299367 |
| cg02540826 | PHKA2 | 19002582 | cg11395386 | MECP2 | 153360157 | cg21049587 | RGN | 46938148 |
| cg02547323 | RAB9B | 103087380 | cg11395955 | SAT1 | 23801087 | cg21067846 | MORC4 | 106228404 |
| cg02549418 | MBNL3 | 131573634 | cg11424273 | DLG3 | 69668937 | cg21080294 | PRPS1 | 106871478 |
| cg02559685 | MID11P1 | 38663484 | cg11425349 | MORF4L2 | 102943712 | cg21109542 | SLC10A3 | 153719397 |
| cg02564124 | CXorf36 | 45020057 | cg11426662 | ZNF185 | 152127457 | cg21112796 | ATP2B3 | 152801238 |
| cg02573613 | OTUD5 | 48814806 | cg11434986 | BCOR | 39948609 | cg21113886 | MTM1 | 149737755 |
| cg02579620 | BCAP31 | 152988658 | cg11441847 | IL13RA1 | 117861165 | cg21114303 | PHF8 | 54069147 |
| cg02580986 | MUM1L1 | 105421602 | cg11442732 | GPC3 | 133118088 | cg21114806 | DUSP9 | 152909208 |
| cg02581653 | USP11 | 47092037 | cg11452781 | GAB3 | 153979598 | cg21118250 | MIDI | 10589926 |
| cg02582887 | FAM58A | 152860531 | cg11466638 | MAP7 D2 | 20060410 | cg21120047 | XPNPEP2 | 128872961 |
| cg02583883 | AMOT | 112083504 | cg11468748 | IDS | 148587019 | cg21127593 | PHF6 | 133507742 |
| cg02587153 | UBQLN2 | 56589768 | cg11469866 | APOOL | 84258483 | cg21128398 | FRMPD4 | 12600986 |
| cg02589074 | IDS | 148586938 | cg11471507 | RPS6KA3 | 20283423 | cg21131239 | ABCB7 | 74376811 |
| cg02590287 | CLDN2 | 106161451 | cg11472424 | PFKFB1 | 55020450 | cg21139772 | PDHA1 | 19362100 |
| cg02590710 | KLF8 | 56258440 | cg11475300 | CLCN4 | 10128853 | cg21153181 | DNASE1L1 | 153637497 |
| cg02600622 | LHFPL1 | 111885771 | cg11479591 | MECP2 | 153362176 | cg21154114 | SLC16A2 | 73752397 |
| cg02602182 | BCOR | 40026844 | cg11482392 | DIAPH2 | 96854396 | cg21155787 | RBM41 | 106361899 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02602528 | PLP2 | 49028148 | cg11494656 | ARR3 | 69487197 | cg21156383 | HSD17B10 | 53461271 |
| cg02604836 | MPP1 | 154032629 | cg11506821 | MECP2 | 153362123 | cg21158178 | PHF8 | 54070874 |
| cg02611122 | TMEM187 | 153247499 | cg11509903 | HTATSF1 | 135580080 | cg21159768 | MAGED2 | 54834846 |
| cg02616921 | AWAT1 | 69455617 | cg11530701 | ARHGAP6 | 11157610 | cg21161328 | ZNF41 | 47342498 |
| cg02621658 | STARD8 | 67867676 | cg11538407 | ZNF41 | 47305922 | cg21179255 | BGN | 152772707 |
| cg02623256 | PDK3 | 24483231 | cg11550332 | TSC22D3 | 106960273 | cg21180513 | BCOR | 39947231 |
| cg02623604 | CD99L2 | 150067276 | cg11555315 | TSPYL2 | 53115787 | cg21183872 | LONRF3 | 118108506 |
| cg02624484 | KLHL15 | 24043392 | cg11565248 | RPS6KA3 | 20284128 | cg21184454 | SH3KBP1 | 19765879 |
| cg02625237 | AR | 66793272 | cg11565980 | RAB33A | 129305480 | cg21190253 | DMD | 33231153 |
| cg02626719 | ARMCX2 | 100914838 | cg11590714 | CLCN5 | 49832148 | cg21195120 | MBTPS2 | 21858304 |
| cg02629615 | DMD | 33146500 | cg11593337 | AIFM1 | 129272595 | cg21197919 | SLC38A5 | 48328694 |
| cg02634083 | HMGB3 | 150153136 | cg11598929 | FAAH2 | 57313075 | cg21198428 | PGRMC1 | 118370278 |
| cg02634916 | THOC2 | 122866442 | cg11609760 | FGD1 | 54522363 | cg21202708 | MTMR1 | 149861743 |
| cg02645343 | DDX26B | 134656105 | cg11616438 | GK | 30671316 | cg21205654 | LONRF3 | 118110766 |
| cg02647042 | ARHGEF6 | 135849065 | cg11620557 | BCOR | 39963545 | cg21206285 | OTUD5 | 48814935 |
| cg02649608 | ZBTB33 | 119384792 | cg11631710 | GPKOW | 48979845 | cg21209948 | TMEM164 | 109245514 |
| cg02671603 | KIAA2022 | 74146242 | cg11633007 | KLF8 | 56310955 | cg21224730 | STAG2 | 123220554 |
| cg02685544 | BCOR | 39956797 | cg11633086 | AIFM1 | 129299950 | cg21224759 | PIN4 | 71401335 |
| cg02690554 | CITED1 | 71527606 | cg11637006 | SH3KBP1 | 19905535 | cg21232685 | GPRASP1 | 101909064 |
| cg02693068 | BCOR | 39941630 | cg11640914 | FAM50A | 153675452 | cg21248478 | CLIC2 | 154563968 |
| cg02698615 | HTATSF1 | 135580098 | cg11641061 | MORF4L2 | 102941859 | cg21253375 | MAP7 D2 | 20130615 |
| cg02710173 | FLNA | 153603482 | cg11645133 | FLNA | 153590634 | cg21258987 | GSPT2 | 51485834 |
| cg02714492 | EDA | 68836610 | cg11647489 | ZDHHC9 | 128977488 | cg21265341 | MAGEB16 | 35816432 |
| cg02725692 | PORCN | 48367969 | cg11650160 | BCOR | 40017883 | cg21267792 | PIM2 | 48776812 |
| cg02733596 | LAMP2 | 119604444 | cg11653314 | RPS6KA3 | 20285129 | cg21284034 | TCEAL1 | 102883667 |
| cg02734755 | HUWE1 | 53710671 | cg11653864 | ELK1 | 47509884 | cg21284636 | PIN4 | 71401009 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02742918 | IRAK1 | 153284103 | cg11654517 | ARHGEF6 | 135849165 | cg21290775 | TSR2 | 54466879 |
| cg02742990 | PCYT1B | 24665458 | cg11655867 | FRMPD4 | 12513550 | cg21293846 | CENPI | 100356086 |
| cg02752267 | DLG3 | 69671886 | cg11656852 | KIAA2022 | 74146608 | cg21294096 | AMMECR1 | 109564207 |
| cg02758183 | FIGF | 15376140 | cg11661234 | RLIM | 73834596 | cg21305413 | PORCN | 48368136 |
| cg02763888 | ZNF75D | 134427883 | cg11662345 | HEPH | 65381074 | cg21323637 | FLNA | 153600494 |
| cg02764898 | REPS2 | 16966893 | cg11667509 | SLC10A3 | 153719406 | cg21327343 | KLF8 | 56258579 |
| cg02770249 | LAMP2 | 119603233 | cg11673471 | ZDHHC9 | 128977578 | cg21327581 | SYN1 | 47479500 |
| cg02772106 | ABCD1 | 152992265 | cg11675904 | ARAF | 47420403 | cg21337832 | PCYT1B | 24665655 |
| cg02772823 | SLC6A14 | 115567582 | cg11681617 | GAB3 | 153979649 | cg21342611 | DDX26B | 134655893 |
| cg02773050 | DDX26B | 134654287 | cg11696431 | SLITRK2 | 144902062 | cg21360798 | SLC9A6 | 135067436 |
| cg02775175 | EFNB1 | 68048001 | cg11696466 | ATP1B4 | 119510970 | cg21362390 | SLITRK2 | 144903565 |
| cg02775256 | ZC3H12B | 64708686 | cg11704309 | ARMCX3 | 100878326 | cg21365235 | OCRL | 128674765 |
| cg02779729 | SMARCA1 | 128631361 | cg11704979 | ZNF275 | 152599450 | cg21374587 | FHL1 | 135278952 |
| cg02784144 | GPC4 | 132547235 | cg11707352 | CITED1 | 71525415 | cg21378690 | HDAC8 | 71694534 |
| cg02804166 | PHF6 | 133506996 | cg11708328 | TRMT2B | 100307142 | cg21380860 | TSC22D3 | 106958499 |
| cg02804708 | WDR45 | 48955534 | cg11708870 | KLHL13 | 117251123 | cg21384883 | WDR13 | 48457289 |
| cg02811588 | FHL1 | 135249966 | cg11717930 | TSPYL2 | 53111116 | cg21395967 | TBC1D8B | 106046174 |
| cg02821380 | HTATSF1 | 135579081 | cg11720010 | PRRG1 | 37266204 | cg21397157 | FUNDC2 | 154254928 |
| cg02830187 | DUSP9 | 152906667 | cg11721100 | MAP7 D2 | 20134346 | cg21398507 | OCRL | 128674145 |
| cg02841824 | SPIN2A | 57162355 | cg11721498 | RAB9B | 103080633 | cg21401951 | CITED1 | 71527096 |
| cg02853686 | MBTPS2 | 21858179 | cg11723766 | FTSJ1 | 48335004 | cg21410798 | LONRF3 | 118108538 |
| cg02855049 | FAM3A | 153734472 | cg11727174 | PDHA1 | 19362342 | cg21411291 | IDS | 148577025 |
| cg02856792 | SLC35A2 | 48769319 | cg11764747 | EFNB1 | 68050216 | cg21411942 | TIMM8A | 100603678 |
| cg02859554 | GPR64 | 19140359 | cg11799529 | FHL1 | 135292566 | cg21417660 | TCEAL4 | 102840997 |
| cg02871887 | RPL10 | 153626455 | cg11805814 | MAGEB16 | 35820968 | cg21456313 | TSC22D3 | 107019610 |
| cg02875834 | DIAPH2 | 95939848 | cg11806565 | PHKA1 | 71934695 | cg21459545 | DNASE1L1 | 153637814 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02877744 | XIAP | 123047040 | cg11816360 | C1GALT1C1 | 119763444 | cg21471515 | MBNL3 | 131623259 |
| cg02878966 | PCYT1B | 24692176 | cg11824111 | DKC1 | 153990676 | cg21484926 | NRK | 105066912 |
| cg02879223 | DLG3 | 69672415 | cg11825731 | TFE3 | 48901162 | cg21491240 | HTATSF1 | 135578828 |
| cg02882301 | PPP1R3F | 49126157 | cg11825763 | TSR2 | 54466428 | cg21502819 | FTSJ1 | 48336970 |
| cg02883100 | HSD17B10 | 53460671 | cg11837390 | TSC22D3 | 106957165 | cg21516819 | TSPAN7 | 38421078 |
| cg02893780 | ATP6AP1 | 153655823 | cg11839979 | TMEM164 | 109246372 | cg21517121 | PORCN | 48366924 |
| cg02896496 | WNK3 | 54385394 | cg11844737 | BCOR | 40037471 | cg21544051 | EFNB1 | 68048104 |
| cg02904064 | MTMR8 | 63616177 | cg11849673 | CXorf36 | 45022008 | cg21547183 | RBMX | 135963051 |
| cg02907689 | PDZD4 | 153093784 | cg11851349 | FLNA | 153603154 | cg21557231 | MCF2 | 138725077 |
| cg02909397 | RPS6KA3 | 20284697 | cg11860160 | GD11 | 153667209 | cg21593552 | HTATSF1 | 135579428 |
| cg02924298 | DMD | 33146609 | cg11863519 | MECP2 | 153362995 | cg21597797 | AMMECR1 | 109561513 |
| cg02929371 | MAOB | 43741601 | cg11864871 | MAGIX | 49018988 | cg21601364 | C1GALT1C1 | 119764469 |
| cg02931149 | FLNA | 153586557 | cg11865600 | MORC4 | 106243848 | cg21602092 | IGBP1 | 69353812 |
| cg02931660 | BCOR | 40002443 | cg11867195 | ZBTB33 | 119384450 | cg21604054 | MST4 | 131156970 |
| cg02932805 | BCOR | 40031550 | cg11871337 | SPIN3 | 57017815 | cg21626493 | LRCH2 | 114469591 |
| cg02934281 | CSTF2 | 100075531 | cg11871628 | CITED1 | 71528352 | cg21634944 | EDA | 68835885 |
| cg02935635 | BCORL1 | 129147515 | cg11887015 | PGK1 | 77359083 | cg21635515 | ASB9 | 15272308 |
| cg02937293 | CLCN5 | 49687792 | cg11898425 | CCNB3 | 50026326 | cg21635956 | BGN | 152772215 |
| cg02939364 | CDKL5 | 18443708 | cg11901680 | TFE3 | 48901069 | cg21648502 | CFP | 47489285 |
| cg02940028 | TRMT2B | 100306693 | cg11902811 | SLC25A14 | 129473879 | cg21648914 | DLG3 | 69672831 |
| cg02940221 | TKTL1 | 153536952 | cg11906997 | FHL1 | 135230024 | cg21649182 | ARHGEF6 | 135864872 |
| cg02944422 | ZNF81 | 47696228 | cg11924796 | UXT | 47513194 | cg21652638 | PDZD4 | 153068796 |
| cg02949713 | ACOT9 | 23757953 | cg11926150 | EGFL6 | 13645186 | cg21662807 | BCOR | 40034240 |
| cg02952729 | CFP | 47489470 | cg11932249 | CITED1 | 71524953 | cg21662836 | FUNDC2 | 154255263 |
| cg02953041 | TMEM35 | 100351158 | cg11941526 | MID11P1 | 38660299 | cg21665294 | MED12 | 70337635 |
| cg02954623 | APOOL | 84258791 | cg11941773 | ATP6AP2 | 40444672 | cg21672057 | KIAA2022 | 74144324 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg02969243 | ARHGAP6 | 11445762 | cg11957438 | BRWD3 | 80065114 | cg21692664 | TBC1D8B | 106045287 |
| cg02971064 | MAGIX | 49021148 | cg12026625 | PLP2 | 49028165 | cg21693321 | MED12 | 70338331 |
| cg02975846 | NUDT10 | 51075126 | cg12030231 | GATA1 | 48650312 | cg21697779 | FUNDC2 | 154255534 |
| cg02991082 | TSC22D3 | 106961541 | cg12030973 | ARMCX6 | 100872993 | cg21699341 | FLNA | 153603503 |
| cg02994943 | MPP1 | 154033409 | cg12033483 | NLGN3 | 70389536 | cg21711683 | XIAP | 122994030 |
| cg02998933 | BTK | 100640796 | cg12039967 | YIPF6 | 67718493 | cg21729235 | ARMCX4 | 100739272 |
| cg03003036 | RBM10 | 47038480 | cg12045126 | BCOR | 40003547 | cg21729244 | ZNF449 | 134496604 |
| cg03005718 | CSTF2 | 100078905 | cg12047536 | MCTS1 | 119737620 | cg21730821 | ARMCX4 | 100717965 |
| cg03020597 | SLITRK2 | 144898810 | cg12050949 | KLHL13 | 117246275 | cg21738316 | PLXNA3 | 153688775 |
| cg03021892 | SLC38A5 | 48329278 | cg12051064 | ATP2B3 | 152847462 | cg21744850 | DGKK | 50214878 |
| cg03022736 | MMGT1 | 135053854 | cg12057563 | FHL1 | 135230841 | cg21757148 | LAMP2 | 119571514 |
| cg03025340 | SLC10A3 | 153716662 | cg12058262 | HAUS7 | 152736032 | cg21757436 | ATP11C | 138810080 |
| cg03025760 | SLITRK2 | 144906672 | cg12058508 | WDR45 | 48937975 | cg21758197 | OTUD5 | 48815822 |
| cg03026311 | CSTF2 | 100075083 | cg12058840 | HTATSF1 | 135579744 | cg21760862 | FAM104B | 55187532 |
| cg03031357 | SMS | 21959700 | cg12059303 | CXorf36 | 45016074 | cg21768287 | CD99L2 | 150068356 |
| cg03031520 | PLP1 | 103031374 | cg12064213 | WWC3 | 9982520 | cg21771569 | CDKL5 | 18446114 |
| cg03039483 | HDX | 83757499 | cg12071142 | MIDI | 10588311 | cg21776057 | NKAP | 119077741 |
| cg03040210 | MBNL3 | 131624077 | cg12075609 | GAB3 | 153979476 | cg21784182 | CENPI | 100354923 |
| cg03043911 | KLHL4 | 86771551 | cg12075996 | SCML1 | 17758966 | cg21785054 | PRAF2 | 48931699 |
| cg03046946 | HAUS7 | 152735584 | cg12082130 | ARR3 | 69486740 | cg21786233 | CXorf56 | 118699375 |
| cg03055372 | SLC16A2 | 73641073 | cg12111783 | BCOR | 40027674 | cg21786852 | ARMCX4 | 100672869 |
| cg03056849 | MTMR1 | 149861683 | cg12115116 | TMLHE | 154842856 | cg21799736 | PDHA1 | 19361320 |
| cg03057808 | CXorf57 | 105855296 | cg12115759 | DMD | 31284505 | cg21805978 | WDR44 | 117479430 |
| cg03088756 | BCOR | 40011385 | cg12116395 | ATP6AP2 | 40441216 | cg21808407 | AMMECR1 | 109557508 |
| cg03094405 | ARHGAP6 | 11284830 | cg12116535 | MCF2 | 138775076 | cg21836062 | DMD | 33357661 |
| cg03096488 | AR | 66765289 | cg12124733 | DDX26B | 134655103 | cg21844331 | NUDT10 | 51074836 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03100992 | ARHGAP6 | 11157546 | cg12130976 | FAAH2 | 57312244 | cg21860846 | ELK1 | 47510507 |
| cg03102729 | GLUD2 | 120181504 | cg12137402 | EDA | 68836202 | cg21862797 | MID1 | 10590009 |
| cg03104298 | HTATSF1 | 135579224 | cg12143138 | BRWD3 | 80063471 | cg21875666 | MORF4L2 | 102930542 |
| cg03122511 | NKAP | 119077820 | cg12145026 | IL13RA1 | 117861636 | cg21880156 | ARHGAP6 | 11683548 |
| cg03130686 | ZC3H12B | 64708843 | cg12152167 | DIAPH2 | 95939864 | cg21881821 | EDA | 68836113 |
| cg03139204 | RGN | 46937658 | cg12156154 | AR | 66763447 | cg21883848 | PLXNA3 | 153694383 |
| cg03140487 | BCOR | 40027582 | cg12158214 | FAM104B | 55187822 | cg21887683 | HTATSF1 | 135579043 |
| cg03157806 | TMEM185A | 148713279 | cg12162305 | IGSF1 | 130423207 | cg21888438 | MID1IP1 | 38663117 |
| cg03161453 | BCOR | 39962049 | cg12165338 | GSPT2 | 51486594 | cg21889537 | ATRX | 77041835 |
| cg03164831 | BRWD3 | 80065461 | cg12166502 | ACOT9 | 23761331 | cg21892973 | CLCN4 | 10124573 |
| cg03166428 | BGN | 152760167 | cg12166599 | APEX2 | 55026360 | cg21899276 | ARMCX6 | 100872999 |
| cg03172171 | FUNDC2 | 154254936 | cg12175122 | PDHA1 | 19361925 | cg21900764 | UPF3B | 118986897 |
| cg03181214 | RPL36A | 100646162 | cg12177454 | ARMCX3 | 100877938 | cg21902627 | TSC22D3 | 107019166 |
| cg03183094 | CLIC2 | 154522141 | cg12180028 | AR | 66763604 | cg21905818 | PPP1R3F | 49126164 |
| cg03184366 | WWC3 | 9984977 | cg12194828 | PIGA | 15353857 | cg21906379 | CHRDL1 | 109936239 |
| cg03188519 | MID1 | 10851838 | cg12217778 | PORCN | 48367176 | cg21907582 | MAGIX | 49019190 |
| cg03191149 | CLDN2 | 106161946 | cg12220696 | ATRX | 77042017 | cg21909660 | BTK | 100638850 |
| cg03191359 | RPGR | 38187009 | cg12221218 | DYNLT3 | 37707671 | cg21910711 | WDR13 | 48454657 |
| cg03199955 | GK | 30671630 | cg12226826 | MTMR1 | 149930750 | cg21910941 | IGBP1 | 69353515 |
| cg03201658 | SH3KBP1 | 19553482 | cg12228772 | SPIN4 | 62571231 | cg21911363 | CLCN5 | 49687331 |
| cg03204600 | ARHGEF9 | 62975269 | cg12233790 | CLCN4 | 10126321 | cg21912308 | PRAF2 | 48929987 |
| cg03210912 | SLC10A3 | 153719169 | cg12234922 | HDAC8 | 71793049 | cg21918369 | KLHL13 | 117250968 |
| cg03212133 | MMGT1 | 135057092 | cg12239365 | NUDT10 | 51075790 | cg21932800 | MMGT1 | 135056858 |
| cg03219705 | TSPAN7 | 38420578 | cg12263679 | FAM122C | 133941121 | cg21939789 | TMEM185A | 148713362 |
| cg03221436 | CCDC120 | 48916091 | cg12274524 | PIM2 | 48772845 | cg21943052 | BGN | 152760157 |
| cg03240752 | EFNB1 | 68058033 | cg12277627 | ELF4 | 129245225 | cg21945665 | XIAP | 122993695 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03244432 | PORCN | 48367513 | cg12284142 | HMGB3 | 150151762 | cg21953876 | PRPS2 | 12809345 |
| cg03248529 | PAK3 | 110186893 | cg12287800 | ZNF182 | 47860854 | cg21956484 | XPNPEP2 | 128872153 |
| cg03250346 | KLHL15 | 24042976 | cg12291192 | TBC1D25 | 48397840 | cg21966410 | AR | 66765882 |
| cg03252296 | DMD | 31283706 | cg12294334 | AR | 66763501 | cg21978299 | MCTS1 | 119737955 |
| cg03266942 | POLA1 | 24712207 | cg12295100 | GPRASP1 | 101906886 | cg21983484 | CLCN5 | 49687062 |
| cg03267787 | APOOL | 84344388 | cg12298823 | OTUD5 | 48815744 | cg21988739 | CENPI | 100353462 |
| cg03273615 | RBM41 | 106362373 | cg12312457 | MTMR8 | 63615339 | cg21992771 | ZNF185 | 152109741 |
| cg03273694 | MST4 | 131156448 | cg12324831 | VBP1 | 154444470 | cg21994514 | STARD8 | 67906568 |
| cg03278754 | SHROOM4 | 50553629 | cg12360110 | BCOR | 40036589 | cg22009709 | PIN4 | 71402430 |
| cg03280420 | SLC16A2 | 73640648 | cg12373280 | SLC9A7 | 46618860 | cg22010317 | CXorf36 | 45060544 |
| cg03291819 | RAI2 | 17874725 | cg12375308 | XIAP | 122994071 | cg22014878 | BEX2 | 102565906 |
| cg03305185 | ZNF75D | 134429653 | cg12377327 | WDR45 | 48937697 | cg22016571 | MST4 | 131157426 |
| cg03315531 | FANCB | 14867594 | cg12403148 | TMLHE | 154842718 | cg22036140 | EFNB1 | 68060446 |
| cg03319489 | HUWE1 | 53713873 | cg12408777 | CDKL5 | 18448417 | cg22036814 | MID11P1 | 38660585 |
| cg03321558 | TMEM185A | 148709321 | cg12417438 | PIN4 | 71401510 | cg22044840 | HCCS | 11129352 |
| cg03323167 | ZNF275 | 152617344 | cg12417702 | TIMM8A | 100604006 | cg22045077 | MBNL3 | 131574127 |
| cg03323437 | MAGED2 | 54835446 | cg12427017 | DMD | 32174226 | cg22050539 | MMGT1 | 135056691 |
| cg03325532 | GPR173 | 53105818 | cg12434779 | ARHGAP6 | 11684104 | cg22053855 | FHL1 | 135229186 |
| cg03327000 | TCEAL1 | 102884633 | cg12440269 | RPL36A | 100645895 | cg22069989 | DMD | 31285009 |
| cg03329583 | SYP | 49053740 | cg12443477 | GAB3 | 153979635 | cg22070189 | DMD | 32431479 |
| cg03331695 | RAB40A | 102775412 | cg12447182 | EDA | 68835857 | cg22137300 | UBL4A | 153712066 |
| cg03334316 | FAM120C | 54209370 | cg12449211 | CXorf36 | 45017139 | cg22145512 | ARL13A | 100245441 |
| cg03341064 | KCND1 | 48828399 | cg12452512 | ARMCX4 | 100740439 | cg22151131 | EFNB1 | 68048814 |
| cg03347749 | BEX2 | 102565889 | cg12454245 | NDUFB11 | 47001792 | cg22151216 | ARMCX6 | 100873010 |
| cg03350932 | PDZD4 | 153085101 | cg12456011 | RPS6KA3 | 20284519 | cg22164912 | GNL3L | 54556670 |
| cg03353482 | ZDHHC15 | 74743466 | cg12458993 | MAGIX | 49019062 | cg22166305 | SLC9A6 | 135067468 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03356806 | GLUD2 | 120181820 | cg12459719 | CSTF2 | 100074813 | cg22170936 | TSPYL2 | 53111391 |
| cg03359503 | CLIC2 | 154563952 | cg12461113 | EGFL6 | 13587550 | cg22191603 | HMGB3 | 150151572 |
| cg03360374 | GPKOW | 48980258 | cg12464216 | BEX4 | 102469889 | cg22203323 | CACNA1F | 49084529 |
| cg03363535 | TMSB15A | 101771438 | cg12467143 | ARHGAP6 | 11684543 | cg22206855 | ARMCX3 | 100878328 |
| cg03370193 | DMD | 31284813 | cg12467288 | SLC9A6 | 135067367 | cg22213614 | MBTPS2 | 21861065 |
| cg03372815 | CD99L2 | 150067397 | cg12472203 | PHF8 | 54071365 | cg22221554 | ELF4 | 129245238 |
| cg03375088 | TMEM185A | 148712409 | cg12472218 | SCML1 | 17755211 | cg22222592 | MSN | 64887587 |
| cg03422380 | TSC22D3 | 107019244 | cg12477608 | KLHL13 | 117107923 | cg22226984 | RBM10 | 47041168 |
| cg03424927 | SLC16A2 | 73646094 | cg12481306 | HCCS | 11129329 | cg22238863 | ZNF41 | 47342101 |
| cg03426344 | REPS2 | 16964341 | cg12481479 | TSPAN7 | 38421436 | cg22252245 | CASK | 41781891 |
| cg03435229 | RBMX | 135955827 | cg12485020 | CHRDL1 | 110039269 | cg22252475 | DDX26B | 134654383 |
| cg03445896 | LPAR4 | 78010565 | cg12491455 | HCCS | 11128728 | cg22256180 | RGN | 46936363 |
| cg03448884 | CLDN2 | 106162993 | cg12491738 | KLHL13 | 117251577 | cg22265142 | SYN1 | 47479101 |
| cg03449040 | WWC3 | 10094363 | cg12497304 | LPAR4 | 78002454 | cg22267947 | CUL4B | 119694554 |
| cg03455762 | XPNPEP2 | 128873475 | cg12501871 | PRRG1 | 37208277 | cg22306749 | GPRASP2 | 101967085 |
| cg03460546 | APLN | 128788127 | cg12506468 | DMD | 33147978 | cg22322928 | CXorf36 | 45060415 |
| cg03460558 | SMARCA1 | 128657473 | cg12510923 | HDAC6 | 48660520 | cg22324667 | VMA21 | 150573911 |
| cg03464416 | RPL10 | 153626979 | cg12513189 | FTSJ1 | 48334335 | cg22328457 | USP11 | 47092729 |
| cg03465562 | PHKA2 | 19001031 | cg12516234 | CLCN4 | 10144998 | cg22332233 | TFE3 | 48897515 |
| cg03477368 | WWC3 | 10092308 | cg12518259 | HNRNPH2 | 100668953 | cg22332884 | GPKOW | 48980256 |
| cg03508597 | CXorf36 | 45060356 | cg12521790 | SYP | 49056693 | cg22337136 | KCND1 | 48829251 |
| cg03509908 | CAPN6 | 110489592 | cg12523974 | PDZD4 | 153084761 | cg22343001 | ARMCX2 | 100914848 |
| cg03514678 | IDS | 148586584 | cg12526869 | DYNLT3 | 37706251 | cg22354945 | REPS2 | 16966619 |
| cg03517379 | NKRF | 118740406 | cg12527112 | UPF3B | 118986976 | cg22355031 | EFNB1 | 68060738 |
| cg03523119 | CHM | 85302864 | cg12530936 | SPIN3 | 57022190 | cg22363867 | NUDT10 | 51074936 |
| cg03523199 | HPRT1 | 133593973 | cg12531767 | TAZ | 153644319 | cg22363893 | PAK3 | 110366476 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03527511 | BCOR | 40006072 | cg12543132 | ACOT9 | 23761732 | cg22364374 | CITED1 | 71526984 |
| cg03534718 | HAUS7 | 152736932 | cg12543929 | YIPF6 | 67722985 | cg22364384 | WDR45 | 48941838 |
| cg03536032 | ARHGAP6 | 11683383 | cg12544391 | SAT1 | 23801333 | cg22366498 | PLP2 | 49027884 |
| cg03544771 | MID1IP1 | 38661483 | cg12546665 | MAMLD1 | 149675952 | cg22393371 | MBNL3 | 131547841 |
| cg03553451 | PRICKLE3 | 49032262 | cg12549513 | BEX2 | 102565951 | cg22396288 | ARR3 | 69486749 |
| cg03559234 | NRK | 105067054 | cg12553336 | PDZD4 | 153090536 | cg22404117 | CENPI | 100353450 |
| cg03575468 | PDHA1 | 19362613 | cg12576145 | AMMECR1 | 109560751 | cg22417589 | SMS | 21958631 |
| cg03578806 | PLXNA3 | 153693023 | cg12579866 | NYX | 41331869 | cg22419482 | MPP1 | 154007394 |
| cg03580328 | SLC16A2 | 73641615 | cg12579995 | RAB40A | 102755696 | cg22455616 | HDAC8 | 71793001 |
| cg03598919 | CXorf67 | 51150887 | cg12584551 | OPHN1 | 67653401 | cg22471695 | FGD1 | 54522366 |
| cg03605858 | CXorf67 | 51150443 | cg12586718 | UBL4A | 153715055 | cg22474124 | SASH3 | 128913876 |
| cg03610137 | KLF8 | 56259040 | cg12589433 | AR | 66765978 | cg22484503 | VMA21 | 150565302 |
| cg03646906 | PRAF2 | 48928869 | cg12591117 | OTUD5 | 48815643 | cg22502059 | MTM1 | 149736852 |
| cg03657257 | ARMCX4 | 100673072 | cg12591207 | GPR64 | 19008006 | cg22522913 | RP2 | 46696086 |
| cg03660541 | PORCN | 48368122 | cg12592455 | FAM127A | 134166801 | cg22526309 | PHKA2 | 19003316 |
| cg03660876 | ARHGAP6 | 11284010 | cg12594800 | ELF4 | 129243426 | cg22527050 | RPL36A | 100645695 |
| cg03661529 | ATRX | 77041828 | cg12600766 | GPC4 | 132550131 | cg22534545 | ABCD1 | 152995701 |
| cg03662494 | BCOR | 39958956 | cg12614178 | CASK | 41782685 | cg22543648 | GATA1 | 48644610 |
| cg03665601 | ATRX | 77040152 | cg12614702 | SLC35A2 | 48768927 | cg22560211 | PHF16 | 46787709 |
| cg03669491 | TMSB15A | 101770334 | cg12620265 | GNL3L | 54556429 | cg22561883 | HAUS7 | 152736022 |
| cg03670355 | CLCN4 | 10126353 | cg12623328 | FHL1 | 135229045 | cg22562219 | DNASE1L1 | 153637700 |
| cg03672021 | FLNA | 153602658 | cg12642693 | SHROOM4 | 50556917 | cg22569627 | ZBTB33 | 119385611 |
| cg03679005 | RPL39 | 118926017 | cg12644927 | MAGIX | 49019168 | cg22573003 | FIGF | 15402648 |
| cg03683587 | SHROOM4 | 50557066 | cg12653105 | PLP2 | 49028299 | cg22574818 | MAGEH1 | 55479616 |
| cg03687305 | MOSPD1 | 134031461 | cg12654845 | CLDN2 | 106163009 | cg22575892 | DOCK11 | 117629344 |
| cg03688409 | APEX2 | 55027018 | cg12687215 | FAM50A | 153672898 | cg22590594 | FAM58A | 152853752 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03689540 | UXT | 47516380 | cg12691219 | ARMCX3 | 100878134 | cg22601215 | GSPT2 | 51486845 |
| cg03691313 | FLNA | 153603062 | cg12704708 | PPP1R3F | 49126241 | cg22604777 | MAGEH1 | 55479357 |
| cg03692134 | BCORL1 | 129116990 | cg12707233 | WNK3 | 54384964 | cg22606540 | ARMCX2 | 100914483 |
| cg03692149 | PLXNA3 | 153694773 | cg12724827 | CENPI | 100355703 | cg22607365 | CHM | 85117544 |
| cg03692228 | PIM2 | 48776349 | cg12728820 | CSTF2 | 100075247 | cg22617367 | NKRF | 118740614 |
| cg03705894 | CLCN5 | 49687068 | cg12731488 | SYTL5 | 37893211 | cg22618086 | ARHGAP6 | 11446101 |
| cg03706022 | MID11P1 | 38660704 | cg12737514 | BCOR | 40005177 | cg22618269 | BRWD3 | 80065135 |
| cg03709474 | TMEM187 | 153248513 | cg12742739 | GAB3 | 153906447 | cg22622899 | CXorf36 | 45016993 |
| cg03711046 | OTUD5 | 48814205 | cg12743696 | RAB9B | 103088500 | cg22625945 | RRAGB | 55744275 |
| cg03714991 | DRP2 | 100474897 | cg12753358 | EFNB1 | 68047716 | cg22643918 | NKAP | 119078200 |
| cg03716231 | PRDX4 | 23684892 | cg12763824 | PDZD4 | 153094947 | cg22645859 | UPF3B | 118987043 |
| cg03717198 | RPS6KA3 | 20285056 | cg12775788 | BCOR | 40031643 | cg22646149 | SYP | 49047879 |
| cg03717424 | OPHN1 | 67651132 | cg12799835 | FAAH2 | 57313034 | cg22647743 | WWC3 | 10094051 |
| cg03717635 | EGFL6 | 13586855 | cg12817924 | EGFL6 | 13588162 | cg22655251 | PRICKLE3 | 49043166 |
| cg03718309 | MECP2 | 153363472 | cg12830022 | TMLHE | 154843228 | cg22655582 | MTM1 | 149822475 |
| cg03735049 | TSR2 | 54466618 | cg12838207 | WDR13 | 48457337 | cg22657475 | MAP7 D2 | 20135285 |
| cg03742148 | MAGED1 | 51636629 | cg12841929 | SHROOM4 | 50557522 | cg22660483 | MAGED2 | 54835382 |
| cg03748372 | PPP1R3F | 49126128 | cg12848223 | NRK | 105065721 | cg22662205 | LONRF3 | 118108528 |
| cg03748858 | RAB41 | 69502014 | cg12856112 | FAM50A | 153674274 | cg22672606 | FRMPD4 | 12156718 |
| cg03749630 | WDR44 | 117484304 | cg12856407 | ITM2A | 78622565 | cg22673121 | ASB9 | 15289414 |
| cg03751162 | TSR2 | 54466849 | cg12857026 | PLXNA3 | 153694581 | cg22674664 | MAGED2 | 54835315 |
| cg03752015 | CITED1 | 71526944 | cg12857702 | ATRX | 77041745 | cg22682567 | RPL36A | 100645938 |
| cg03754195 | CUL4B | 119694964 | cg12858650 | RBM10 | 47039497 | cg22686892 | CITED1 | 71525906 |
| cg03759948 | SUV39H1 | 48555275 | cg12861974 | CXorf57 | 105855157 | cg22693205 | PDZD4 | 153084681 |
| cg03761894 | CITED1 | 71527796 | cg12865398 | SYP | 49056670 | cg22695906 | FAM122C | 133955535 |
| cg03761923 | GAB3 | 153946773 | cg12869615 | GPRASP1 | 101906288 | cg22696612 | TKTL1 | 153527313 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03765359 | SYN1 | 47479190 | cg12869875 | TREX2 | 152710928 | cg22710633 | SSX8 | 52655675 |
| cg03765898 | CLCN5 | 49691109 | cg12883250 | TMEM185A | 148713830 | cg22711601 | GK | 30670148 |
| cg03766586 | KLHL4 | 86772789 | cg12888005 | PIM2 | 48776567 | cg22713892 | ZNF41 | 47342160 |
| cg03768687 | SNX12 | 70288358 | cg12889449 | PAK3 | 110339051 | cg22729310 | AIFM1 | 129272178 |
| cg03787796 | CHM | 85302489 | cg12900739 | RRAGB | 55744069 | cg22731373 | PCYT1B | 24665296 |
| cg03790146 | P2RY4 | 69480001 | cg12914043 | SLITRK2 | 144899415 | cg22750044 | SLC16A2 | 73642662 |
| cg03790891 | CLDN2 | 106173458 | cg12920408 | RPL36A | 100645993 | cg22771999 | MSN | 64888437 |
| cg03791434 | OCRL | 128677987 | cg12932046 | ZNF630 | 47930653 | cg22772711 | GPRASP2 | 101967509 |
| cg03791917 | BTK | 100641287 | cg12935118 | SMARCA1 | 128657038 | cg22773241 | RAB41 | 69502066 |
| cg03802931 | FRMPD4 | 12155999 | cg12938998 | GAB3 | 153978709 | cg22807592 | GPRASP2 | 101967307 |
| cg03806192 | DGKK | 50212665 | cg12940781 | NDUFA1 | 119009673 | cg22810426 | ATP2B3 | 152801393 |
| cg03817616 | MTMR8 | 63615495 | cg12942133 | MBNL3 | 131624026 | cg22817042 | IL13RA1 | 117861413 |
| cg03825574 | BCORL1 | 129116503 | cg12944030 | FAM104B | 55187470 | cg22817647 | GPRASP2 | 101967266 |
| cg03828160 | SPIN3 | 57022102 | cg12947498 | ARMCX1 | 100806397 | cg22821597 | OPHN1 | 67352856 |
| cg03829216 | ELK1 | 47497456 | cg12967624 | GSPT2 | 51486354 | cg22823009 | TSC22D3 | 107019005 |
| cg03829491 | NSDHL | 152002367 | cg12972869 | TREX2 | 152711957 | cg22823767 | SCML2 | 18373028 |
| cg03831206 | OTUD5 | 48814955 | cg12974607 | ASB9 | 15264894 | cg22824415 | LONRF3 | 118108546 |
| cg03834574 | KLF8 | 56259094 | cg12980185 | FUNDC2 | 154255174 | cg22829182 | KLF8 | 56258808 |
| cg03840091 | WWC3 | 10111282 | cg13014545 | PGK1 | 77359749 | cg22840251 | RPL36A | 100645007 |
| cg03853861 | EDA2R | 65836683 | cg13015740 | C1GALT1C1 | 119760591 | cg22844499 | PHKA2 | 19002190 |
| cg03887682 | PCYT1B | 24576369 | cg13016553 | UPF3B | 118987015 | cg22845087 | SYN1 | 47433922 |
| cg03892808 | PDZD4 | 153096307 | cg13023833 | ELK1 | 47510183 | cg22848013 | ZNF185 | 152082879 |
| cg03894002 | DLG3 | 69664599 | cg13024202 | KLHL13 | 117250753 | cg22849541 | PORCN | 48368195 |
| cg03900860 | MCTS1 | 119737944 | cg13027792 | YIPF6 | 67720914 | cg22850802 | GD11 | 153665697 |
| cg03908845 | MCF2 | 138724737 | cg13033283 | DKC1 | 153990915 | cg22858728 | MPP1 | 154034149 |
| cg03910337 | KLHL13 | 117119877 | cg13069450 | NKAP | 119077366 | cg22873145 | ARMCX2 | 100914906 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg03915527 | TSC22D3 | 106957793 | cg13077484 | DLG3 | 69665036 | cg22873668 | CXorf56 | 118699347 |
| cg03927113 | MCTS1 | 119738603 | cg13088417 | ATG4A | 107397682 | cg22876425 | HMGB3 | 150152212 |
| cg03927302 | PRPS1 | 106871544 | cg13095029 | GLUD2 | 120181341 | cg22880666 | NKAP | 119076813 |
| cg03933846 | TMSB15A | 101771857 | cg13095698 | SATL1 | 84364580 | cg22887748 | ELK1 | 47497392 |
| cg03937594 | CITED1 | 71523280 | cg13098917 | SYN1 | 47434561 | cg22896615 | RPL36A | 100645005 |
| cg03939693 | YIPF6 | 67718700 | cg13100667 | FHL1 | 135279018 | cg22903061 | FAM127B | 134185575 |
| cg03941517 | MOSPD1 | 134048505 | cg13105517 | GAB3 | 153978857 | cg22911167 | MUM1L1 | 105452427 |
| cg03944921 | WDR44 | 117479980 | cg13108472 | NKRF | 118722692 | cg22912011 | ZNF81 | 47696222 |
| cg03946442 | RPS6KA3 | 20284665 | cg13108852 | GPKOW | 48981300 | cg22918218 | WDR44 | 117479997 |
| cg03959273 | HTATSF1 | 135579015 | cg13113748 | BCOR | 39942822 | cg22921643 | AIFM1 | 129270060 |
| cg03962288 | SLC35A2 | 48769670 | cg13118590 | SMPX | 21777160 | cg22944282 | ZDHHC9 | 128978119 |
| cg03962769 | RAB40A | 102775697 | cg13122496 | AR | 66788286 | cg22946377 | OTUD5 | 48812173 |
| cg03964424 | GRIPAP1 | 48859762 | cg13125542 | RAB33A | 129304952 | cg22951728 | GDI1 | 153664905 |
| cg03972560 | RAI2 | 17877110 | cg13128531 | ZBTB33 | 119384384 | cg22957651 | MSN | 64886649 |
| cg03983969 | CASK | 41782695 | cg13138625 | ZNF182 | 47835221 | cg22964346 | TMEM164 | 109411307 |
| cg04010032 | ELF4 | 129244631 | cg13140681 | IGBP1 | 69385932 | cg22969541 | MTM1 | 149736453 |
| cg04012614 | MAGIX | 49020468 | cg13143866 | STARD8 | 67909943 | cg22969661 | PDK3 | 24483476 |
| cg04013810 | HAUS7 | 152736791 | cg13143954 | CD99L2 | 150062889 | cg22970017 | LDOC1 | 140270501 |
| cg04025582 | RPL36A | 100645906 | cg13145750 | CAPN6 | 110512115 | cg22972692 | FAM58A | 152865117 |
| cg04027004 | TSC22D3 | 107019426 | cg13172884 | P2RY4 | 69479607 | cg22975864 | RBM41 | 106361500 |
| cg04027312 | SLC9A7 | 46618632 | cg13174077 | CUL4B | 119709371 | cg22982510 | BGN | 152772061 |
| cg04027940 | ARHGEF9 | 62975824 | cg13176197 | MID1 | 10449873 | cg22983460 | SUV39H1 | 48554902 |
| cg04029282 | MBNL3 | 131624049 | cg13177658 | CSTF2 | 100074985 | cg22985134 | CLDN2 | 106163661 |
| cg04029366 | CXorf36 | 45017072 | cg13178935 | HDAC6 | 48660228 | cg22993498 | PHF6 | 133506724 |
| cg04029630 | MAGEH1 | 55478329 | cg13182391 | RAI2 | 17878832 | cg23026995 | XPNPEP2 | 128872940 |
| cg04029664 | KLHL13 | 117250962 | cg13183496 | FAM120C | 54209920 | cg23056602 | EDA2R | 65825449 |

(continued)

| Probe ID | Gene | Position in ChrX (hg19) |
|---|---|---|
| cg04031645 | HUWE1 | 53713676 |
| cg04033011 | ARMCX1 | 100807961 |
| cg04035233 | RAB40A | 102775367 |
| cg04037732 | NLGN3 | 70364775 |
| cg04039555 | BHLHB9 | 102000746 |
| cg04053131 | HCCS | 11140682 |
| cg04055739 | ARHGAP6 | 11157142 |
| cg04058675 | HUWE1 | 53680779 |
| cg04061482 | DOCK11 | 117629338 |
| cg04064330 | SSR4 | 153063892 |
| cg04069100 | TCEAL7 | 102584347 |
| cg04070122 | MBNL3 | 131573551 |
| cg04071644 | RAB39B | 154493215 |
| cg04071770 | MAMLD1 | 149613662 |
| cg04075675 | BCOR | 39910902 |
| cg04075827 | MTMR1 | 149861694 |
| cg04094482 | BEX2 | 102566490 |
| cg04101819 | RLIM | 73834489 |
| cg04137897 | BCOR | 40006699 |
| cg04139820 | WDR45 | 48935660 |
| cg04143870 | GPC3 | 133121112 |
| cg04144603 | GPC4 | 132544017 |
| cg04149024 | CFP | 47489551 |
| cg04155024 | ATP6AP1 | 153656891 |
| cg04158739 | PIGA | 15338707 |
| — | HDAC8 | 71792943 |
| cg13187778 | MAGED1 | 51637971 |
| cg13189687 | MECP2 | 153363353 |
| cg13190238 | PRDX4 | 23685407 |
| cg13202398 | OPHN1 | 67267400 |
| cg13203541 | PGK1 | 77359450 |
| cg13204040 | ARMCX2 | 100912211 |
| cg13204375 | TSR2 | 54466432 |
| cg13208429 | AMMECR1 | 109561508 |
| cg13218284 | MID11P1 | 38662289 |
| cg13219667 | FAM50A | 153671953 |
| cg13223227 | MAOB | 43741310 |
| cg13232664 | RAP2C | 131352497 |
| cg13236039 | FAM127A | 134167092 |
| cg13240932 | GDI1 | 153665624 |
| cg13243544 | MAP7 D3 | 135333698 |
| cg13257485 | NXT2 | 108778854 |
| cg13270115 | MIDI | 10589769 |
| cg13273653 | PAK3 | 110339032 |
| cg13279286 | PPP1R3F | 49130160 |
| cg13279774 | NGFRAP1 | 102632528 |
| cg13284386 | RAP2C | 131348267 |
| cg13286902 | TRMT2B | 100306780 |
| cg13304035 | MAP7 D3 | 135333674 |
| cg13307142 | ZDHHC15 | 74743056 |
| cg13307200 | BCOR | 39939117 |
| cg13311758 | APEX2 | 55026811 |
| cg23060203 | ATP11C | 13915046 |
| cg23066860 | GPRASP2 | 101967557 |
| cg23083672 | EGFL6 | 13587603 |
| cg23091104 | TMEM164 | 109246341 |
| cg23106251 | SLITRK2 | 144900190 |
| cg23112505 | LONRF3 | 118110534 |
| cg23116589 | ARMCX1 | 100805503 |
| cg23121114 | IRAK1 | 153285118 |
| cg23124111 | TMEM187 | 153238479 |
| cg23124867 | FHL1 | 135228969 |
| cg23137005 | KIAA2022 | 73960742 |
| cg23137494 | SLC16A2 | 73639809 |
| cg23140620 | PAK3 | 110398983 |
| cg23143524 | PDK3 | 24483077 |
| cg23154024 | SPIN3 | 57021680 |
| cg23156356 | RRAGB | 55743977 |
| cg23157067 | CXorf57 | 105855386 |
| cg23161359 | SMS | 21960400 |
| cg23163644 | TCEAL2 | 101381018 |
| cg23165983 | MAGED1 | 51545767 |
| cg23170483 | FGD1 | 54508656 |
| cg23171231 | ARHGAP6 | 11672048 |
| cg23174160 | SUV39H1 | 48554729 |
| cg23181142 | HAUS7 | 152735919 |
| cg23194060 | MBTPS2 | 21857529 |
| cg23194346 | PRICKLE3 | 49033828 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04162504 | UBE2A | 118715888 | cg13312942 | SPIN3 | 57021244 | cg23212799 | PHF16 | 46773278 |
| cg04166210 | RAB9B | 103087157 | cg13318368 | FAM127B | 134185920 | cg23213278 | TRO | 54946801 |
| cg04167532 | NGFRAP1 | 102631697 | cg13326840 | POLA1 | 25014481 | cg23214992 | VBP1 | 154444717 |
| cg04174234 | FIGF | 15364248 | cg13328614 | PIM2 | 48775123 | cg23216058 | SYN1 | 47434477 |
| cg04177332 | BGN | 152759883 | cg13338084 | APLN | 128788977 | cg23221544 | DUSP9 | 152907546 |
| cg04182378 | SMS | 21958416 | cg13340269 | TMEM35 | 100333778 | cg23232084 | UBL4A | 153715609 |
| cg04184316 | UPF3B | 118987146 | cg13358623 | FIGF | 15402593 | cg23242236 | PDHA1 | 19362639 |
| cg04189389 | SCML2 | 18373603 | cg13365334 | FLNA | 153598742 | cg23245147 | CHRDL1 | 110039542 |
| cg04210922 | GPRASP2 | 101966398 | cg13368805 | ARAF | 47420179 | cg23245497 | LHFPL1 | 111923245 |
| cg04216286 | UTP14A | 129039782 | cg13370412 | PHF16 | 46772948 | cg23247524 | MAGEB16 | 35816354 |
| cg04217946 | DMD | 31285018 | cg13370439 | MST4 | 131157113 | cg23247655 | TSR2 | 54466701 |
| cg04223454 | ARHGAP6 | 11284932 | cg13370916 | STARD8 | 67867403 | cg23250973 | PLXNA3 | 153690367 |
| cg04224247 | WWC3 | 9984515 | cg13371527 | ARAF | 47420583 | cg23251359 | DGKK | 50214465 |
| cg04225046 | GPRASP1 | 101906179 | cg13372830 | ARAF | 47420231 | cg23269489 | BCORL1 | 129117241 |
| cg04225254 | MAP7D3 | 135329779 | cg13391512 | BEX2 | 102564733 | cg23277098 | LASLL | 64754804 |
| cg04235544 | ACOT9 | 23762372 | cg13392184 | IDS | 148587355 | cg23279136 | ABCB7 | 74375966 |
| cg04238548 | TBC1D8B | 106045825 | cg13412357 | PDZD4 | 153071536 | cg23299576 | TMEM185A | 148713663 |
| cg04238575 | GSPT2 | 51486666 | cg13420803 | CXorf56 | 118699970 | cg23299641 | KCNE1L | 108868237 |
| cg04241572 | HSD17B10 | 53461213 | cg13431666 | TSPAN7 | 38420436 | cg23300485 | LPAR4 | 78012038 |
| cg04262605 | MAP7 D3 | 135333761 | cg13441872 | GNL3L | 54556943 | cg23317607 | GRIPAP1 | 48842038 |
| cg04270663 | UBQLN2 | 56590513 | cg13442104 | GK | 30671410 | cg23322383 | STARD8 | 67916187 |
| cg04277998 | GRIPAP1 | 48858688 | cg13443410 | GRIPAP1 | 48858686 | cg23323276 | FAM127B | 134186288 |
| cg04283377 | ARAF | 47420348 | cg13444289 | HMGB3 | 150158593 | cg23323745 | ZNF81 | 47696132 |
| cg04287686 | GATA1 | 48644859 | cg13448203 | ASB12 | 63444339 | cg23352597 | CHST7 | 46435742 |
| cg04288012 | HUWE1 | 53711065 | cg13482208 | PDZD4 | 153070352 | cg23356769 | ELF4 | 129245245 |
| cg04288265 | MAMLD1 | 149680306 | cg13482233 | HEPH | 65382529 | cg23360535 | TRO | 54947822 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04290452 | PGRMC1 | 118370187 | cg13484245 | ARMCX4 | 100673096 | cg23362763 | TCEAL1 | 102883550 |
| cg04290766 | TREX2 | 152712044 | cg13486082 | NGFRAP1 | 102629870 | cg23374711 | SMS | 21958612 |
| cg04292836 | ACOT9 | 23761484 | cg13489005 | ARHGEF9 | 62975162 | cg23387422 | DNASE1L1 | 153637603 |
| cg04293108 | SYTL5 | 37891792 | cg13489054 | DDX26B | 134653741 | cg23392495 | ZNF185 | 152081932 |
| cg04297329 | NAP1L2 | 72434720 | cg13501371 | CHRDL1 | 110039194 | cg23396935 | FAM3A | 153745704 |
| cg04297907 | CLCN4 | 10126668 | cg13503613 | FHL1 | 135278915 | cg23408987 | RPL10 | 153626606 |
| cg04298609 | ATRX | 77041722 | cg13510429 | PDZD4 | 153094195 | cg23429746 | DLG3 | 69665118 |
| cg04301343 | GPR64 | 19140658 | cg13524834 | MAGED1 | 51545659 | cg23431897 | PLP2 | 49027992 |
| cg04302178 | XIAP | 122993941 | cg13525739 | ZNF41 | 47337961 | cg23446945 | NKRF | 118740132 |
| cg04302192 | ZNF41 | 47341740 | cg13530507 | MAGED2 | 54833796 | cg23452168 | MTM1 | 149741299 |
| cg04310247 | CXorf56 | 118699362 | cg13532816 | OCRL | 128674686 | cg23455407 | SNX12 | 70288342 |
| cg04317640 | SLC16A2 | 73640431 | cg13532885 | SYN1 | 47478770 | cg23475462 | MAGT1 | 77151042 |
| cg04324559 | DNASE1L1 | 153637261 | cg13536141 | GPC4 | 132548278 | cg23475921 | MAGED1 | 51636726 |
| cg04325187 | MTMR1 | 149930807 | cg13539181 | TMEM164 | 109246271 | cg23476456 | CHRDL1 | 110039192 |
| cg04327763 | RPL39 | 118925713 | cg13542480 | MAGED1 | 51639920 | cg23476908 | PAK3 | 110365603 |
| cg04329736 | TFE3 | 48899020 | cg13549303 | RPS6KA6 | 83443165 | cg23483095 | NAP1L2 | 72434554 |
| cg04334684 | ATP6AP1 | 153657362 | cg13569417 | STAG2 | 123094304 | cg23485564 | ASB12 | 63444921 |
| cg04336572 | ATP1B4 | 119496002 | cg13571540 | SAT1 | 23801624 | cg23490611 | ZNF75D | 134475099 |
| cg04341767 | CLCN5 | 49687028 | cg13572003 | DGKK | 50213849 | cg23493704 | SLC10A3 | 153719780 |
| cg04342904 | PHF16 | 46852029 | cg13574945 | OTUD5 | 48814580 | cg23493872 | SLC9A6 | 135067542 |
| cg04345928 | TSPAN7 | 38420783 | cg13582495 | ZDHHC15 | 74743534 | cg23494279 | PLXNA3 | 153698358 |
| cg04368919 | EFNB1 | 68048131 | cg13606829 | VMA21 | 150565921 | cg23496314 | BCOR | 39956534 |
| cg04369555 | HTATSF1 | 135578793 | cg13616144 | USP11 | 47093305 | cg23497752 | FLNA | 153598077 |
| cg04371001 | UBL4A | 153715321 | cg13620040 | ZNF275 | 152614935 | cg23501813 | TIMM8A | 100604329 |
| cg04376185 | SCML1 | 17755081 | cg13627141 | BCORL1 | 129118953 | cg23503517 | POLA1 | 24712096 |
| cg04388383 | LASLL | 64754908 | cg13633856 | MBNL3 | 131547702 | cg23505798 | FAM3A | 153735204 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04392694 | MAGIX | 49019049 | cg13640071 | TCEAL7 | 102585147 | cg23508470 | IL13RA1 | 117864239 |
| cg04393989 | PLXNA3 | 153688713 | cg13643725 | PPP1R3F | 49143789 | cg23517015 | ZNF275 | 152612692 |
| cg04399994 | PAK3 | 110339005 | cg13645300 | PLP2 | 49027939 | cg23518228 | SMPX | 21777644 |
| cg04402371 | PCYT1B | 24691042 | cg13646297 | LRCH2 | 114468303 | cg23522780 | UBE2A | 118712792 |
| cg04402486 | RAP2C | 131352242 | cg13649400 | FAM120C | 54209968 | cg23524524 | MAGIX | 49020116 |
| cg04412186 | HDX | 83757470 | cg13651586 | SLC35A2 | 48769094 | cg23527532 | FLNA | 153600130 |
| cg04413754 | BCOR | 39956558 | cg13652795 | BCOR | 40000784 | cg23530509 | CASK | 41783785 |
| cg04419618 | RBMX | 135962940 | cg13655660 | RPL39 | 118925773 | cg23530721 | SAT1 | 23801067 |
| cg04424654 | AMMECR1 | 109561524 | cg13658777 | PDHA1 | 19361740 | cg23533179 | HTATSF1 | 135583940 |
| cg04437194 | CACNALF | 49085918 | cg13663390 | NKRF | 118740602 | cg23534593 | ZNF41 | 47342469 |
| cg04464742 | CUL4B | 119694953 | cg13663706 | SLITRK2 | 144899995 | cg23543821 | GLUD2 | 120182714 |
| cg04483797 | TCEAL1 | 102883838 | cg13664654 | LASLL | 64754752 | cg23545272 | EFNB1 | 68049613 |
| cg04484695 | MAOB | 43742501 | cg13674559 | WDR44 | 117479752 | cg23549061 | PAK3 | 110187233 |
| cg04489366 | SMPX | 21776494 | cg13677598 | DLG3 | 69672096 | cg23550962 | FRMPD4 | 12602374 |
| cg04491396 | TSC22D3 | 107019182 | cg13677770 | TLR7 | 12907574 | cg23552913 | BCORL1 | 129190217 |
| cg04493740 | SLC25A14 | 129474199 | cg13680016 | AMMECR1 | 109561549 | cg23554546 | UPF3B | 118987202 |
| cg04499381 | SASH3 | 128913671 | cg13681367 | ELF4 | 129214223 | cg23556525 | KLHL15 | 24043380 |
| cg04501372 | SCML1 | 17756628 | cg13682241 | RBMX | 135962950 | cg23571457 | GPRASP1 | 101907558 |
| cg04514385 | DDX26B | 134653547 | cg13699152 | GK | 30671314 | cg23572319 | MORF4L2 | 102941768 |
| cg04533591 | RPL39 | 118926128 | cg13700552 | GPRASP2 | 101968505 | cg23572455 | SLC9A7 | 46614442 |
| cg04544154 | ELF4 | 129245509 | cg13712154 | DIAPH2 | 95939171 | cg23574225 | DDX26B | 134684704 |
| cg04549977 | MTMR8 | 63613026 | cg13723766 | NKRF | 118739835 | cg23577962 | ZDHHC15 | 74590752 |
| cg04569165 | PLXNA3 | 153688677 | cg13726176 | ZNF275 | 152612785 | cg23595826 | HTATSF1 | 135580181 |
| cg04574723 | P2RY4 | 69479214 | cg13728115 | BCOR | 40030819 | cg23604959 | IRAK1 | 153283694 |
| cg04575501 | ZNF41 | 47342663 | cg13741548 | MCTS1 | 119738453 | cg23607813 | AKAP4 | 49964918 |
| cg04586456 | RP2 | 46696297 | cg13744306 | PPP1R3F | 49126087 | cg23612178 | TSPYL2 | 53111677 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04587983 | KCND1 | 48825777 | cg13747195 | BEX4 | 102471167 | cg23627980 | SCML1 | 17755717 |
| cg04594276 | DMD | 32830005 | cg13749508 | CFP | 47485820 | cg23660678 | TSC22D3 | 106959609 |
| cg04595393 | BHLHB9 | 102001018 | cg13758960 | CXorf36 | 45018692 | cg23662469 | FGD1 | 54472372 |
| cg04596655 | SMARCA1 | 128656768 | cg13766601 | ATP6AP1 | 153657086 | cg23663092 | PRDX4 | 23686290 |
| cg04598760 | RAB9B | 103083533 | cg13768026 | ENOX2 | 130036695 | cg23675214 | SUV39H1 | 48554935 |
| cg04602218 | SLC25A43 | 118533786 | cg13771263 | YIPF6 | 67718708 | cg23687192 | HUWE1 | 53713791 |
| cg04616757 | FRMPD4 | 12601534 | cg13777527 | PDK3 | 24557620 | cg23688843 | CCNB3 | 50026733 |
| cg04619644 | PDZD4 | 153083134 | cg13780945 | RPGR | 38144773 | cg23694557 | IGSF1 | 130423081 |
| cg04620228 | UTP14A | 129062457 | cg13781574 | DLG3 | 69675294 | cg23696472 | TSPYL2 | 53111786 |
| cg04626171 | ELK1 | 47510405 | cg13781721 | MBNL3 | 131543695 | cg23698956 | FAM3A | 153744915 |
| cg04642759 | SLITRK2 | 144898689 | cg13783167 | DLG3 | 69678896 | cg23699176 | CITED1 | 71522070 |
| cg04646860 | CETN2 | 151996001 | cg13786096 | ZBTB33 | 119384537 | cg23701759 | BCOR | 39959596 |
| cg04647039 | DMD | 31285105 | cg13788061 | ITM2A | 78623020 | cg23708288 | DLG3 | 69674800 |
| cg04647094 | MBTPS2 | 21857776 | cg13789561 | ARAF | 47420453 | cg23709838 | SLC35A2 | 48768883 |
| cg04651193 | NUDT10 | 51074068 | cg13795116 | CLCN4 | 10202184 | cg23710475 | CITED1 | 71525713 |
| cg04663649 | MST4 | 131157704 | cg13797960 | STARD8 | 67906191 | cg23712855 | DKC1 | 153991009 |
| cg04665139 | PIGA | 15353772 | cg13801593 | TSC22D3 | 106960186 | cg23714856 | TBC1D8B | 106045823 |
| cg04669465 | MAGIX | 49019098 | cg13807550 | MAGED2 | 54838543 | cg23722529 | ARHGAP6 | 11445498 |
| cg04675170 | LASLL | 64754950 | cg13810915 | ALAS2 | 55050253 | cg23726559 | SNX12 | 70288735 |
| cg04675306 | SLC9A6 | 135067450 | cg13811328 | CFP | 47490390 | cg23731466 | SCML1 | 17755254 |
| cg04675919 | PRICKLE3 | 49042889 | cg13817994 | ZNF275 | 152600484 | cg23737407 | BCOR | 40029094 |
| cg04681171 | POLA1 | 24712459 | cg13818628 | MAP7D3 | 135333705 | cg23756219 | DRP2 | 100475124 |
| cg04688327 | IGSF1 | 130419944 | cg13822691 | MSN | 64887355 | cg23771329 | HUWE1 | 53713697 |
| cg04690567 | PHF8 | 53964887 | cg13824755 | ASB9 | 15271183 | cg23774896 | CITED1 | 71527048 |
| cg04699313 | SPIN2A | 57163839 | cg13839778 | SYN1 | 47479048 | cg23800739 | PFKFB1 | 54959707 |
| cg04700811 | SLC38A5 | 48317196 | cg13851721 | CHRDL1 | 109917662 | cg23832225 | MAGT1 | 77151104 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04702045 | OPHN1 | 67653598 | cg13866977 | AMOT | 112051826 | cg23834117 | MAGED2 | 54833994 |
| cg04703500 | GRIPAP1 | 48858679 | cg13868987 | ARHGAP6 | 11683131 | cg23837911 | GPC3 | 133119687 |
| cg04709809 | ZNF630 | 47930636 | cg13897449 | PFKFB1 | 55021841 | cg23839682 | OPHN1 | 67653496 |
| cg04716051 | CDKL5 | 18443256 | cg13915481 | RAI2 | 17878772 | cg23850281 | GPR64 | 19137976 |
| cg04738169 | CCNB3 | 50027319 | cg13915726 | DUSP9 | 152908309 | cg23851088 | BCOR | 40004432 |
| cg04739874 | MST4 | 131157495 | cg13918312 | ZDHHC9 | 128977954 | cg23851205 | RPGR | 38186919 |
| cg04743474 | MBNL3 | 131624555 | cg13929917 | BCOR | 40017269 | cg23852001 | FLNA | 153599682 |
| cg04746792 | APOOL | 84258939 | cg13933535 | DIAPH2 | 96795331 | cg23852772 | AWAT1 | 69459685 |
| cg04747879 | SNX12 | 70288391 | cg13934764 | PIN4 | 71417979 | cg23857909 | MECP2 | 153362596 |
| cg04748497 | GPRASP1 | 101906989 | cg13935560 | MOSPD1 | 134048490 | cg23859208 | AIFM1 | 129297005 |
| cg04751297 | BCOR | 39960365 | cg13936874 | DCX | 110654436 | cg23873430 | DCX | 110655626 |
| cg04754076 | ZNF275 | 152599461 | cg13940798 | SHROOM4 | 50556181 | cg23881693 | TMEM164 | 109246236 |
| cg04755662 | AR | 66763359 | cg13941987 | BCORL1 | 129118022 | cg23892645 | PGRMC1 | 118370162 |
| cg04768312 | MED12 | 70337980 | cg13943560 | BCOR | 40004481 | cg23896353 | MTM1 | 149737039 |
| cg04769341 | TCEAL7 | 102585202 | cg13977434 | EFNB1 | 68048117 | cg23896806 | GAB3 | 153979543 |
| cg04777103 | MCTS1 | 119738402 | cg13980651 | FHL1 | 135233206 | cg23897336 | SLITRK2 | 144900532 |
| cg04778337 | FHL1 | 135228444 | cg13984511 | ACOT9 | 23760918 | cg23898859 | FAM104B | 55187392 |
| cg04779297 | REPS2 | 16964882 | cg3987972 | MBNL3 | 131624114 | cg23905908 | PIN4 | 71401384 |
| cg04782269 | UPRT | 74493844 | cg13988667 | IDS | 148586944 | cg23906224 | BCOR | 40013450 |
| cg04784122 | MPP1 | 154033688 | cg14001161 | POLA1 | 24713101 | cg23914031 | CHRDL1 | 110036810 |
| cg04811950 | GDI1 | 153666533 | cg14002781 | IDH3G | 153051367 | cg23926715 | ZC4H2 | 64196278 |
| cg04812084 | SH3KBP1 | 19819286 | cg14004892 | SLC9A6 | 135067651 | cg23932180 | AMOT | 112083746 |
| cg04812249 | CLDN2 | 106171680 | cg14006678 | OTUD5 | 48814864 | cg23934598 | BCORL1 | 129118276 |
| cg04820593 | WNK3 | 54383906 | cg14018172 | REPS2 | 16964347 | cg23939077 | TSR2 | 54466697 |
| cg04821091 | SLC25A14 | 129473898 | cg14019861 | ELK1 | 47496001 | cg23947872 | MTMR1 | 149862363 |
| cg04822213 | ARL13A | 100223435 | cg14022645 | TFE3 | 48901405 | cg23948362 | SLC38A5 | 48317264 |

58

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg04843066 | DDX26B | 134654334 | cg14028178 | BGN | 152760034 | cg23948993 | WWC3 | 9983205 |
| cg04853367 | ZC3H12B | 64708046 | cg14029629 | MAGIX | 49023064 | cg23949973 | NRK | 105065796 |
| cg04855977 | ZDHHC9 | 128977653 | cg14051366 | PPP1R3F | 49126150 | cg23951868 | CLCN5 | 49687084 |
| cg04856605 | IGBP1 | 69353178 | cg14053556 | ARMCX6 | 100873362 | cg23954847 | TFE3 | 48887326 |
| cg04861259 | CLCN5 | 49774573 | cg14057181 | CXorf57 | 105855056 | cg23955970 | AMOT | 112067833 |
| cg04872051 | CHST7 | 46433447 | cg14059333 | SATL1 | 84363758 | cg23959569 | MAP7D2 | 20135311 |
| cg04882894 | IGSF1 | 130423289 | cg14061423 | ATRX | 77041452 | cg23985370 | PJA1 | 68385233 |
| cg04886514 | AR | 66764893 | cg14071112 | TSPAN7 | 38421503 | cg23987345 | NUDT10 | 51076387 |
| cg04907664 | EFNB1 | 68049805 | cg14073590 | CITED1 | 71526883 | cg23991947 | TBC1D25 | 48397845 |
| cg04920616 | FOXP3 | 49121288 | cg14076132 | LPAR4 | 78003346 | cg23992130 | LONRF3 | 118113800 |
| cg04922020 | FAM127A | 134166009 | cg14084448 | ARMCX4 | 100737870 | cg23993375 | UBQLN2 | 56589951 |
| cg04924141 | RAB39B | 154493858 | cg14091713 | RP2 | 46696093 | cg23996121 | IGSF1 | 130407545 |
| cg04924962 | BCOR | 39948134 | cg14106263 | RBMX2 | 129535683 | cg24000218 | GPR64 | 19140934 |
| cg04926827 | TMEM164 | 109246494 | cg14107136 | ARHGAP6 | 11445620 | cg24000816 | RAB41 | 69501942 |
| cg04929015 | RAI2 | 17818629 | cg14107346 | DGKK | 50213549 | cg24001644 | MAP7D2 | 20134719 |
| cg04929865 | BGN | 152760078 | cg14111132 | ATP2B3 | 152800237 | cg24034992 | YIPF6 | 67719066 |
| cg04930858 | PIM2 | 48777487 | cg14111789 | CASK | 41374859 | cg24035229 | BCORL1 | 129115416 |
| cg04932755 | EFNB1 | 68047893 | cg14113919 | MAP7D2 | 20134908 | cg24046087 | PDZD4 | 153095663 |
| cg04944936 | FAM120C | 54209594 | cg14123135 | MSN | 64887466 | cg24052239 | CACNA1F | 49087465 |
| cg04992335 | BCOR | 40017469 | cg14126185 | TSPYL2 | 53114138 | cg24054653 | C1GALT1C1 | 119763829 |
| cg05019001 | AR | 66766334 | cg14127907 | HTATSF1 | 135579045 | cg24076965 | OCRL | 128675022 |
| cg05026884 | BCOR | 40032180 | cg14132995 | SLC35A2 | 48769370 | cg24088023 | TSPAN7 | 38547207 |
| cg05028071 | TFE3 | 48900948 | cg14134864 | CXorf67 | 51151061 | cg24090346 | DYNLT3 | 37707025 |
| cg05032098 | TCEAL4 | 102840912 | cg14137558 | UTP14A | 129040091 | cg24113411 | MCTS1 | 119737351 |
| cg05032353 | OCRL | 128674224 | cg14148995 | LONRF3 | 118151666 | cg24123780 | ACOT9 | 23761651 |
| cg05039054 | DMD | 33357782 | cg14161977 | MAP7D2 | 20027140 | cg24126759 | NGFRAP1 | 102632060 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05040675 | PLP2 | 49027496 | cg14168975 | ALAS2 | 55057838 | cg24138084 | MOSPD1 | 134048712 |
| cg05044339 | KLHL15 | 24040675 | cg14179628 | TCEAL7 | 102584953 | cg24139739 | EFNB1 | 68048818 |
| cg05045028 | AIFM1 | 129299924 | cg14191036 | FTSJ1 | 48335457 | cg24139895 | NXT2 | 108780677 |
| cg05045659 | OPHN1 | 67352784 | cg14196261 | ACOT9 | 23761469 | cg24142775 | TSC22D3 | 107019430 |
| cg05045738 | RLIM | 73834707 | cg14214586 | WDR44 | 117479910 | cg24149051 | STARD8 | 67913327 |
| cg05049545 | SCML2 | 18372696 | cg14220025 | EFNB1 | 68047833 | cg24149658 | CAPN6 | 110513817 |
| cg05065576 | RAB9B | 103087808 | cg14230133 | IDS | 148587055 | cg24152857 | SLC38A5 | 48326650 |
| cg05066631 | BCOR | 40018892 | cg14233178 | CACNA1F | 49089987 | cg24156613 | BCOR | 39950501 |
| cg05071823 | DOCK11 | 117628671 | cg14245501 | CUL4B | 119710463 | cg24157854 | GAB3 | 153979233 |
| cg05085049 | DUSP9 | 152909992 | cg14247154 | MTMR8 | 63615380 | cg24183173 | BCOR | 39962195 |
| cg05088006 | DMD | 32886461 | cg14248531 | DLG3 | 69670903 | cg24186901 | EDA | 68835895 |
| cg05091873 | CD99L2 | 150067687 | cg14251060 | WDR13 | 48463511 | cg24187269 | TKTL1 | 153533256 |
| cg05093881 | SASH3 | 128928771 | cg14252169 | PHF6 | 133560550 | cg24192264 | MBTPS2 | 21877894 |
| cg05093999 | MIDI | 10851789 | cg14253153 | HSD17B10 | 53458243 | cg24194941 | VBP1 | 154444570 |
| cg05096434 | MORC4 | 106243404 | cg14254545 | UBE2A | 118717762 | cg24208785 | BCOR | 40013348 |
| cg05096515 | RBM41 | 106362254 | cg14258356 | ELK1 | 47510110 | cg24239230 | EFNB1 | 68058376 |
| cg05100261 | RPL10 | 153626754 | cg14261068 | BCOR | 39946573 | cg24248049 | FAM122C | 133941190 |
| cg05104476 | CCDC22 | 49092076 | cg14267065 | GK | 30671787 | cg24249486 | ARHGAP6 | 11684119 |
| cg05105069 | TCEAL7 | 102585354 | cg14271953 | MIDI | 10525587 | cg24253627 | STARD8 | 67944616 |
| cg05106824 | GLUD2 | 120181294 | cg14273219 | PPP1R3F | 49127199 | cg24258005 | FAM104B | 55187696 |
| cg05121989 | EFNB1 | 68047520 | cg14291118 | UTP14A | 129038758 | cg24258259 | WWC3 | 10083177 |
| cg05127178 | MAGED1 | 51636111 | cg14295696 | FHL1 | 135230717 | cg24263233 | CXorf56 | 118699475 |
| cg05131629 | NYX | 41307114 | cg14295915 | FTSJ1 | 48334557 | cg24264679 | DLG3 | 69675064 |
| cg05138704 | FTSJ1 | 48336406 | cg14296882 | ATP11C | 138915561 | cg24265865 | ELK1 | 47497341 |
| cg05143403 | RRAGB | 55744049 | cg14304890 | HDAC6 | 48663864 | cg24266622 | SLC25A14 | 129476243 |
| cg05147108 | OCRL | 128674369 | cg14306994 | MAGIX | 49019804 | cg24268343 | KLF8 | 56260186 |

EP 4 495 263 A1

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05147330 | DOCK11 | 117731074 | cg14314132 | UPF3B | 118986954 | cg24274662 | MOSPD1 | 134049505 |
| cg05152874 | SHROOM4 | 50557122 | cg14320120 | GATA1 | 48646959 | cg24281651 | GK | 30671637 |
| cg05155058 | SMS | 21961975 | cg14321399 | RRAGB | 55744191 | cg24326574 | UBL4A | 153713930 |
| cg05162528 | TMEN187 | 153240299 | cg14322166 | NLGN3 | 70364735 | cg24331682 | KLH15 | 24046285 |
| cg05169062 | TREX2 | 152712186 | cg14331377 | MID1 | 10588732 | cg24332600 | ATP6AP1 | 153662580 |
| cg05181702 | WWC3 | 9983462 | cg14338025 | ZNF185 | 152127622 | cg24336035 | MAGIX | 49018789 |
| cg05184076 | MORF4L2 | 102941799 | cg14343687 | KLHL13 | 117250477 | cg24340926 | RAB33A | 129305036 |
| cg05184682 | MBTPS2 | 21857796 | cg14350469 | DKC1 | 153991165 | cg24343614 | KLHL13 | 117251558 |
| cg05185776 | FAM104B | 55187570 | cg14351440 | MCF2 | 138726210 | cg24347720 | MAP7D3 | 135333661 |
| cg05200254 | MCTS1 | 119737969 | cg14368286 | PRAF2 | 48931953 | cg24351198 | WDR13 | 48455856 |
| cg05200893 | BCOR | 39956808 | cg14368852 | ZNF185 | 152130179 | cg24354925 | NLGN3 | 70387171 |
| cg05204037 | APOOL | 84258754 | cg14369777 | DKC1 | 153990478 | cg24376537 | DLG3 | 69665103 |
| cg05206294 | UBE2A | 118708375 | cg14372520 | EDA2R | 65835804 | cg24376810 | ELK1 | 47508758 |
| cg05206587 | GLRA2 | 14547880 | cg14375772 | GLUD2 | 120181969 | cg24389217 | APOOL | 84258886 |
| cg05215598 | ELF4 | 129199609 | cg14381673 | SLITRK2 | 144904883 | cg24392532 | BHLHB9 | 101975840 |
| cg05222959 | GD11 | 153665103 | cg14381745 | TSR2 | 54471253 | cg24395545 | CUL4B | 119709733 |
| cg05223249 | PFKFB1 | 55020520 | cg14383004 | ZNF75D | 134427561 | cg24401049 | ARHGAP6 | 11157158 |
| cg05223760 | PPP1R3F | 49126243 | cg14388993 | ZNF41 | 47342690 | cg24411090 | PLP2 | 49031256 |
| cg05225083 | GPR173 | 53107941 | cg14389582 | CACNA1F | 49083474 | cg24443136 | ATP2B3 | 152801587 |
| cg05226646 | BCOR | 39948256 | cg14395423 | UPRT | 74516489 | cg24447042 | SMARCA1 | 128657893 |
| cg05237470 | DMD | 31526531 | cg14397820 | TSPYL2 | 53112860 | cg24455325 | SLC38A5 | 48323782 |
| cg05239824 | NKAP | 119074770 | cg14398372 | CLCN5 | 49686139 | cg24461939 | HDAC8 | 71793047 |
| cg05240083 | DOCK11 | 117629034 | cg14403309 | PRRG1 | 37213086 | cg24464999 | RAI2 | 17879701 |
| cg05254049 | SYN1 | 47479662 | cg14429073 | CITED1 | 71526179 | cg24468804 | UPF3B | 118987251 |
| cg05257898 | NKAP | 119078173 | cg14430524 | PJA1 | 68385397 | cg24479484 | MECP2 | 153362589 |
| cg05272837 | PHF8 | 54071268 | cg14452204 | MOSPD1 | 134046597 | cg24490154 | BCORL1 | 129122111 |

EP 4 495 263 A1

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05275343 | PIGA | 15353925 | cg14454242 | HDAC6 | 48683061 | cg24504300 | TIMM8A | 100604063 |
| cg05288740 | AMOT | 112020662 | cg14457256 | ACSL4 | 108977252 | cg24505863 | AMOT | 112084143 |
| cg05290899 | SLC35A2 | 48769310 | cg14457691 | ARMCX4 | 100740937 | cg24507210 | AMOT | 112084814 |
| cg05294909 | SCML1 | 17756060 | cg14460470 | MORF4L2 | 102942485 | cg24508310 | BCOR | 39947559 |
| cg05295417 | CXorf40A | 148623231 | cg14464718 | TMEM35 | 100334126 | cg24511534 | RP2 | 46696351 |
| cg05295688 | PHF16 | 46771201 | cg14479037 | MORC4 | 106242642 | cg24533311 | DDX26B | 134656011 |
| cg05302110 | DGKK | 50214570 | cg14493612 | TBC1D25 | 48399110 | cg24546622 | SLC10A3 | 153719308 |
| cg05302212 | CHST7 | 46433015 | cg14505980 | CXorf67 | 51149711 | cg24547622 | ACOT9 | 23761653 |
| cg05327750 | RBM41 | 106361572 | cg14506668 | FHL1 | 135228853 | cg24548265 | ZNF275 | 152602151 |
| cg05347431 | GPC4 | 132547568 | cg14508997 | SYN1 | 47475578 | cg24552529 | HSD17B10 | 53461540 |
| cg05361475 | ZDHHC15 | 74744538 | cg14513804 | UTP14A | 129040123 | cg24552876 | PLXNA3 | 153695707 |
| cg05363666 | PRPS1 | 106872025 | cg14520512 | MBNL3 | 131547697 | cg4557160 | MCF2 | 138690622 |
| cg05365121 | ZNF275 | 152599997 | cg14520892 | POLA1 | 24711633 | cg24559073 | ZNF41 | 47342471 |
| cg05370638 | DYNLT3 | 37706736 | cg14522327 | SLC38A5 | 48326779 | cg24562355 | GPKOW | 48976141 |
| cg5371395 | FRMPD4 | 12156894 | cg14533133 | SLITRK2 | 144902897 | cg24565395 | PLXNA3 | 153696700 |
| cg05373692 | ZDHHC15 | 74743375 | cg14533874 | PHF8 | 54071285 | cg24585667 | MIDI | 10584246 |
| cg05374090 | FAM58A | 152864599 | cg14550383 | SAT1 | 23801656 | cg24590452 | CDKL5 | 18458900 |
| cg05379196 | HMGB3 | 150151580 | cg14562990 | LDOC1 | 140271626 | cg24596823 | GLRA2 | 14547320 |
| cg05385216 | ATP11C | 138915704 | cg14570389 | RAP2C | 131351543 | cg24597825 | WDR44 | 117480246 |
| cg05397738 | PGRMC1 | 118369571 | cg14586560 | GPC3 | 133118453 | cg24606370 | TMEM187 | 153245332 |
| cg05397813 | ZNF275 | 152599146 | cg14600040 | GPC4 | 132549764 | cg24616736 | HDAC6 | 48659713 |
| cg05400670 | STARD8 | 67867332 | cg14601312 | MAP7 D3 | 135333917 | cg24618413 | BCOR | 40007471 |
| cg05407550 | F8 | 154250784 | cg14603520 | BGN | 152760248 | cg24630097 | HDAC8 | 71787640 |
| cg05414241 | NUDT11 | 51240059 | cg14604474 | PHF6 | 133507642 | cg24654385 | MOSPD1 | 134049411 |
| cg05414556 | SASH3 | 128913922 | cg14605748 | AMOT | 11206508 | cg24655865 | HUWE1 | 53560148 |
| cg05418443 | PGRMC1 | 118370461 | cg14622782 | CASK | 41782111 | cg24657313 | PGK1 | 77359739 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05418779 | MCF2 | 138771271 | cg14625604 | UBE2A | 118708625 | cg24658899 | DLG3 | 69664580 |
| cg05423956 | MPP1 | 154030224 | cg14637830 | PLXNA3 | 153692253 | cg24663206 | DLG3 | 69664617 |
| cg05424879 | SLC10A3 | 153719176 | cg14642832 | RBMX | 135963137 | cg24663315 | SLC16A2 | 73640043 |
| cg05425577 | FGD1 | 54522221 | cg14644616 | ARAF | 47421098 | cg24670916 | PDZD4 | 153071026 |
| cg05427163 | TBC1D25 | 48397938 | cg14680768 | STAG2 | 123094326 | cg24671891 | FRMPD4 | 12601783 |
| cg05434903 | KLHL13 | 117251118 | cg14688956 | PAK3 | 110339862 | cg24672310 | GATA1 | 48644026 |
| cg05437059 | KCNE1L | 108868775 | cg14694234 | TCEAL4 | 102839366 | cg24678243 | PFKFB1 | 55021561 |
| cg05438092 | FLNA | 153603066 | cg14699257 | USP11 | 47092993 | cg24690818 | AMOT | 112084127 |
| cg05441864 | ACSL4 | 108976893 | cg14699343 | AIFM1 | 129271092 | cg24711407 | HCCS | 11129171 |
| cg05445331 | GPC3 | 133119028 | cg14699869 | TREX2 | 152710205 | cg24713382 | PRDX4 | 23689686 |
| cg05445588 | SH3KBP1 | 19818774 | cg14701129 | KLF8 | 56258113 | cg24714634 | FAM50A | 153672805 |
| cg05446138 | SMPX | 21776242 | cg14708847 | PAK3 | 110339313 | cg24721916 | SLC25A14 | 129473599 |
| cg05449830 | CITED1 | 71527091 | cg14711543 | TCEAL2 | 101380592 | cg24735671 | TLR7 | 12883760 |
| cg05450314 | GPRASP2 | 101968430 | cg14712359 | TBC1D8B | 106045737 | cg24737468 | PAK3 | 110188323 |
| cg05453458 | ARMCX3 | 100878340 | cg14713449 | PDZD4 | 153070180 | cg24738321 | FLNA | 153577041 |
| cg05455393 | FHL1 | 135228215 | cg14725109 | ZNF81 | 47696106 | cg24741392 | RPGR | 38186472 |
| cg05462236 | MTMR8 | 63615456 | cg14725537 | PLP1 | 103031822 | cg24741770 | FAAH2 | 57312189 |
| cg05464374 | CITED1 | 71525859 | cg14743649 | CENPI | 100353353 | cg24748621 | WDR45 | 48958121 |
| cg05473496 | BCOR | 39953435 | cg14745824 | EDA | 68979753 | cg24761863 | BCORL1 | 129190003 |
| cg05475280 | VMA21 | 150569790 | cg14745925 | FTSJ1 | 48334240 | cg24762459 | PJA1 | 68381019 |
| cg05476522 | FGD1 | 54521732 | cg14755341 | CDKL5 | 18444361 | cg24764759 | MAGEB16 | 35816510 |
| cg05478253 | KLHL13 | 117252422 | cg14766682 | CCDC120 | 48916658 | cg24765005 | BEX2 | 102565608 |
| cg05491276 | EDA | 68836772 | cg14772068 | MAGEH1 | 55479202 | cg24772920 | TRMT2B | 100307180 |
| cg05494459 | FTSJ1 | 48334337 | cg14773858 | FLNA | 153603488 | cg24774956 | SH3KBP1 | 19907122 |
| cg05511138 | ARHGAP6 | 11682622 | cg14794494 | PGK1 | 77359564 | cg24779040 | TSPYL2 | 53111607 |
| cg05511752 | MED12 | 70338656 | cg14805868 | ZDHHC9 | 128978072 | cg24780240 | EFNB1 | 68052409 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05512407 | SATL1 | 84350393 | cg14805882 | TCEAL2 | 101381594 | cg24783624 | ZBTB33 | 119384693 |
| cg05515118 | BEX2 | 102565934 | cg14807303 | AR | 66763445 | cg24787889 | SLC35A2 | 48768632 |
| cg05516935 | RRAGB | 55744028 | cg14812623 | SLC25A14 | 129474169 | cg24790801 | BEX4 | 102470074 |
| cg05526804 | ZNF41 | 47342124 | cg14814331 | DRP2 | 100474543 | cg24805886 | SMPX | 21776400 |
| cg05532720 | ARMCX3 | 100880276 | cg14815778 | FAM58A | 152865295 | cg24814801 | MAGT1 | 77151194 |
| cg05534333 | LAGE3 | 153707029 | cg14824933 | UBQLN2 | 56589949 | cg24816350 | CITED1 | 71526972 |
| cg05545272 | MAOB | 43741376 | cg14828865 | OPHN1 | 67653156 | cg24817502 | KCNE1L | 108868931 |
| cg05555725 | FHL1 | 135251416 | cg14831390 | GK | 30671327 | cg24818524 | FRMPD4 | 12155663 |
| cg05558522 | SNX12 | 70288033 | cg14834143 | LONRF3 | 118109844 | cg24818939 | GPRASP1 | 101906109 |
| cg05559023 | BCOR | 39952171 | cg14841098 | SLC9A7 | 46618968 | cg24822140 | ZNF81 | 47760424 |
| cg05559795 | KLF8 | 56258812 | cg14854487 | LAMP2 | 119603098 | cg24823082 | MID11P1 | 38660587 |
| cg05563347 | APOOL | 84257832 | cg14867863 | GPC3 | 133119961 | cg24826812 | DDX26B | 134654612 |
| cg05564395 | PRAF2 | 48929391 | cg14870234 | ARMCX3 | 100879017 | cg24829975 | ARMCX3 | 100878548 |
| cg05570079 | MBTPS2 | 21856948 | cg14871604 | IGBP1 | 69353710 | cg24832428 | ARHGEF6 | 135849827 |
| cg05570609 | ARHGEF6 | 135852617 | cg14873818 | HDX | 83757652 | cg24834461 | DNASE1L1 | 153637645 |
| cg05572164 | PIGA | 15349961 | cg14886579 | OPHN1 | 67351469 | cg24841909 | PHKA2 | 18998506 |
| cg05576548 | ELK1 | 47500695 | cg14892037 | SNX12 | 70288026 | cg24844608 | HCCS | 11129831 |
| cg05578291 | KLHL15 | 24042365 | cg14923176 | SLC9A6 | 135071071 | cg24845318 | FRMPD4 | 12156532 |
| cg05585317 | TSPYL2 | 53117682 | cg14927076 | MID11P1 | 38663045 | cg24850178 | RBM41 | 106362074 |
| cg05587158 | ARR3 | 69487748 | cg14931238 | RAB41 | 69504569 | cg24880787 | SLITRK2 | 144903661 |
| cg05592527 | ARMCX6 | 100872988 | cg4949922 | RP2 | 46696330 | cg24881209 | MAGIX | 49019150 |
| cg05599151 | TSPYL2 | 53111398 | cg14951132 | NRK | 105067038 | cg24888621 | DDX26B | 134654536 |
| cg05605944 | MAOB | 43741945 | cg14961598 | MTMR8 | 63614857 | cg24922864 | CDR1 | 139866080 |
| cg05630320 | HPRT1 | 133594116 | cg14961988 | MED12 | 70338337 | cg24925526 | ARAF | 47420577 |
| cg05633597 | KLF8 | 56258193 | cg14964387 | WDR45 | 48958082 | cg24925698 | TBC1D25 | 48397615 |
| cg05673346 | MID11P1 | 38662707 | cg14972002 | FAM122C | 133941107 | cg24931094 | MCTS1 | 119737891 |

64

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05682970 | TMLHE | 154839539 | cg14973366 | FLNA | 153599377 | cg24931882 | CASK | 41782328 |
| cg05685296 | BCOR | 40020363 | cg14986420 | YIPF6 | 67718299 | cg24932289 | BCOR | 40036253 |
| cg05688478 | APLN | 128789194 | cg14990368 | PHF6 | 133507339 | cg24964466 | HAUS7 | 152735785 |
| cg05695951 | CHST7 | 46437318 | cg14993186 | RPS6KA6 | 83443017 | cg24971873 | KIAA2022 | 74144705 |
| cg05699139 | DIAPH2 | 95940153 | cg14998855 | ELK1 | 47507483 | cg24974356 | PLP1 | 103035890 |
| cg05707815 | PHKA1 | 71934233 | cg15024277 | NGFRAP1 | 102632067 | cg24976080 | TIMM8A | 100604037 |
| cg05720573 | DYNLT3 | 37703454 | cg15024309 | HDX | 83757693 | cg24980481 | SMS | 21968128 |
| cg05721877 | BCOR | 39953075 | cg15027606 | GPR173 | 53077819 | cg25051948 | TBC1D25 | 48397977 |
| cg05740522 | ENOX2 | 130036828 | cg15029285 | CXorf36 | 45060311 | cg25052176 | WNK3 | 54385662 |
| cg05740959 | CACNA1F | 49087234 | cg15029878 | RBM10 | 47046178 | cg25053301 | WDR13 | 48456550 |
| cg05782651 | AMMECR1 | 109561747 | cg15039826 | BCOR | 39958196 | cg25063710 | BRWD3 | 80064750 |
| cg05782751 | MCTS1 | 119737675 | cg15041487 | WDR45 | 48958242 | cg25085925 | AR | 66763691 |
| cg05782975 | NGFRAP1 | 102631408 | cg15042310 | BCOR | 39948097 | cg25124605 | BCORL1 | 129116500 |
| cg05783039 | MID1 | 10851898 | cg15043283 | FAM127A | 134166205 | cg25125622 | SLITRK2 | 144900458 |
| cg05784171 | LRCH2 | 114465952 | cg15043492 | DKC1 | 153990749 | cg25125652 | CHRDL1 | 110039604 |
| cg05785344 | BCORL1 | 129118279 | cg15048430 | WNK3 | 54384161 | cg25125864 | CACNA1F | 49086910 |
| cg05786601 | AR | 66764126 | cg15050093 | RPL39 | 118925759 | cg25126296 | PHKA1 | 71897854 |
| cg05799859 | FAM3A | 153744612 | cg15057941 | KCNE1L | 108867725 | cg25130333 | CD99L2 | 150067942 |
| cg05803370 | UBL4A | 153715241 | cg15068728 | STARD8 | 67913143 | cg25131597 | OPHN1 | 67423243 |
| cg05803913 | MID1IP1 | 38664442 | cg15075851 | HPRT1 | 133593945 | cg25131897 | XPNPEP2 | 128893354 |
| cg05803942 | STARD8 | 67906758 | cg15082071 | MAGED1 | 51546021 | cg25141133 | FRMPD4 | 12156437 |
| cg05804339 | BCOR | 40036023 | cg15132216 | PLP2 | 49028291 | cg25153266 | APOOL | 84258452 |
| cg05826817 | ACOT9 | 23760460 | ra15133344 | BCOR | 40034881 | cg25153755 | SYN1 | 47433716 |
| cg05832562 | ARMCX3 | 100878294 | cg15144825 | FAM127B | 134186281 | cg25156485 | MSN | 64887827 |
| cg05833518 | CLCN5 | 49832094 | cg15159291 | NUDT10 | 51074953 | cg25158628 | RAI2 | 17879602 |
| cg05835545 | BRWD3 | 80065003 | cg15162843 | MID1IP1 | 38660509 | cg25165659 | UBE2A | 118708113 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05849149 | EFNB1 | 68048224 | cg15165694 | SLC25A43 | 118533179 | cg25166062 | WDR45 | 48958423 |
| cg05856884 | SMPX | 21776372 | cg15167893 | EDA | 68836774 | cg25172243 | MCF2 | 138724688 |
| cg05858130 | PRAF2 | 48929808 | cg15170964 | TSPAN7 | 38420418 | cg25179313 | ARMCX3 | 100878309 |
| cg05860920 | ARHGAP6 | 11679501 | cg15199886 | SYP | 49056688 | cg25198830 | NGFRAP1 | 102629912 |
| cg05861409 | SLC25A43 | 118533164 | cg15200096 | KCND1 | 48829104 | cg25205946 | WDR45 | 48958189 |
| cg05866836 | TSC22D3 | 106960378 | cg15208718 | ZNF630 | 47925446 | cg25206026 | MOSPD1 | 134049257 |
| cg05872215 | SASH3 | 128913854 | cg15224006 | PPP1R3F | 49125404 | cg25206760 | MAGED1 | 51636664 |
| cg05872808 | ATP6AP1 | 153657411 | cg15225044 | NKAP | 119077171 | cg25220235 | MID1IP1 | 38663916 |
| cg05876899 | PDZD4 | 153072756 | cg15230046 | GPRASP1 | 101909850 | cg25220716 | MCF2 | 138791201 |
| cg05877833 | TRO | 54957261 | cg15231886 | UTP14A | 129039863 | cg25225807 | ARHGEF9 | 62974844 |
| cg05883299 | BHLHB9 | 101997645 | cg15235844 | CYBB | 37671328 | cg25257360 | CXorf23 | 19983591 |
| cg05887304 | PRPS2 | 12809548 | cg15236541 | SLC16A2 | 73642618 | cg25258879 | MAGIX | 49022996 |
| cg05892376 | ARHGEF6 | 135862961 | cg15237731 | MAP7D2 | 20042464 | cg25270201 | WWC3 | 9984045 |
| cg05895666 | SLC25A43 | 118532787 | cg15241084 | TLR7 | 12886121 | cg25279183 | ZDHHC9 | 128977842 |
| cg05896813 | MAGED1 | 51636892 | cg15245437 | PDZD4 | 153072605 | cg25280509 | FAM58A | 152865012 |
| cg05897571 | CXorf67 | 51148629 | cg15259400 | RBM41 | 106362067 | cg25280589 | IGBP1 | 69353974 |
| cg05899999 | TIMM8A | 100603765 | cg15259889 | PCYT1B | 24665506 | cg25289658 | FHL1 | 135278646 |
| cg05903800 | SUV39H1 | 48554185 | cg15299258 | ZC4H2 | 64196688 | cg25298085 | ARHGAP6 | 11621141 |
| cg05905814 | HMGB3 | 150151809 | cg15308487 | PDK3 | 24483292 | cg25301621 | MAGT1 | 77149220 |
| cg05911704 | RAB33A | 129304869 | cg15309236 | BHLHB9 | 101975597 | cg25308044 | CSAG1 | 151909300 |
| cg05921207 | CHRDL1 | 110038886 | cg15314890 | BCOR | 40031314 | cg25308681 | CXorf23 | 19988480 |
| cg05922253 | DGKK | 50213977 | cg15317375 | ZNF275 | 152608863 | cg25316166 | USP11 | 47092288 |
| cg05935584 | HMGB3 | 150151823 | cg15317927 | MID1IP1 | 38665168 | cg25317260 | AIFM1 | 129300090 |
| cg05939965 | UPF3B | 118983320 | cg15322230 | BCOR | 40015467 | cg25355896 | PAK3 | 110365767 |
| cg05943154 | ARHGEF6 | 135845705 | cg15322420 | MORF4L2 | 102942967 | cg25359581 | DYNLT3 | 37706819 |
| cg05946161 | BCOR | 40006261 | cg15324340 | PRPS1 | 106871509 | cg25364049 | APLN | 128788749 |

66

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg05947181 | SHROOM4 | 50557283 | cg15334207 | DCX | 110654829 | cg25366157 | RP2 | 46696142 |
| cg05950094 | REPS2 | 16964965 | cg15336560 | HDX | 83757519 | cg25373998 | NLGN3 | 70363458 |
| cg05961595 | APEX2 | 55026485 | cg15339180 | NLGN3 | 70364545 | cg25377867 | PLP2 | 49026774 |
| cg05965444 | MOSPD1 | 134049328 | cg15345580 | PDK3 | 24483294 | cg25395546 | MST4 | 131157336 |
| cg05996419 | MID1IP1 | 38663393 | cg15356502 | PHF6 | 133509972 | cg25410279 | LAGE3 | 153707517 |
| cg05999377 | TREX2 | 152712061 | cg15363435 | CACNA1F | 49087477 | cg25413720 | SAT1 | 23801037 |
| cg06010380 | BCOR | 40011867 | cg15363890 | IDS | 148587383 | cg25424856 | SHROOM4 | 50557036 |
| cg06016684 | MAP7D2 | 20134557 | cg15365038 | PRAF2 | 48930360 | cg25427918 | CLCN5 | 49687176 |
| cg06027835 | WDR13 | 48456564 | cg15366127 | BCOR | 40003935 | cg25434856 | SYP | 49050740 |
| cg06041240 | NXT2 | 108778793 | cg15374982 | CLCN4 | 10157676 | cg25441219 | PLXNA3 | 153699453 |
| cg06042004 | LAGE3 | 153707710 | cg15383797 | AKAP4 | 49965284 | cg25445853 | FHL1 | 135230696 |
| cg06042574 | DMD | 33231137 | cg15388264 | PHKA1 | 71933990 | cg25449245 | RPL39 | 118925442 |
| cg06046085 | FAM3A | 153745887 | cg15391239 | FLNA | 153603171 | cg25450034 | ENOX2 | 129806493 |
| cg06046696 | TMEM185A | 148714569 | cg15392343 | GRIPAP1 | 48858832 | cg25452717 | RPS6KA6 | 83442847 |
| cg06051391 | RAP2C | 131352293 | cg15418221 | PGK1 | 77359544 | cg25456959 | ASB12 | 63446044 |
| cg06051662 | PDZD4 | 153096786 | cg15443127 | RAB41 | 69501934 | cg25488547 | CHM | 85303309 |
| cg06053110 | MAGED1 | 51636681 | cg15450752 | PRPS2 | 12812615 | cg25492195 | HCCS | 11129297 |
| cg06055266 | TSPYL2 | 53111187 | cg15450782 | FAM120C | 54209682 | cg25493146 | PDHA1 | 19361210 |
| cg06055478 | ARHGEF9 | 62974905 | cg15450986 | ARMCX4 | 100673417 | cg25496792 | GNL3L | 54557002 |
| cg06055803 | OCRL | 128724288 | cg15454357 | PORCN | 48368144 | cg25497053 | WWC3 | 10087726 |
| cg06068891 | FHL1 | 135228207 | cg15454483 | SNX12 | 70288604 | cg25499687 | PHF6 | 133507336 |
| cg06069996 | TSR2 | 54466836 | cg15465300 | PRPS1 | 106893350 | cg25504143 | AMMECR1 | 109473163 |
| cg06072560 | CSTF2 | 100075379 | cg15465809 | PRAF2 | 48931782 | cg25514427 | XIAP | 122994594 |
| cg06079646 | POLA1 | 24711474 | cg15474769 | TSPYL2 | 53113522 | cg25525476 | TMEM185A | 148712902 |
| cg06079963 | POLA1 | 24711994 | cg15480941 | PIM2 | 48776125 | cg25528264 | IGBP1 | 69353387 |
| cg06084034 | DNASE1L1 | 153637827 | cg15490039 | LDOC1 | 140271719 | cg25529319 | TMLHE | 154842320 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06102971 | RAI2 | 17878442 | cg15508284 | TMEM187 | 153248051 | cg25529889 | HCCS | 11129757 |
| cg06104510 | PHKA2 | 19002482 | cg15511898 | BRWD3 | 80065754 | cg25537319 | ARL13A | 100224631 |
| cg06104959 | STARD8 | 67913324 | cg15516728 | UPRT | 74524038 | cg25538342 | AR | 66944044 |
| cg06106988 | TREX2 | 152712041 | cg15517690 | FLNA | 153599656 | cg25547663 | WDR13 | 48456173 |
| cg06107108 | DLG3 | 69723652 | cg15520863 | C1GALT1C1 | 119763941 | cg25547772 | DGKK | 50213681 |
| cg06109624 | MCF2 | 138664151 | cg15529512 | ARHGAP6 | 11161960 | cg25549819 | RAP2C | 131352230 |
| cg06119392 | CD99L2 | 150067307 | cg15534364 | CITED1 | 71527089 | cg25555657 | FTSJ1 | 48334381 |
| cg06122016 | RBM10 | 47039463 | cg15536552 | ACSL4 | 108976035 | cg25556752 | CLCN4 | 10124797 |
| cg06126323 | EDA | 68836571 | cg15554342 | SYP | 49046065 | cg25557806 | NSDHL | 152031029 |
| cg06128881 | SLC9A7 | 46616842 | cg15554694 | KLHL4 | 86772364 | cg25558406 | HPRT1 | 133594204 |
| cg06130360 | DOCK11 | 117633362 | cg15565409 | SLC25A43 | 118533172 | cg25560327 | ZNF185 | 152127478 |
| cg06131666 | SCML2 | 18373515 | cg15568009 | TMEM185A | 148713744 | cg25565608 | ZNF630 | 47930609 |
| cg06135529 | UPF3B | 118986703 | cg15570803 | SNX12 | 70288412 | cg25571060 | PJA1 | 68385381 |
| cg06135650 | MID1IP1 | 38662916 | cg15579650 | GPRASP1 | 101906119 | cg25576048 | ARMCX1 | 100805516 |
| cg06136002 | HMGB3 | 150151615 | cg15582794 | BEX4 | 102470223 | cg25619303 | BCOR | 40017965 |
| cg06137651 | RGN | 46940664 | cg15586998 | NXT2 | 108779030 | cg25622910 | MCTS1 | 119737885 |
| cg06139288 | ARHGAP6 | 11683125 | cg15590532 | FRMPD4 | 12604752 | cg25624831 | GPRASP2 | 101967183 |
| cg06140460 | KCND1 | 48828397 | cg15594578 | PHKA1 | 71800011 | cg25626441 | MAGT1 | 77152114 |
| cg06140761 | CCDC120 | 48915032 | cg15604347 | LRCH2 | 114420262 | cg25630838 | STARD8 | 67913165 |
| cg06143713 | ARHGEF9 | 62856362 | cg15613687 | F8 | 154252172 | cg25634978 | MAGED1 | 51546036 |
| cg06144608 | MTM1 | 149737646 | cg15614573 | FOXP3 | 49122364 | cg25640635 | ARMCX4 | 100740535 |
| cg06144999 | HCCS | 11129626 | cg15622077 | MAP7D2 | 20135978 | cg25645198 | GK | 30675444 |
| cg06145195 | ZNF185 | 152107440 | cg15627188 | BCOR | 40005337 | cg25645559 | BEX4 | 102470643 |
| cg06148972 | MTM1 | 149736729 | cg15658365 | SYN1 | 47437398 | cg25645693 | MPP1 | 154033932 |
| cg06152508 | TKTL1 | 153533551 | cg15659974 | SLC6A14 | 115567527 | cg25647989 | BRWD3 | 80061160 |
| cg06157561 | PCYT1B | 24690870 | cg15661671 | UBE2A | 118708349 | cg25656981 | KLF8 | 56262829 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06170215 | CITED1 | 71525152 | cg15662246 | PIN4 | 71401536 | cg25676950 | KCND1 | 48828779 |
| cg06172119 | SHROOM4 | 50337553 | cg15667675 | RAP2C | 131351164 | cg25685741 | HMGB3 | 150151769 |
| cg06172626 | TBC1D25 | 48398133 | cg15670241 | AMOT | 112084007 | cg25686125 | AR | 66763009 |
| cg06190216 | BGN | 152771994 | cg15672146 | AMOT | 112084792 | cg25692732 | UBL4A | 153715426 |
| cg06204009 | ATP2B3 | 152801503 | cg15681351 | UPF3B | 118987163 | cg25697726 | UBQLN2 | 56589918 |
| cg06206449 | UBE2A | 118708409 | cg15682807 | ITM2A | 78622682 | cg25698940 | CUL4B | 119695102 |
| cg06208111 | ITM2A | 78624108 | cg15685776 | FAM127B | 134186273 | cg25708850 | BRWD3 | 80065793 |
| cg06217046 | WWC3 | 10094247 | cg15694789 | STARD8 | 67915858 | cg25710140 | MID1 | 10852037 |
| cg06220755 | RAI2 | 17821058 | cg15715844 | PGRMC1 | 118369789 | cg25713737 | MBNL3 | 131574228 |
| cg06228453 | MORC4 | 106242522 | cg15735483 | MAP7D2 | 20135349 | cg25714218 | HMGB3 | 150151618 |
| cg06232789 | MAGED2 | 54833768 | cg15737470 | CLCN4 | 10124914 | cg25714610 | FAM3A | 153744216 |
| cg06232846 | NLGN3 | 70389504 | cg15737490 | NLGN3 | 70390672 | cg25717578 | CASK | 41778212 |
| cg06234064 | FLNA | 153584449 | cg15757320 | NKRF | 118740401 | cg25737241 | MAOB | 43739887 |
| cg06238055 | DGKK | 50211642 | cg15766608 | SPIN3 | 57021771 | cg25766534 | FAM104B | 55187322 |
| cg06252876 | AIFM1 | 129299948 | cg15769969 | AIFM1 | 129299941 | cg25775033 | TREX2 | 152711884 |
| cg06253229 | PLP1 | 103030593 | cg15770613 | GLUD2 | 120183585 | cg25777540 | GPRASP1 | 101906428 |
| cg06266461 | SLC10A3 | 153719192 | cg15771339 | NKAP | 119077447 | cg25790081 | YIPF6 | 67718547 |
| cg06267203 | OPHN1 | 67356424 | cg15771597 | PORCN | 48367980 | cg25790279 | FLNA | 153602509 |
| cg06269080 | PHKA2 | 19003031 | cg15802548 | AMMECR1 | 109561793 | cg25790968 | TMEM164 | 109420139 |
| cg06270497 | STARD8 | 67906442 | cg15804018 | CDKL5 | 18459878 | cg25812668 | MID1IP1 | 38662926 |
| cg06272245 | TBC1D25 | 48398018 | cg15806723 | SYP | 49056711 | cg25813447 | MID1IP1 | 38660511 |
| cg06273903 | UBE2A | 118707913 | ra15813836 | GPR64 | 19141093 | cg25813820 | MPP1 | 154033598 |
| cg06277838 | SLC16A2 | 73641370 | cg15814001 | DMD | 31285492 | cg25821416 | SYN1 | 47431407 |
| cg06288270 | RBMX | 135963596 | cg15815114 | CUL4B | 119695227 | cg25832410 | PDZD4 | 153096039 |
| cg06289882 | RBMX2 | 129535465 | cg15819935 | MOSPD2 | 14893979 | cg25837617 | ZNF275 | 152599662 |
| cg06293983 | ARHGEF6 | 135863104 | cg15824287 | FAM104B | 55187561 | cg25841909 | CHM | 85302794 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06295352 | ENOX2 | 130037150 | cg15837118 | NUDT11 | 51233377 | cg25845235 | SUV39H1 | 48558950 |
| cg06298190 | ATP11C | 138913929 | cg15840039 | AR | 66788098 | cg25846190 | GRIPAP1 | 48858796 |
| cg06298361 | CCDC22 | 49091907 | cg15840253 | AMMECR1 | 109439501 | cg25857333 | DMD | 31285204 |
| cg06300107 | MTMR8 | 63616425 | cg15841434 | PORCN | 48367203 | cg25858008 | PHKA1 | 71934184 |
| cg06302025 | BHLHB9 | 102000758 | cg15844109 | LPAR4 | 78003205 | cg25863147 | PDK3 | 24484361 |
| cg06302218 | BEX2 | 102566123 | cg15847896 | BTK | 100607195 | cg25870731 | LDOC1 | 140271029 |
| cg06306751 | F8 | 154251005 | cg15850513 | USP11 | 47091513 | cg25873934 | ARHGAP6 | 11446095 |
| cg06307153 | ZNF75D | 134429853 | cg15855671 | ARHGEF6 | 135863688 | cg25874079 | RLIM | 73834484 |
| cg06323885 | TCEAL1 | 102883827 | cg15858894 | GRIPAP1 | 48858776 | cg25877387 | AKAP4 | 49965358 |
| cg06327799 | ZC4H2 | 64196367 | cg15864992 | MCTS1 | 119736432 | cg25880538 | EDA | 68840309 |
| cg06334238 | IRAK1 | 153284899 | cg15870809 | MAP7D2 | 20134805 | cg25881824 | FAM127B | 134186640 |
| cg06340713 | CHM | 85303427 | cg15872383 | WWC3 | 10087808 | cg25883790 | FHL1 | 135251484 |
| cg06341336 | APEX2 | 55026590 | cg15874629 | MAOB | 43741557 | cg25888867 | IGSF1 | 130423014 |
| cg06363801 | MOSPD1 | 134049271 | cg15879104 | FAM50A | 153674100 | cg25892640 | PRPS2 | 12809773 |
| cg06372522 | PGRMC1 | 118374789 | cg15879692 | FLNA | 153603126 | cg25896944 | PRDX4 | 23685894 |
| cg06375559 | RS1 | 18690400 | cg15887754 | BCOR | 39949685 | cg25897349 | GAB3 | 153979507 |
| cg06376033 | TCEAL1 | 102883934 | cg15891447 | TRMT2B | 100306812 | cg25903474 | CHRDL1 | 110039472 |
| cg06380355 | NUDT11 | 51239518 | cg15892676 | ASB12 | 63444159 | cg25905965 | MTMR1 | 149861779 |
| cg06384491 | SPIN2A | 57163888 | cg15895359 | EFNB1 | 68048613 | cg25910467 | THOC2 | 122865777 |
| cg06384623 | GPC3 | 133119308 | cg15898026 | WDR45 | 48937418 | cg25916188 | PDZD4 | 153087306 |
| cg06408185 | CDKL5 | 18443714 | cg15904420 | CCNB3 | 50074134 | cg25918359 | ELF4 | 129244732 |
| cg06408301 | PRRG1 | 37208607 | cg15904427 | FAM122C | 133941745 | cg25927711 | LAS1L | 64754995 |
| cg06408533 | STARD8 | 67867352 | cg15917060 | MSN | 64891199 | cg25933726 | RPGR | 38186399 |
| cg06411441 | TRMT2B | 100307072 | cg15917786 | ARHGEF9 | 62975151 | cg25937978 | CDR1 | 139866322 |
| cg06428055 | ELF4 | 129243872 | cg15919369 | MAGT1 | 77151050 | cg25941716 | SH3KBP1 | 19905525 |
| cg06431415 | GPC3 | 133115985 | cg15922956 | FUNDC2 | 154254964 | cg25943872 | SLC16A2 | 73642385 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06438727 | LRCH2 | 114430258 | cg15954878 | DMD | 33229714 | cg25943986 | RPS6KA6 | 83442397 |
| cg06438901 | WDR45 | 48937877 | cg15958715 | PPP1R3F | 49127115 | cg25947525 | DLG3 | 69674902 |
| cg06439589 | SSR4 | 153061818 | cg15977272 | EFNB1 | 68048909 | cg25950739 | GPRASP1 | 101907254 |
| cg06442736 | PORCN | 48368034 | cg16022279 | ZNF185 | 152086723 | cg25958561 | PAK3 | 110187179 |
| cg06448666 | MID1 | 10645625 | cg16049598 | MORC4 | 106243646 | cg25967516 | ARMCX1 | 100805271 |
| cg06455967 | PAK3 | 110187421 | cg16059374 | EDA | 68835678 | cg25968748 | IL13RA1 | 117862288 |
| cg06457149 | LRCH2 | 114468096 | cg16062292 | TKTL1 | 153558585 | cg25969791 | EDA | 69080800 |
| cg06457357 | KLF8 | 56259243 | cg16063272 | TMSB15A | 101771222 | cg25972680 | CAPN6 | 110489914 |
| cg06461462 | TSC22D3 | 106960878 | cg16065628 | NUDT11 | 51237838 | cg25987936 | AR | 66765446 |
| cg06462425 | TBC1D8B | 106045920 | cg16068020 | TMSB15A | 101770958 | cg25996557 | FRMPD4 | 12158396 |
| cg06464066 | REPS2 | 16964808 | cg16068924 | HDAC6 | 48682043 | cg25999375 | DMD | 31287275 |
| cg06472116 | RGN | 46937653 | cg16078210 | MAGT1 | 77151179 | cg26000542 | MAOB | 43741890 |
| cg06475150 | WDR45 | 48937814 | cg16082125 | USP11 | 47092560 | cg26003280 | AR | 66762467 |
| cg06476341 | SLITRK2 | 144903005 | cg16091746 | VBP1 | 154444538 | cg26004099 | GPR173 | 53078072 |
| cg06479978 | MST4 | 131161297 | cg16091895 | TMEM185A | 148713715 | cg26010110 | SMARCA1 | 128657699 |
| cg06481089 | NUDT10 | 51075797 | cg16097355 | RLIM | 73834332 | cg26010707 | KLHL13 | 117250381 |
| cg06482328 | ZBTB33 | 119385834 | cg16107992 | HDX | 83756854 | cg26014066 | AR | 66763094 |
| cg06489418 | FTSJ1 | 48334722 | cg16116006 | DMD | 31526745 | cg26016246 | BEX2 | 102564442 |
| cg06492112 | IDS | 148587249 | cg16126759 | RRAGB | 55743906 | cg26020914 | CDKL5 | 18444359 |
| cg06494770 | KLHL13 | 117107675 | cg16135771 | PLP1 | 103030149 | cg26023405 | DOCK11 | 117629911 |
| cg06504563 | LAS1L | 64754017 | cg16136096 | MCTS1 | 119744207 | cg26028155 | NYX | 41334113 |
| cg06505213 | SLC35A2 | 48769091 | cg16140952 | CHRDL1 | 110039485 | cg26032662 | TSPAN7 | 38421011 |
| cg06505768 | RAB33A | 129309750 | cg16141290 | ALAS2 | 55058095 | cg26046341 | BCOR | 40023353 |
| cg06511189 | FAM58A | 152864830 | cg16149200 | GLUD2 | 120183084 | cg26046924 | MTMR8 | 63616811 |
| cg06521531 | FHL1 | 135229551 | cg16151342 | HNRNPH2 | 100667571 | cg26051018 | MAGT1 | 77147035 |
| cg06522219 | STARD8 | 67879834 | cg16152676 | CLCN4 | 10126878 | cg26051590 | KLHL13 | 117107897 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06526829 | KCND1 | 48828070 | cg16153146 | BCOR | 40014151 | cg26055054 | DDX26B | 134654347 |
| cg06535161 | RAI2 | 17878715 | cg16159925 | CDR1 | 139866657 | cg26059639 | PRPS2 | 12809315 |
| cg06540859 | CHST7 | 46434696 | cg16161440 | UBQLN2 | 56590187 | cg26064831 | POLA1 | 24711899 |
| cg06544877 | HDX | 83757131 | cg16166036 | WWC3 | 10085753 | cg26068514 | SLC9A7 | 46619235 |
| cg06548617 | BCOR | 40012100 | cg16167304 | COL4A5 | 107784996 | cg26070134 | FAM50A | 153678842 |
| cg06549249 | UTP14A | 129040144 | cg16167673 | STARD8 | 67867262 | cg26101161 | THOC2 | 122736584 |
| cg06551391 | MAGED1 | 51636562 | cg16168153 | SMPX | 21776376 | cg26104731 | PRICKLE3 | 49042631 |
| cg06558166 | GPC4 | 132549003 | cg16170760 | LAGE3 | 153708103 | cg26104800 | RBM10 | 47041779 |
| cg06583412 | BHLHB9 | 102001318 | cg16170944 | HPRT1 | 133594110 | cg26106058 | DMD | 33146493 |
| cg06614969 | MAGED2 | 54835405 | cg16175497 | SLC38A5 | 48328591 | cg26116400 | FUNDC2 | 154254947 |
| cg06616051 | PDZD4 | 153094332 | cg16181613 | PGK1 | 77359860 | cg26119746 | ACSL4 | 108976838 |
| cg06616857 | SLC10A3 | 153719132 | cg16190093 | KLHL15 | 24044093 | cg26121752 | REPS2 | 16964805 |
| cg06621863 | RPS6KA6 | 83443154 | cg16201681 | BCAP31 | 152966020 | cg26122495 | MTMR8 | 63615185 |
| cg06625174 | GAB3 | 153979792 | cg16202017 | NUDT10 | 51074202 | cg26129027 | MID1IP1 | 38662279 |
| cg06625916 | NYX | 41332957 | cg16203368 | ARAF | 47431033 | cg26140100 | APLN | 128784925 |
| cg06630839 | REPS2 | 16964684 | cg16204757 | MSN | 64887739 | cg26144445 | SLC35A2 | 48769075 |
| cg06633205 | DDX26B | 134656158 | cg16206013 | APOO | 23922912 | cg26144456 | NXT2 | 108779006 |
| cg06645047 | GRIPAP1 | 48858904 | cg16211147 | UBE2A | 118708616 | cg26146690 | PLXNA3 | 153686222 |
| cg06646098 | ZBTB33 | 119388798 | cg16213807 | DUSP9 | 152910052 | cg26155828 | MID1IP1 | 38664606 |
| cg06650776 | NGFRAP1 | 102632072 | cg16215037 | KLHL15 | 24006074 | cg26157279 | RPS6KA6 | 83443186 |
| cg06655100 | KLF8 | 56259373 | cg16221895 | EDA | 68835891 | cg26159385 | DUSP9 | 152912611 |
| cg06657741 | SLC9A6 | 135067456 | cg16227684 | GDI1 | 153664862 | cg26176649 | ZNF41 | 47342033 |
| cg06659128 | TMLHE | 154842535 | cg16227775 | ABCB7 | 74376251 | cg26190455 | PRPS2 | 12809173 |
| cg06663923 | MAGIX | 49020767 | cg16235803 | WNK3 | 54384719 | cg26197836 | PLP1 | 103046141 |
| cg06667703 | CXorf57 | 105854952 | cg16242260 | FRMPD4 | 12601679 | cg26207017 | ACOT9 | 23761589 |
| cg06673178 | SHROOM4 | 50557022 | cg16242750 | ARHGAP6 | 11161309 | cg26214064 | FAM133A | 92964855 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06675660 | SMARCA1 | 128657364 | cg16243644 | STAG2 | 123095766 | cg26222407 | STAG2 | 123094884 |
| cg06700462 | PHKA2 | 19002006 | cg16245086 | TIMM8A | 100603828 | cg26222437 | FRMPD4 | 12157128 |
| cg06705078 | TCEAL1 | 102884538 | cg16248169 | FTSJ1 | 48334308 | cg26246138 | SCML2 | 18372612 |
| cg06720669 | KCNE1L | 108868282 | cg16250105 | WDR45 | 48958174 | cg26280904 | MAGED1 | 51635421 |
| cg06721573 | PDK3 | 24483234 | cg16251458 | MTMR8 | 63614980 | cg26288719 | FRMPD4 | 12602509 |
| cg06731599 | ATP6AP2 | 40440704 | cg16259583 | MOSPD1 | 134049614 | cg26294718 | ARHGEF6 | 135864527 |
| cg06742180 | XIAP | 123039177 | cg16269097 | CASK | 41783155 | cg26296266 | SYTL5 | 37987646 |
| cg06746884 | DMD | 31285129 | cg16270385 | GLUD2 | 120181300 | cg26304243 | PLXNA3 | 153696867 |
| cg06758848 | FLNA | 153603268 | cg16272791 | GPC3 | 133119921 | cg26306815 | MED12 | 70339207 |
| cg06767008 | FOXP3 | 49114545 | cg16314146 | HTATSF1 | 135579481 | cg26308359 | VMA21 | 150565446 |
| cg06769752 | GAB3 | 153978839 | cg16318983 | MTM1 | 149737055 | cg26309951 | MORF4L2 | 102941669 |
| cg06772067 | XIAP | 122993679 | cg16328033 | PIGA | 15353419 | cg26310256 | EGFL6 | 13588301 |
| cg06774124 | TSC22D3 | 106959517 | cg16328412 | IGSF1 | 130423458 | cg26323797 | HSD17B10 | 53461386 |
| cg06774787 | KLF8 | 56258973 | cg16343842 | CD99L2 | 150066722 | cg26328611 | EDA2R | 65835992 |
| cg06776257 | DUSP9 | 152912692 | cg16349029 | SMS | 22012693 | cg26333397 | SYP | 49048031 |
| cg06778844 | PAK3 | 110335660 | cg16350494 | FOXP3 | 49121910 | cg26335568 | APEX2 | 55026135 |
| cg06780601 | NUDT10 | 51079689 | cg16353361 | APLN | 128790057 | cg26339788 | FAM3A | 153745603 |
| cg06780606 | EDA | 69257154 | cg16355818 | PAK3 | 110365847 | cg26342575 | WDR45 | 48937688 |
| cg06783548 | SYN1 | 47479436 | cg16356987 | ZDHHC9 | 128977928 | cg26345260 | UTP14A | 129060087 |
| cg06786167 | DLG3 | 69673642 | cg16359563 | ZC4H2 | 64196429 | rq26352143 | POLA1 | 25012890 |
| cg06804788 | ZNF81 | 47696363 | cg16360370 | NLGN3 | 70363992 | cg26359238 | MORC4 | 106243175 |
| cg06805513 | SLC16A2 | 73641955 | cg16367232 | UTP14A | 129040481 | cg26359958 | MBNL3 | 131623375 |
| cg06807018 | LAMP2 | 119602917 | cg16371598 | NAP1L2 | 72433997 | cg26367351 | DLG3 | 69665494 |
| cg06814121 | MID1 | 10646761 | cg16385415 | TRMT2B | 100307293 | cg26367537 | SCML1 | 17755775 |
| cg06815914 | SLC25A14 | 129472572 | cg16396396 | MTMR1 | 149928147 | cg26368752 | GK | 30671338 |
| cg06818142 | TIMM8A | 100603949 | cg16397002 | BTK | 100641870 | cg26375421 | MORC4 | 106243592 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg06822229 | ACSL4 | 108976856 | cg16399859 | SH3KBP1 | 19689070 | cg26376518 | NXT2 | 108780127 |
| cg06834235 | FTSJ1 | 48334621 | cg16403901 | PPP1R3F | 49126486 | cg26381742 | TFE3 | 48901172 |
| cg06839398 | BCOR | 39949008 | cg16412513 | GNL3L | 54556692 | cg26382607 | WWC3 | 10096192 |
| cg06845960 | SLITRK2 | 144901492 | cg16426291 | HDAC8 | 71793639 | cg26382696 | FAM104B | 55187708 |
| cg06848990 | AMOT | 112082290 | cg16428758 | TMLHE | 154769629 | cg26406249 | PFKFB1 | 55021574 |
| cg06851753 | FAM3A | 153745734 | cg16429439 | MAGT1 | 77150966 | cg26410404 | EFNB1 | 68050206 |
| cg06852103 | FTSJ1 | 48334966 | cg16431713 | RPL10 | 153626523 | cg26413007 | MID1 | 10591947 |
| cg06879828 | TMEM35 | 100333798 | cg16440909 | MAMLD1 | 149613745 | cg26419091 | LAGE3 | 153705417 |
| cg06891491 | ZNF75D | 134429749 | cg16452396 | PHF6 | 133507394 | cg26421947 | PIM2 | 48775820 |
| cg06900776 | ARMCX3 | 100878107 | cg16481170 | KIAA2022 | 74145312 | cg26428339 | FAM120C | 54209679 |
| cg06908232 | ZDHHC9 | 128977816 | cg16488754 | MTMR1 | 149861936 | cg26438739 | ARMCX6 | 100872944 |
| cg06918955 | C1GALT1C1 | 119760024 | cg16497626 | PLP1 | 103031450 | cg26445203 | MCF2 | 138773467 |
| cg06919398 | GDI1 | 153665799 | cg16501779 | TMEM185A | 148713353 | cg26457179 | MAGT1 | 77151200 |
| cg06920429 | RAP2C | 131353502 | cg16502355 | SMPX | 21771431 | cg26457272 | TBC1D25 | 48398004 |
| cg06922569 | ARAF | 47420444 | cg16510010 | FAM122C | 133941163 | cg26461352 | TIMM8A | 100603561 |
| cg06936709 | HDX | 83757476 | cg16510200 | HCCS | 11129336 | cg26482893 | KCNE1L | 108868465 |
| cg06942445 | TRO | 54947373 | cg16510657 | TMEM35 | 100333950 | cg26483766 | FAM122C | 133941187 |
| cg06943437 | MSN | 64887365 | cg16520030 | MBNL3 | 131549050 | cg26488590 | PLXNA3 | 153696502 |
| cg06944050 | LONRF3 | 118109068 | cg16533561 | ZNF81 | 47699239 | cg26489179 | BCOR | 39921143 |
| cg06944922 | RPGR | 38186842 | cg16536563 | SLC38A5 | 48328775 | cg26498015 | AR | 66788089 |
| cg06948553 | BCOR | 40037418 | cg16546861 | MAGIX | 49022452 | cg26499561 | POLA1 | 24952147 |
| cg06957108 | ZNF185 | 152082798 | cg16551468 | ARMCX2 | 100915108 | cg26505478 | CUL4B | 119695213 |
| cg06959635 | MCF2 | 138724627 | cg16561743 | CXorf23 | 19984992 | cg26506212 | ARMCX4 | 100740275 |
| cg06963664 | PLXNA3 | 153695784 | cg16564271 | MCF2 | 138769193 | cg26510526 | UPF3B | 118987238 |
| cg06980881 | ARMCX4 | 100673431 | cg16576300 | ZC4H2 | 64136881 | cg26511293 | RPS6KA6 | 83443097 |
| cg06988760 | AKAP4 | 49965182 | cg16584431 | KLHL13 | 117250153 | cg26511551 | PAK3 | 110339365 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07001318 | CXorf36 | 45060034 | cg16586165 | DOCK11 | 117630362 | cg26519783 | STAG2 | 123093616 |
| cg07011790 | TCEAL1 | 102883836 | cg16590821 | LONRF3 | 118107876 | cg26540943 | TMLHE | 154842296 |
| cg07012573 | BEX4 | 102470153 | cg16594779 | PGRMC1 | 118370873 | cg26545968 | P2RY4 | 69480213 |
| cg07038409 | ELK1 | 47509941 | cg16594878 | PRICKLE3 | 49040277 | cg26550835 | TSC22D3 | 107017362 |
| cg07042747 | MST4 | 131158005 | cg16595877 | XPNPEP2 | 128903490 | cg26552464 | HUWE1 | 53713209 |
| cg07049424 | RBMX | 135959015 | cg16613750 | MSN | 64887634 | cg26561721 | DUSP9 | 152916602 |
| cg07051162 | GK | 30671305 | cg16613854 | ENOX2 | 129758482 | cg26578373 | PIN4 | 71401206 |
| cg07051329 | CHRDL1 | 110039369 | cg16617753 | GK | 30671090 | cg26581933 | LAGE3 | 153707734 |
| cg07053481 | TMEM185A | 148714613 | cg16620229 | RAB33A | 129307331 | cg26584771 | KIAA2022 | 74145551 |
| cg07055143 | MAGEH1 | 55477154 | cg16635881 | CLDN2 | 106160456 | cg26598749 | RRAGB | 55774510 |
| cg07055845 | MID1 | 10588667 | cg16637019 | CHRDL1 | 110039349 | cg26601187 | DCX | 110653460 |
| cg07066594 | ZC4H2 | 64195554 | cg16639627 | HDX | 83757414 | cg26602702 | MECP2 | 153316012 |
| cg07069575 | CLCN5 | 49688313 | cg16641060 | NKRF | 118739737 | cg26605073 | TMEM35 | 100333473 |
| cg07069660 | AKAP4 | 49957476 | cg16641190 | TMEM164 | 109246250 | cg26606552 | CCDC22 | 49092437 |
| cg07076318 | SYTL5 | 37879976 | cg16641638 | GPR64 | 19141251 | cg26617333 | GLRA2 | 14743108 |
| cg07089438 | FAM122C | 133988190 | cg16653057 | KIAA2022 | 74144727 | cg26617508 | EDA | 68835489 |
| cg07096038 | BCOR | 39958181 | cg16678835 | NGFRAP1 | 102632131 | cg26621897 | TMSB15A | 101772051 |
| cg07098277 | RBM41 | 106361852 | cg16679837 | AR | 66764000 | cg26644504 | KCNE1L | 108869644 |
| cg07099245 | BCOR | 39954231 | cg16680433 | ZNF630 | 47928236 | cg26655138 | RBMX2 | 129536087 |
| cg07109010 | WDR45 | 48958025 | cg16680922 | ACOT9 | 23761492 | cg26670011 | IL13RA1 | 117861092 |
| cg07136610 | HUWE1 | 53713793 | cg16692308 | MID1 | 10588407 | cg26672162 | CFP | 47490133 |
| cg07150680 | HUWE1 | 53710374 | cg16697940 | LDOC1 | 140271321 | cg26674297 | RAB39B | 154494416 |
| cg07153944 | HDAC6 | 48660373 | cg16700576 | STARD8 | 67913309 | cg26674361 | TBC1D25 | 48420069 |
| cg07156563 | CITED1 | 71525984 | cg16708895 | SLITRK2 | 144898249 | cg26674826 | MMGT1 | 135056110 |
| cg07158153 | BCORL1 | 129118568 | cg16710588 | PLP2 | 49027990 | cg26680189 | HDAC6 | 48659965 |
| cg07161993 | WDR45 | 48957691 | cg16716035 | OCRL | 128674366 | cg26682103 | LONRF3 | 118109413 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07172550 | SASH3 | 128913842 | cg16716983 | DRP2 | 100474247 | cg26684826 | RAB40A | 102754778 |
| cg07174097 | TSC22D3 | 107018607 | cg16718054 | MTMR8 | 63615366 | cg26695278 | BCOR | 40010468 |
| cg07177300 | FRMPD4 | 12156499 | cg16718139 | MORC4 | 106244935 | cg26699341 | RAB9B | 103088089 |
| cg07181891 | UPRT | 74493908 | cg16725234 | KLHL13 | 117063623 | cg26703008 | CUL4B | 119709530 |
| cg07187723 | PAK3 | 110463862 | cg16726201 | CACNA1F | 49087385 | cg26704637 | PRPS2 | 12810444 |
| cg07192043 | MTMR1 | 149933113 | cg16730652 | HEPH | 65384051 | cg26735105 | TAZ | 153649375 |
| cg07195469 | DNASE1L1 | 153637739 | cg16734817 | CLCN5 | 49687071 | cg26735834 | SLC38A5 | 48329975 |
| cg07198345 | HEPH | 65382828 | cg16735667 | AR | 66788193 | cg26744454 | PGK1 | 77359613 |
| cg07204290 | RLIM | 73833865 | cg16737869 | MPP1 | 154007585 | cg26745032 | REPS2 | 16964743 |
| cg07206010 | TREX2 | 152710312 | cg16742675 | FLNA | 153602721 | cg26746069 | WDR44 | 117479618 |
| cg07208231 | ENOX2 | 130037545 | cg16756025 | ZNF275 | 152599402 | cg26747299 | CCDC120 | 48926828 |
| cg07210774 | C1GALT1C1 | 119764206 | cg16778620 | XIAP | 122994037 | cg26748472 | BCOR | 40013727 |
| cg07210835 | PLP2 | 49029104 | cg16781964 | LHFPL1 | 111924209 | cg26751631 | RLIM | 73834295 |
| cg07214066 | ZNF630 | 47930589 | cg16789335 | PPP1R3F | 49127366 | cg26756979 | LONRF3 | 118109365 |
| cg07223177 | CHST7 | 46434198 | cg16796150 | RPS6KA3 | 20285575 | cg26764180 | FAM122C | 133941194 |
| cg07240096 | GPKOW | 48980092 | cg16798761 | OCRL | 128674000 | cg26777746 | CXorf57 | 105855218 |
| cg07242375 | PRICKLE3 | 49042556 | cg16801826 | GNL3L | 54587084 | cg26785999 | TCEAL1 | 102885043 |
| cg07253552 | MBNL3 | 131624141 | cg16802801 | BCOR | 40036466 | cg26786476 | USP11 | 47093323 |
| cg07256221 | SLC25A43 | 118533106 | cg16808568 | SCML2 | 18368120 | cg26791349 | GDI1 | 153668416 |
| cg07256643 | DUSP9 | 152907168 | cg16822572 | PIM2 | 48776486 | cg26800155 | PJA1 | 68384259 |
| cg07261646 | FLNA | 153602918 | cg16830479 | ZC3H12B | 64707774 | cg26801383 | LAMP2 | 119603333 |
| cg07295246 | SH3KBP1 | 19904780 | cg16832275 | RRAGB | 55744084 | cg26802102 | IL13RA1 | 117861398 |
| cg07297906 | GPC3 | 133119578 | cg16840743 | THOC2 | 122866753 | cg26805585 | RBM10 | 47035685 |
| cg07309821 | SPIN4 | 62571385 | cg16847374 | BGN | 152772343 | cg26807389 | SLC35A2 | 48760712 |
| cg07311845 | TMLHE | 154842736 | cg16857852 | SLC6A14 | 115567695 | cg26807533 | UTP14A | 129039711 |
| cg07312966 | GPC4 | 132549205 | cg16864365 | WDR45 | 48959149 | cg26811602 | SLC9A7 | 46618762 |

EP 4 495 263 A1

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07314984 | PHKA2 | 19003243 | cg16868497 | PRPS1 | 106871338 | cg26817974 | RAP2C | 131352376 |
| cg07318999 | ARMCX4 | 100742903 | cg16869809 | ARL13A | 100236140 | cg26822601 | GNL3L | 54556833 |
| cg07320017 | MCF2 | 138774347 | cg16888859 | CLCN4 | 10123962 | cg26848126 | CYSLTR1 | 77582913 |
| cg07342016 | RAB33A | 129306129 | cg16899229 | UBE2A | 118715099 | cg26872564 | ZNF185 | 152086636 |
| cg07343739 | SLC9A7 | 46617524 | cg16917996 | NRK | 105066516 | cg26883351 | PRRG1 | 37208437 |
| cg07349006 | ARMCX1 | 100808047 | cg16925682 | PRICKLE3 | 49032012 | cg26889826 | PCYT1B | 24691052 |
| cg07358738 | SLC6A14 | 115569065 | cg16930349 | GRIPAP1 | 48858660 | cg26893124 | ELF4 | 129245188 |
| cg07363416 | MAGT1 | 77151100 | cg16931980 | MAGEB16 | 35816452 | cg26901920 | SUV39H1 | 48555977 |
| cg07368951 | SPIN4 | 62570953 | cg16934986 | MID1IP1 | 38660266 | cg26909705 | GSPT2 | 51485489 |
| cg07381502 | ATP6AP2 | 40440191 | cg16940942 | FAM50A | 153672336 | cg26918919 | ARHGAP6 | 11285195 |
| cg07382899 | APLN | 128789004 | cg16946767 | LAMP2 | 119604500 | cg26927606 | APEX2 | 55026807 |
| cg07390373 | MAOB | 43741933 | cg16946997 | NUDT10 | 51076330 | cg26932018 | FAAH2 | 57312549 |
| cg07393670 | AIFM1 | 129299802 | cg16948369 | KLHL4 | 86772436 | cg26933504 | RAB39B | 154490503 |
| cg07395435 | MCF2 | 138774080 | cg16949923 | TIMM8A | 100603782 | cg26936230 | SYN1 | 47479593 |
| cg07409400 | SLC25A43 | 118532412 | cg16950696 | MORF4L2 | 102942205 | cg26937423 | TCEAL2 | 101380437 |
| cg07416427 | WWC3 | 10085204 | cg16953966 | TSC22D3 | 106962264 | cg26939525 | PIGA | 15353660 |
| cg07419178 | CCDC120 | 48916555 | cg16976875 | MID1IP1 | 38663177 | cg26948870 | MID1 | 10646183 |
| cg07419680 | ZNF75D | 134429972 | cg16976876 | CLCN4 | 10124911 | cg26950400 | PRICKLE3 | 49043021 |
| cg07419801 | MST4 | 131157622 | cg16984885 | RPL39 | 118925542 | cg26953234 | GPC4 | 132547142 |
| cg07427073 | ZNF182 | 47837436 | cg16986238 | MSN | 64960335 | cg26954928 | TSC22D3 | 106978259 |
| cg07428182 | PLXNA3 | 153686926 | cg16991511 | MST4 | 131156989 | cg26955512 | HDAC8 | 71792545 |
| cg07434817 | SH3KBP1 | 19906542 | cg17001703 | LAS1L | 64754863 | cg26961103 | CDR1 | 139865953 |
| cg07442775 | KLHL13 | 117120182 | cg17003375 | NKRF | 118740514 | cg26967211 | PRRG1 | 37208351 |
| cg07446674 | MAP7D3 | 135333561 | cg17006131 | ZDHHC15 | 74744787 | cg26985119 | MAGIX | 49022801 |
| cg07457143 | RBMX2 | 129547142 | cg17008556 | TFE3 | 48901167 | cg27013947 | SMPX | 21776178 |
| cg07459438 | WDR45 | 48934313 | cg17015217 | FAM104B | 55187812 | cg27024381 | ABCD1 | 153009999 |

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07464229 | USP11 | 47091239 | cg17033281 | XIAP | 122993976 | cg27038067 | PHF8 | 54067038 |
| cg07464524 | TSC22D3 | 106960206 | cg17035182 | KLHL13 | 117250736 | cg27046940 | ELF4 | 129239794 |
| cg07464598 | ARAF | 47420411 | cg17037297 | CYBB | 37649522 | cg27047283 | MAP7D3 | 135333567 |
| cg07468307 | KLHL13 | 117106802 | cg17039808 | PDZD4 | 153076144 | cg27048639 | F8 | 154252341 |
| cg07469105 | TMSB15A | 101772034 | cg17051440 | CLDN2 | 106163689 | cg27054331 | DUSP9 | 152910369 |
| cg07470526 | RRAGB | 55784972 | cg17056186 | C1GALT1C1 | 119763575 | cg27058480 | PDHA1 | 19361727 |
| cg07471703 | ALAS2 | 55057010 | cg17058724 | FAM127A | 134166347 | cg27059259 | ARAF | 47420742 |
| cg07473550 | TSC22D3 | 107019191 | cg17066973 | VBP1 | 154444148 | cg27062326 | DOCK11 | 117629163 |
| cg07480829 | CUL4B | 119694550 | cg17068766 | RAI2 | 17879816 | cg27065374 | EFNB1 | 68060181 |
| cg07492878 | BEX4 | 102470002 | cg17074762 | EFNB1 | 68052208 | cg27066211 | MSN | 64887745 |
| cg07507339 | FAM3A | 153744384 | cg17077516 | REPS2 | 16964700 | cg27070532 | GLRA2 | 14748882 |
| cg07508090 | ZC4H2 | 64196343 | cg17082365 | ARHGAP6 | 11157323 | cg27074040 | RBMX | 135963877 |
| cg07519908 | CDKL5 | 18443723 | cg17119559 | GPR64 | 19100990 | cg27074837 | CDR1 | 139866026 |
| cg07520976 | GPRASP2 | 101971960 | cg17122979 | TBC1D25 | 48397915 | cg27076487 | LDOC1 | 140271364 |
| cg07525100 | FGD1 | 54522721 | cg17123338 | MECP2 | 153361684 | cg27078591 | MTM1 | 149737335 |
| cg07559615 | ELK1 | 47497286 | cg17134286 | ARHGEF6 | 135849989 | cg27086638 | WDR13 | 48456805 |
| cg07573912 | MECP2 | 153363369 | cg17141186 | SYTL5 | 37925026 | cg27088126 | F8 | 154110093 |
| cg07581973 | PRDX4 | 23685729 | cg17149359 | C1GALT1C1 | 119764131 | cg27112414 | UBL4A | 153715339 |
| cg07590102 | CDKL5 | 18460454 | cg17152375 | HDAC6 | 48660379 | cg27123903 | FAM50A | 153672721 |
| cg07592828 | NLGN3 | 70364353 | cg17155577 | TMEM164 | 109245482 | cg27128905 | ZBTB33 | 119391968 |
| cg07601068 | BCOR | 39953107 | cg17157129 | RBM41 | 106362429 | cg27138147 | RBMX | 135962969 |
| cg07605754 | CLCN5 | 49687822 | cg17192356 | PIM2 | 48776465 | cg27153798 | ARAF | 47420292 |
| cg07608505 | TCEAL2 | 101380476 | cg17193417 | RLIM | 73834683 | cg27166673 | ARHGAP6 | 11683995 |
| cg07646007 | FAM104B | 55169964 | cg17195879 | RPL10 | 153626255 | cg27192418 | DMD | 32886526 |
| cg07647518 | SH3KBP1 | 19904979 | cg17196716 | CXorf36 | 45059906 | cg27198071 | FAM127A | 134166610 |
| cg07649160 | PRAF2 | 48931826 | cg17197278 | C1GALT1C1 | 119764200 | cg27207144 | RPL10 | 153626500 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07650623 | GLRA2 | 14547147 | cg17203703 | YIPF6 | 67718716 | cg27251926 | PIGA | 15353768 |
| cg07650640 | BCOR | 40007201 | cg17204993 | ARHGEF6 | 135863102 | cg27253485 | STARD8 | 67913388 |
| cg07653640 | CHRDL1 | 110038954 | cg17207724 | ELF4 | 129244725 | cg27254181 | ARMCX1 | 100809355 |
| cg07654896 | RGN | 46937829 | cg17217369 | TFE3 | 48901982 | cg27256435 | LRCH2 | 114468596 |
| cg07673466 | NLGN3 | 70363469 | cg17220866 | DYNLT3 | 37706296 | cg27263894 | FUNDC2 | 154254814 |
| cg07674075 | FAM104B | 55187543 | cg17223593 | GPC4 | 132549297 | cg27264374 | ARAF | 47420425 |
| cg07674139 | NRK | 105066526 | cg17227030 | EDA | 68837158 | cg27271368 | AR | 66764411 |
| cg07674503 | RAP2C | 131352282 | cg17236781 | ZDHHC9 | 128977919 | cg27273125 | LHFPL1 | 111919438 |
| cg07674520 | HDAC6 | 48659624 | cg17246352 | MCTS1 | 119737962 | cg27278294 | MTM1 | 149736594 |
| cg07679571 | DLG3 | 69712223 | cg17274024 | DKC1 | 153991813 | cg27311392 | TSC22D3 | 106959913 |
| cg07682072 | PORCN | 48367166 | cg17281073 | DIAPH2 | 95942461 | cg27326620 | ZC4H2 | 64196642 |
| cg07686944 | SCML1 | 17755642 | cg17281355 | FAM3A | 153738942 | cg27328073 | CXorf40B | 149103225 |
| cg07688057 | BCOR | 39943025 | cg17281810 | BCOR | 40007949 | cg27329995 | MAGEB16 | 35816376 |
| cg07688234 | CFP | 47489663 | cg17291758 | GRIPAP1 | 48858921 | cg27348515 | ARMCX2 | 100914892 |
| cg07693301 | CD99L2 | 150067249 | cg17294479 | BEX4 | 102470404 | cg27353825 | SMARCA1 | 128656920 |
| cg07693661 | NAP1L2 | 72435007 | cg17299883 | LAS1L | 64754624 | cg27355009 | PRICKLE3 | 49035652 |
| cg07694085 | MAP7 D3 | 135334005 | cg17326835 | PLXNA3 | 153688618 | cg27360006 | ZNF41 | 47311883 |
| cg07745899 | TRMT2B | 100303724 | cg17337021 | IDS | 148586983 | cg27381383 | ARMCX3 | 100879169 |
| cg07746818 | BEX4 | 102469720 | cg17345036 | TRMT2B | 100306958 | cg27382931 | GPC4 | 132549690 |
| cg07748875 | BEX2 | 102565963 | cg17345373 | LAS1L | 64754885 | cg27384106 | FHL1 | 135250267 |
| cg07749147 | RPL10 | 153625894 | cg17346042 | FRMPD4 | 12597644 | cg27389529 | PHF6 | 133507040 |
| cg07750402 | OTUD5 | 48814932 | cg17354708 | HDAC8 | 71793175 | cg27389648 | XPNPEP2 | 128873170 |
| cg07752550 | MAGEB16 | 35816656 | cg17376045 | GNL3L | 54556668 | cg27395754 | TSPYL2 | 53117341 |
| cg07761273 | EGFL6 | 13591311 | cg17380767 | AMOT | 112084913 | cg27398247 | FAAH2 | 57361729 |
| cg07764473 | BCOR | 40037510 | cg17383222 | KCND1 | 48823848 | cg27419400 | ABCB7 | 74376313 |
| cg07764932 | ARHGAP6 | 11278904 | cg17385437 | FGD1 | 54523049 | cg27429230 | SLC35A2 | 48769380 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07767690 | MAGED2 | 54834292 | cg17391028 | FGD1 | 54523088 | cg27435491 | FAM3A | 153744582 |
| cg07771637 | FAM127B | 134186198 | cg17395800 | CACNA1F | 49087762 | cg27441063 | FRMPD4 | 12719942 |
| cg07772467 | GK | 30671633 | cg17396400 | ACOT9 | 23761459 | cg27450030 | BCOR | 40008861 |
| cg07780118 | AR | 66766506 | cg17398312 | FRMPD4 | 12157040 | cg27450971 | TMEM35 | 100350134 |
| cg07781082 | PGK1 | 77359553 | cg17398485 | CXorf56 | 118699448 | cg27452062 | CHRDL1 | 110039414 |
| cg07792041 | HAUS7 | 152735637 | cg17399684 | ELK1 | 47510170 | cg27465531 | MBTPS2 | 21857712 |
| cg07796014 | HCCS | 11129290 | cg17403974 | AGTR2 | 115304403 | cg27465534 | ASB9 | 15288295 |
| cg07799491 | MTMR1 | 149861526 | cg17404000 | SLC9A7 | 46618630 | cg27470278 | STAG2 | 123094132 |
| cg07804196 | DMD | 33229454 | cg17420696 | SMARCA1 | 128657092 | cg27474516 | APLN | 128789105 |
| cg07806797 | ZNF185 | 152127816 | cg17439480 | SLC25A43 | 118533042 | cg27480179 | FANCB | 14889247 |
| cg07810164 | EGFL6 | 13588174 | cg17464366 | ZC4H2 | 64196495 | cg27483305 | NKAP | 119077757 |
| cg07812390 | EFNB1 | 68061003 | cg17472432 | STARD8 | 67867429 | cg27488875 | BGN | 152760493 |
| cg07816287 | GPC3 | 133119692 | cg17473615 | MAGED2 | 54835651 | cg27496708 | GPC3 | 133119594 |
| cg07824317 | RPGR | 38187006 | cg17477997 | PIGA | 15353781 | cg27501007 | IL13RA1 | 117861380 |
| cg07825508 | RPL36A | 100645891 | cg17479100 | SLC35A2 | 48768940 | cg27502296 | DUSP9 | 152913300 |
| cg07826058 | MBTPS2 | 21857614 | cg17481638 | WWC3 | 10090596 | cg27513289 | RP2 | 46696888 |
| cg07828380 | ARHGAP6 | 11683977 | cg17482649 | TSPYL2 | 53117672 | cg27519679 | AIFM1 | 129299533 |
| cg07830469 | PJA1 | 68385467 | cg17488844 | TCEAL1 | 102883840 | cg27525527 | MAGED1 | 51636671 |
| cg07834773 | DGKK | 50214061 | cg17499479 | CD99L2 | 150067328 | cg27538352 | PRICKLE3 | 49032410 |
| cg07837161 | FAM3A | 153744667 | cg17503853 | CCDC22 | 49091797 | cg27540791 | DRP2 | 100475082 |
| cg07858069 | SMS | 21958690 | cg17505944 | FAM50A | 153671997 | cg27543728 | MCF2 | 138777468 |
| cg07861014 | UXT | 47515586 | cg17508549 | LAGE3 | 153707810 | cg27545041 | DDX26B | 134654391 |
| cg07864948 | RS1 | 18690197 | cg17509494 | ZDHHC15 | 74743480 | cg27548075 | MAGED1 | 51638041 |
| cg07867687 | FTSJ1 | 48334539 | cg17511128 | PCYT1B | 24691232 | cg27550498 | MED12 | 70340920 |
| cg07874284 | PRPS2 | 12809156 | cg17545301 | KCND1 | 48822639 | cg27557909 | WDR44 | 117480893 |
| cg07874334 | XIAP | 122993913 | cg17546926 | TBC1D8B | 106117602 | cg27559462 | UBE2A | 118709227 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg07876586 | PGRMC1 | 118370356 | cg17547226 | PRICKLE3 | 49032528 | cg27561732 | KLHL15 | 24045379 |
| cg07880562 | NYX | 41333643 | cg17547524 | DOCK11 | 117629324 | cg27581839 | AMOT | 112083581 |
| cg07887243 | MAOB | 43741530 | cg17548613 | CASK | 41782322 | cg27584469 | SLITRK2 | 144899412 |
| cg07897414 | KLHL4 | 86772895 | cg17548969 | PIGA | 15353982 | cg27586390 | CXorf57 | 105855176 |
| cg07902213 | THOC2 | 122866969 | cg17552650 | WDR45 | 48957963 | cg27587954 | HAUS7 | 152726276 |
| cg07903515 | ZDHHC9 | 128975063 | cg17558827 | FAM127A | 134166133 | cg27592925 | RAP2C | 131351970 |
| cg07906564 | GRIPAP1 | 48858799 | cg17571782 | CXorf40A | 148623024 | cg27595778 | MBNL3 | 131624071 |
| cg07908142 | RPL39 | 118926253 | cg17581805 | CXorf67 | 51149400 | cg27606669 | NRK | 105065879 |
| cg07910525 | NUDT10 | 51075582 | cg17582679 | CAPN6 | 110513791 | cg27609342 | KIAA2022 | 74145250 |
| cg07910919 | ZNF185 | 152082866 | cg17590101 | SLC38A5 | 48327749 | cg27615468 | FAM127B | 134186522 |
| cg07911663 | TMEM164 | 109245325 | cg17601118 | ARMCX2 | 100916001 | cg27633753 | OCRL | 128674222 |
| cg07912337 | FAM3A | 153744670 | cg17604239 | TRMT2B | 100307203 | cg27647594 | TCEAL2 | 101379978 |
| cg07917796 | DIAPH2 | 95939710 | cg17607496 | MPP1 | 154033792 | cg27665489 | NUDT10 | 51075530 |
| cg07930217 | PRPS1 | 106871770 | cg17624691 | LAS1L | 64754747 | ch.X.1002740R | HEPH | 65412451 |
| cg07933757 | CD99L2 | 150066987 | cg17626563 | RS1 | 18691141 | ch.X.1023208F | OPHN1 | 67291364 |
| cg07933790 | NYX | 41333435 | cg17627526 | PCYT1B | 24665508 | ch.X.1031144F | YIPF6 | 67741097 |
| cg07940380 | OPHN1 | 67653182 | cg17634825 | PIN4 | 71482842 | ch.X.1171704R | ATRX | 76894657 |
| cg07946630 | GNL3L | 54556446 | cg17639056 | SLC9A7 | 46618639 | ch.X.147603R | WWC3 | 10104133 |
| cg07954607 | TCEAL2 | 101380464 | cg17639969 | TRMT2B | 100264977 | ch.X.1698482R | DOCK11 | 117678274 |
| cg07979856 | PRICKLE3 | 49044231 | cg17656292 | GSPT2 | 51487720 | ch.X.1776338R | THOC2 | 122761822 |
| cg07980716 | AIFM1 | 129272060 | cg17658468 | DYNLT3 | 37706817 | ch.X.188238R | PRPS2 | 12813542 |
| cg07994386 | FRMPD4 | 12156284 | cg17662177 | CFP | 47490087 | ch.X.409130R | PCYT1B | 24597023 |
| cg08021299 | HEPH | 65383076 | cg17662252 | GNL3L | 54560704 | ch.X.798879F | FTSJ1 | 48341687 |
| cg08057040 | MAGT1 | 77084159 | cg17670641 | MAGIX | 49023115 | ch.X.881064F | HUWE1 | 53673061 |
| cg08059778 | BCORL1 | 129116536 | cg17676246 | ARHGEF6 | 135863726 | ch.X.881546R | HUWE1 | 53691005 |

(continued)

| Probe ID | Gene | Position in ChrX (hg19) | Probe ID | Gene | Position in ChrX (hg19) | Probe ID | Gene | Position in ChrX (hg19) |
|---|---|---|---|---|---|---|---|---|
| cg08063601 | AR | 66763942 | cg17694877 | DIAPH2 | 95939631 | ch.X.938089F | FAAH2 | 57358216 |

Probe ID: unique identifier from the Illumina CG database. Chromosomal position (hg19): chromosomal coordinates of the CpG in the X chromosome (build hg19). Associated gene: target gene name from the UCSC database.

For each study in the Examples, the CpGs in the reference panel were selected and the beta methylation levels for each individual were extracted. For each woman in the study, X-Ra was defined as the fraction of selected CpGs that were hypomethylated. Hypomethylation status was defined for those CpGs whose beta values were below 0.2.

Statistical analyses

Data were analyzed using R version 4.1.1 and Bioconductor version 3.13. Functions and libraries are written in italics. The goal was to determine the biological correlates of chromosome X reactivation (X-Ra) in studies with different characteristics and to establish the biological plausibility of the biomarker as a proxy of XCI, and as a factor in biological states believed to be associated with XCI, such as age, infection, fertility, cancer risk, and survival. It was also tested the association between X-Ra and Levine's methylation age (M. E. Levine, et al. An epigenetic biomarker of aging for lifespan and healthspan. Aging (Albany. NY). 10 (2018), doi:10.18632/aging.101414), as computed by the R package methylclock.

In the monocytes study (GSE56046), the association between X-Ra and differential expression in purified monocytes from peripheral blood and with the age of adult women was tested. In this study, the associations of X-Ra with transcriptomic signatures specific to XCI were also tested. Transcriptome-wide association analysis was performed for the effect of X-Ra using massive univariate regressions of gene expression levels on X-Ra, adjusting for cohort, sex, and surrogate variables inferred with SVA. These models were fit with *lmFit* from *limma*, and inferences were performed with eBayes. Associations were considered significant after FOR correction for multiple comparisons. The enrichment in associations ($P < 1e-3$) of inactivated genes was tested using a hypergeometric (Fisher exact) test. Association tests were then performed between X-Ra and the relative expression of inactive genes with respect to escapees. For X-Ra, two associations were performed. First, the differences of logFC, derived from DE expression analyses, with X-Ra between inactive and escapee genes were tested using a t-test for the difference in means. Second, the association between X-Ra and the measure Rxci-Rxescape was tested. The transcription residuals for all the selected genes were computed, adjusting for age, and immune cell contamination. Rxci was computed as the mean transcription residual across the inactive genes, and Rxescape was computed as the mean transcription residual across the escapees. The difference between both means is a measure of the global transcription inactivation relative to those genes not under XCI. For this study, the association between X-Ra and age (>65) was also tested using a linear regression model, adjusting for cohort and immune cell contamination. Dichotomization of age was taken as the median age of cancer at any site according to SEER 22 2015-2019.

Associations of X-Ra in blood with age were performed for the GENOA study and TruDiagnostic DNA biobank. In GENOA (GSE210255), a linear model was fitted adjusting for plate, and in TruDiagnostic, we adjusted for immune cell contamination. The regression coefficients for age across the three studies (Monocytes, GENOA, TruDiagnostic) were meta-analyzed using fixed and random effects models with two-sided tests using *metagen* from *meta*.

In the TruDiagnostic data, the association with telomere length, height, weight, number of children and cancer diagnosis at any site was also tested. We fitted linear regression models, adjusting for immune cell abundance. For cancer diagnosis, a logistic regression model was fitted on X-Ra and the interaction between X-Ra and height. It was tested the interaction taking height as continuous and also dichotomizing at the first quartile (1.60 cm).

In the longitudinal studies GSE142536 and GSE62003 that included follow up of individuals with aging conditions (cancer and Alzheimer's disease) and controls, models were fitted on X-Ra with condition, visit and age as fixed effects and subject as a random effect. The lmer R library was used. Similar mixed models were tested for the Levine's methylation age, to contrast the effect of the condition between X-Ra and global methylation age. In the Alzheimer's disease study, it was tested the significance of the intraclass correlation. It was found that it was initially moderate (ICC<0.5) due to the presence of an individual with outlying trajectory. After removing the individual, who was the eldest (85 years old), the ICC was recovered (ICC=0.9). In the longitudinal studies GSE62003 and GSE37722 that tested the transient change of X-Ra under COVID 19 infection and pregnancy; respectively, we fitted mixed model effects on X-Ra on the fixed effect of visit and age and the random effect of subject. We tested for the significant effect of visit. Results were contrasted with those obtained for Levine's methylation age.

In the TCGA, it was tested the association of X-Ra in tumor and healthy tissues with breast cancer risk and survival. For breast cancer risk, linear regression models were fitted of X-Ra on cancer status of the samples and adjusted for age. It also tested the association between X-Ra and ER, HER, and PER and triple-negative status, adjusting for age. Survival associations were performed with proportional hazards regression models adjusting for age. It was also tested genome-wide associations of X-Ra with the frequency of copy number gains and deletions of breast cancer tissues in sliding windows of 0.5 Mb. To deal with windows with low frequency in copy number changes (~1% at most), it was fitted Bayesian regression models with *bayesglm* of *arm*, adjusting for age. Associations were adjusted for multiple comparisons using Bonferroni correction.

For each of the 12 cancer sites, it was inferred the value of X-Ra and tested the association with cancer status and survival using a meta-analysis with fixed and random effects models. The prediction power of X-Ra on cancer status was tested with a ROC area under the curve statistic. The optimal X-Ra was defined as the Youden distance, which was used to compute the attributable fraction of X-Ra of affected tissue on cancer risk, using the *AFglm* function of the AF library. For transcriptome-wide associations of X-Ra in each cancer site, it used *limma* and adjusted for SVA and age. Log2FC with their standard error were then meta-analyzed across cancer sites, and the associations were adjusted for multiple comparisons for the number of transcripts tested, with Bonferroni correction. On these associations, enrichment analyses on biological processes were performed on genes with nominal significance lower than 0.01, using the *enrichGO* from *clusterProfiler.*

**3. Results**

Fraction of CpG hypomethylation in inactivated genes in monocytes

First, the distribution differences in CpG methylation beta-levels of consistently inactivated genes (Table 2) and consistent escapees was assessed in purified monocytes from a population sample of 605 (60 $\pm$ 9.47years) women (Fig. 1A). It was confirmed that CpG levels of inactivated genes had two primary peaks: one corresponding to a midrange methylation signal, consistent with hypermethylation on Xi and hypomethylation on Xa (tissue active genes), and another corresponding to high methylation, consistent with hypermethylation of both copies on Xi and Xa (tissue inactive genes).
For each individual, it was defined the fraction of CpGs in genes that should be consistently inactivated but had methylation levels lower than 0.2, as a measure of chromosome X reactivation (X-Ra) and shown in Fig. 1B. It was hypothesized that the weaker the overall XCI was in a woman, the higher her X-Ra would be, due to the higher frequency of complete demethylation in sites of genes that should be inactivated in Xi but are not. While this measure is an aggregated signal of individual CpGs, which may be influenced by unmeasured errors, it can serve as a personal biomarker of the individual's hypomethylation signal from Xi.
To study the effect of X-Ra on gene expression, transcriptome-wide differential expression analysis was performed on monocytes of the 605-adult woman. It was found significant genes relevant to cancer and histone modifications (Fig. 1C). Specifically, it was observed significant upregulation of *ABTB2* expression, which has recently been associated with treatment response in breast cancer chemotherapy. It was also observed significant downregulation of SOGA1, which is regulated by the lncRNA TSIX and is a predictor of hepatocellular carcinoma. Additionally, it was observed upregulation of the Histone-binding protein *RBBP4* gene and *RSPO2* encoding for R-spondin 2, which synergizes with WNT to activate beta-catenin.
Evidence of the association of X-Ra with overall downregulation of XCI was obtained from three different analyses. First, it was observed a significant enrichment of inactivated genes from the transcriptome-wide differential analysis of X-Ra (OR = 3.74, P = 0.001),; *XIST* was downregulated at nominal significance (log2FC=-2.44, P=0.02). Second, it was found that the average log2FC of inactivated genes was significantly higher than that of the escapees (mean Log2FC difference: 0.23, P = 0.007). Third, for each individual, it was calculated the difference of average expression residuals between inactive genes (Rxci) and escapees (Rescape), and observed that the difference strongly associated linearly (X-Ra difference: 0.0586, P = 0.0003) and quadratically (X-Ra difference: 0.0581, P = 0.0004) with X-Ra (Fig. 1D). Therefore, high X-Ra values suggest an increased activity of genes that should be under XCI.

X-Ra in blood is associated with aging, age-associated diseases and transient states

Methylation data from three different studies, including GENOA, a large case-control hypertension study between 1996-2005, with 986 African American females; TruDiagnostic DNA biobank, which included 1,414 women recruited between October 2020 and February 2022 was analyzed (N. Carreras-Gallo, et al., "The early-life exposome modulates the effect of polymorphic inversions on DNA methylation". Commun. Biol. 2022 51 5, 1-13 (2022)) ; and the monocyte study from the previous analysis. It was inferred the value of X-Ra in all studies and tested its association with age>65, which is the median age of cancer diagnosis at any site (SEER 22 2015-2019). It was observed significant increases in X-Ra with age in all studies (meta-analyzed beta: $5.69 \times 10^{-3}$, P=0.0004, Fig. 2A).
Interestingly, in TruDiagnostic data, it was found that the association between X-Ra and continuous age was particularly strong in women with reported menopause (X-Ra difference: $5.19 \times 10^{-4}$, P= $2.56 \times 10^{-7}$, Fig. 2B-C). A similar effect was present in the GENOA study where stratification by late menopause age (>60) increased the association between X-Ra and age (X-Ra difference: $2.48 \times 10^{-4}$, P= 0.0031). Back in Trudiagnostic data, it was observed a significant association between X-Ra and whether women had reported given birth at all, adjusting by age immune cell count and slide (OR=0.49, P=0.012, Fig. 2F), confirming the notion that higher levels of X-Ra may be associated with lower fertility, in line with its increasing rates during menopause, and suggesting an active selection pressure during fertile years. In relation to aging, it was observed a significant association between X-Ra and shorter telomere length (X-Ra difference: -8.67 $\times$ $10^{-3}$, P =0.015, Fig. 2D). Adjustment by telomere length explained the association between X-Ra and age. It was then observed a significant association between X-Ra and Levine's methylation age (M. E. Levine, et al., "An epigenetic biomarker of aging for lifespan and healthspan". Aging (Albany. NY). 10 (2018)), which was however fully explained by age adjustment. These results showed that the X-Ra determined by the method of the invention is a reliable indicator of female aging that may be modulated and associated with biological processes such as menopause and telomere shortage, and less with global methylation changes.

The risk of diagnosed cancer in TruDiagnostic biobank was also tested (167 were diagnosed with any type of cancer) and it was found a significant two-fold increase in risk with every 10 units of X-Ra (OR=2.11, P=0.03 Fig. 2E), adjusting for age and immune cell distribution in blood. In particular, the association between X-Ra and cancer was strengthened in women with heights >160cm (OR=3.0, P=0.003), corresponding to the lower quartile of the heights in the study. No significant associations of cancer risk with telomere length and Levine's methylation age was found.

To gain further insight into X-Ra as a reliable marker of female disease, it was analyzed a series of longitudinal data. It was first analyzed methylation data in blood from 17 elderly women assayed immediately before major surgery, in the morning of postoperative day and between 4 and 7 days after surgery. Patients had a mean age of 79 years and three of them had cancer. Taken as a test-retest study, it was observed that the X-Ra trajectories of the individuals were highly stable and reliable (Intra-class-correlation: 0.95, P= $3.13\times10^{-21}$, Fig. 3A). In addition, it was validated that women with cancer had a consistently high value of X-Ra in blood than those without it (X-Ra difference: 0.023, P= 0.003), adjusting by age and including a random effect on subject. No significant effects of surgery (time) and age were observed. It was observed a significant correlation between X-Ra and Levine's methylation age (Correlation: 0.31, P=0.02) but this methylation age did not explain the differences of blood X-Ra in cancer and could not discriminate between cancer status (Fig. 4). As associations of X-Ra in blood with cancer could be indicative of the role of X-Ra in immune upregulation, it was tested its association with Alzheimer's disease (AD), characterized by low blood monocyte count. It was analyzed data of 11 elderly women (mean age 74.18), assayed at two time points and 5 diagnosed with AD. It was observed that women with AD had consistent low values of X-Ra (X-Ra difference: -0.009, P=0.04, Fig. 3C). Interestingly the pattern was not detected by global methylation aging (Fig. 5).

It was then assessed whether a more transient nature of X-Ra could also be observed under acute immunological challenges. Therefore, it was analyzed methylation data in buffy coat from individuals who recovered from severe COVID 19 infection and were admitted to the UCI. Methylation was assayed at admission, during and previous discharge from UCI and at follow-up after discharge. It was analyzed X-Ra progression in 10 women and observed a significant decrease over follow-ups (X-Ra difference: $-3.09\times10^{-3}$ P=0.03, Fig. 3B), adjusting by age and subject as a random effect. While methylation age was shown to strongly decrease between discharge from hospital (times 3-4), it was observed that it did not consistently decrease from the time of admission to UCI. It was then used longitudinal data of 14 pregnant women to further test the transient nature of X-Ra under changes in immune response. Women were assayed in early and mid-pregnancy, delivery and post-partum. While the intra-class correlation for consistency across the four measurements was not as high as in previous cases (ICC: 0.43, P= 0.0003), it was found a significant decrease on X-Ra (X-Ra difference: -0.003, P= 0.004, Fig. 3D).

## X-Ra as a marker of breast cancer in tumor DNA

It was then investigated the extent to which X-Ra was associated with breast cancer (BRCA). Methylation and clinical data were downloaded from The Cancer Genome Atlas (TCGA) and computed the value of X-Ra for 109 normal breast samples and 1084 tumor tissue samples (Fig. 6). For breast tissue, it was observed similar patterns for CpGs in inactivated and escapees as before, albeit with lower hypermethylation peaks. For BRCA, it was observed a significant increase in the frequency of hypomethylation in CpGs of inactivated genes compared to breast tissue. It was calculated X-Ra in all samples and observed a highly significant association with cancer tissue (X-Ra difference=0.061, P=$2.12\times10^{-8}$). X-Ra in tumors was also associated with lower survival, adjusted for age (HR= 6.16, P= 0.022) (Fig. 6B). Interestingly, X-Ra correlated significantly with ER- (X-Ra difference: 0.023, P=0.004), PR- (X-Ra difference: 0.020, P=0.007), and HER2- status, but not significantly (X-Ra difference: 0.014, P=0.15). In line with these results, it was observed that X-Ra was strongly associated with triple-negative (TN) BRCA (X-Ra difference: 0.040, P=$2.42\times10^{-4}$). In this data, it was observed an expected reduction in survival for TN status but it was not statistically significant (HR=1.59, P=0.14), likely due to low power. The association further decreased in significance when adjusting by X-Ra. However, the strength of the association between X-Ra and survival increased in one order of magnitude in the presence of TN status (HR= 14.39, P=0.001), suggesting a X-Ra mediation in the association between TN and survival.

It was then investigated whether increased X-Ra levels in cancer tissue are associated with specific chromosomal copy number variants (CNVs) across the genome. The genome was scanned using 0.05MB windows to detect CNVs in BRCA samples. It was then tested the association between X-Ra and frequent deletions and gains (>0.01) while adjusting for age. While a significant association of X-Ra with deletions was not observed, it was found 492 significant regions with overlapping gains after correcting for multiple comparisons (Fig. 6C). Remarkably, the several of top associations in each chromosome included loci associated with cancer. The most significant association was found with copy number gains at *TNFRSF68*, believed to prevent apoptosis and promote tumor growth. It was also observed important associations with gains at the 8q24.21 region involving the *MYC* locus. Interestingly, ectopic expression of c-Myc and other transcription factors in fibroblasts is known to generate induced pluripotent stem cell reprogramming with reactivated X-chromosomes in females. Other significant gains associated with X-Ra were at *NTRK1* and FRS2, known to have a potential association with breast cancer.

Finally, a random sampling using different number of genes (1, 5,10, 20, 30, 40, etc) to do the calling of X-Ra in breast cancer was carried out and the percentage deviation (average in the whole sample) of the value when all the genes are used was calculated. As shown in Figure 8, the results demonstrate that there was extremely low deviation in the X-Ra calling when the CpGs of more than 100 genes were analyzed.

Furthermore, even when only 10 genes were analyzed, a deviation of -2.8% was found, supporting that the method of the invention to determine X-Ra levels can be carried out even when analyzing the methylation status of a low number of the indicated genes.

X-Ra as a marker of female cancer

It was then examined the association of X-Ra with the tumor status of samples and with patient survival for 12 different cancers types in TCGA, using downloaded data from *curatedTCGA* version 1.16.0. It was found that X-Ra levels were strongly associated with cancer status (meta-analyzed beta: 0.056, P=$5.59\times10^{-7}$) (Fig. 7A). To assess the ability of X-Ra to detect any type of cancer in a tissue, it was performed a ROC analysis and obtained a 95%CI for the AUC of (0.64, 0.69) (Fig. 7B). The optimal Youden value for X-Ra was 0.11, which had 85.4% specificity and 47.5% sensitivity. Dichotomizing X-Ra at this value was strongly associated with the cancer status of the tissue, adjusting for cancer type and age (OR=6.28, P=$4.79\times10^{-36}$). The attributable risk of dichotomized X-Ra on cancer status was 40% (95%CI=23.9%, 56.2%). It was found that the proportion of dichotomized X-Ra was 43% across cancer types, with the highest rates in THCA (75%), LIHC (56%) and BRCA (50%), and the lowest in LUAD (14%) and PAAD (17%), in line with the associations with cancer status (Fig. 7A). Although high X-Ra levels were a common feature across a range of tumors, the effect of X-Ra on survival was borderline significant across the 12 cancer types (meta-analyzed HR: 2.32, P= 0.054).

To further understand the molecular mechanisms derived from high values of X-Ra in cancer, it was conducted transcriptome-wide gene expression analysis on each tumor. Several significant genes were found. Remarkably, it was observed that the downregulation of *XIST* with X-Ra was the highest association for BRCA, COAD, KIRC and READ, and was significant in several other cancer types. A meta-analysis across cancer types was performed and found consistent downregulation of the gene (Log2FC=-6.76, P=$1.32\times10^{-11}$) (Fig. 7C). It was also conducted a meta-analysis of 13,189 genes, which identified 537 significant genes after correction for multiple comparisons. The top genes included *LRCH1* and *PHF11*, which play important roles in CD8+T cell response against tumors and pathogens, as well as T-cell activation in autoimmune disease. Enrichment analyses of these significant genes revealed several pathways relevant to cancer, such as immune response, signal transduction and cell proliferation and apoptosis (Fig. 7D).

In summary, these results demonstrate that the novel measure of global X-Ra levels provided in the present invention is a useful marker for diverse aspects of women health, including aging and cancer risk.

**Citation List**

N. Carreras-Gallo, et al., "The early-life exposome modulates the effect of polymorphic inversions on DNA methylation". Commun. Biol. 2022 51 5, 1-13 (2022)

M. E. Levine, et al., "An epigenetic biomarker of aging for lifespan and healthspan". Aging (Albany. NY). 10 (2018)

Burtis C. A. et al., 2008, Chapter 14, section "Statistical Treatment of Reference Values"

Morris TJ et al., " Analysis pipelines and packages for Infinium HumanMethylation450 BeadChip (450k) data", Methods. 2015 Jan 15; 72: 3-8.

**Claims**

1. An *in vitro* method for determining the X chromosome reactivation level of a female subject, the method comprising the steps of:

   (a) determining the methylation status of at least 10 CpG sites of Table 1 in a DNA-containing sample from the female subject; and

(b) calculating the fraction of the CpG sites analyzed in step (a) that are hypomethylated.

2. The in vitro method according to claim 1, wherein methylation status of the CpG sites is determined by a method selected from the group consisting of methylation chip array, bisulfite sequencing, next-generation bisulfite sequencing (NGBS), methylated DNA immunoprecipitation (MeDIP), enzymatic methyl sequencing (EM-Seq), HELP assay, mass spectrometry, HPLC, ChiP-on-chip assay, High Resolution Melt Analysis (HRM or HRMA), molecular break light assay for DNA adenine methyltransferase activity, methyl sensitive Southern blotting, and Methylation Specific PCR (MSP).

3. The in vitro method according to claim 1 or 2, wherein the hypomethylated CpG sites are the CpG sites with:

   (i) a beta value equal to or lower than about 0.2, where the methylation status is determined by a methylation chip array;
   (ii) a methylation equal to or lower than about 20%, where the methylation status is determined by bisulfite sequencing; or
   (iii) a methylation equal to or lower than about 5%, where the methylation status is determined by next-generation bisulfite sequencing.

4. The in vitro method according to any one of claims 1-3, wherein the DNA-containing sample is a genomic DNA-containing sample; optionally selected from the group consisting of whole blood, plasma, serum, urine, saliva, sputum, nasal mucous, respiratory lavage, tears, chorionic villi, amniotic fluid, cerebrospinal fluid, spinal fluid, lavage fluid, stool, lymphatic fluid, bile, sweat, breast milk, breast fluid, cultured cells, tissue biopsy, tissue swabs, cervical cytology, synovial fluid, and combinations thereof.

5. The in vitro method according to any one of claims 1-4, wherein the method comprises determining the methylation status of at least 20, at least 30, at least 40, at least 50, at least 100, at least 150, at least 200, at least 250, at least 300, at least 350, at least 400, at least 450, or 498 CpG sites of Table 1.

6. The in vitro method according to any one of claims 1-5, wherein the CpG sites are in at least 2, at least 3, at least 4, at least 5, at least 6, at least 7, at least 8, at least 9, at least 10, at least 20, at least 30, at least 40, at least 50, at least 60, at least 70, at least 80, at least 90, at least 100, at least 100, at least 110, at least 120, at least 130, at least 140, at least 150, at least 160, at least 170, at least 180, at least 190, at least 200, at least 210, at least 220, at least 230, at least 240, at least 250, or 277 genes of Table 1.

7. An *in vitro* method for determining the biological age of a female subject, the method comprising the steps of determining the X chromosome reactivation level in a DNA-containing sample from the female subject with a method as defined in any one of claims 1-6 and comparing the determined X chromosome reactivation level with a reference value or a range of reference values.

8. An *in vitro* method for the diagnosis and/or prognosis of an age-related disease or disorder in a female subject, the method comprising the steps of determining the X chromosome reactivation level in a DNA-containing sample from the female subject with a method as defined in any one of claims 1-6 and comparing the determined X chromosome reactivation level with a reference value or a range of reference values; optionally, wherein the age-related disease or disorder is selected from the group consisting of cancer, neurodegenerative disease, sarcopenia, osteopenia, osteoporosis, arthritis, atherosclerosis, cardiovascular disease, hypertension, cataracts, presbyopia, glaucoma, type 2 diabetes, metabolic syndrome, chronic inflammation, immunosenescence, menopausal symptoms, and combinations thereof.

9. The in vitro method according to claim 8, wherein the cancer is breast cancer; particularly triple-negative breast cancer.

10. An *in vitro* method for the diagnosis and/or prognosis of an infection in a female subject, the method comprising the steps of determining the X chromosome reactivation level in a DNA-containing sample from the female subject with a method as defined in any one of claims 1-6 and comparing the determined X chromosome reactivation level with a reference value or a range of reference values.

11. An *in vitro* method for determining the gestational age of a fetus, the method comprising the steps of determining the X chromosome reactivation level in a DNA-containing sample from a female subject pregnant with the fetus with a method as defined in any one of claims 1-6 and comparing the determined X chromosome reactivation level with a reference value or a range of reference values.

12. Use of means for determining, in a DNA-containing sample from a female subject, the methylation status of at least 10 CpG sites of Table 1 in a method for determining the X chromosome reactivation level as defined in any one of claims 1-6; optionally, wherein the means are a methylation chip array.

13. Use of the methylation status of at least 10 CpG sites of Table 1 as a biomarker for:

   - determining the X chromosome reactivation level of a female subject;
   - determining the biological age of a female subject;
   - determining the gestational age of a fetus;
   - diagnosing and/or prognosing an age-related disease or disorder, or an infection, in a female subject;
   - deciding or recommending whether to initiate a therapeutic intervention in a female subject suspicious of suffering an age-related disease or disorder, or an infection;
   - determining the efficacy of a therapeutic intervention in a female subject already diagnosed of suffering an age-related disease or disorder, or an infection; and/or
   - determining the fertility status of a female subject.

14. A computer-implemented method comprising the steps of:

   (a) receiving information corresponding to the methylation status of at least 10 CpG sites of Table 1 in a DNA-containing sample from a female subject;
   (b) calculating the fraction of the CpG sites of step (a) that are hypomethylated, which corresponds to the X chromosome reactivation level of the female subject.

15. A method of identifying a candidate molecule that modulates the X chromosome reactivation level, the method comprising the steps of contacting the candidate molecule with a cell from a female subject and determining the X chromosome reactivation level with a method as defined in any one of claims 1-6.

A — Chr X methlylation in monocytes

CpGs in escapees
CpGs in inactives

Fraction of hypomethylated CpGs in inactivated genes

B

FIG. 1 (cont.)

FIG. 1

FIG. 2 (cont.)

F

FIG. 2

FIG. 3 (cont.)

FIG.3

FIG. 4

FIG. 5

FIG. 6 (cont.)

FIG. 6

A

| TCGA | beta 95% CI | |
|---|---|---|
| BRCA | 0.06 [ 0.04; 0.08] | |
| BLCA | 0.08 [ 0.03; 0.14] | |
| COAD | 0.08 [ 0.04; 0.11] | |
| HNSC | 0.03 [-0.01; 0.08] | |
| KIRC | 0.03 [ 0.01; 0.05] | |
| LIHC | 0.14 [ 0.08; 0.20] | |
| LUAD | -0.01 [-0.04; 0.02] | |
| LUSC | 0.06 [ 0.02; 0.10] | |
| PAAD | -0.02 [-0.10; 0.06] | |
| READ | 0.08 [ 0.00; 0.16] | |
| THCA | 0.07 [ 0.06; 0.09] | |
| UCEC | 0.08 [ 0.04; 0.12] | |
| Total (common effect) | 0.05 [ 0.05; 0.06] | |
| Total (random effect) | 0.06 [ 0.03; 0.08] | |

Effect of cancer on X-Ra

Heterogeneity: $\chi^2_{11} = 43.58$ ($P < .001$), $I^2 = 75\%$

B    ROC

FIG. 7 (cont.)

C

D

FIG. 7

FIG. 8

Europäisches
Patentamt

European
Patent Office

Office européen
des brevets

# EUROPEAN SEARCH REPORT

Application Number

EP 23 38 2740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KANG JUN ET AL: "Cancer-Testis Antigen Expression in Serous Endometrial Cancer with Loss of X Chromosome Inactivation", PLOS ONE, vol. 10, no. 9, 11 September 2015 (2015-09-11), page e0137476, XP093113524, US ISSN: 1932-6203, DOI: 10.1371/journal.pone.0137476 * cf abstract; "Materials and Methods" section from p. 2; "Clustering of X chromosome methylation" section on p. 4, first par.; fig. 1, 5 * | 1-15 | INV. C12Q1/6883 |
| X | SUN ZHIFU ET AL: "Chromosome X genomic and epigenomic aberrations and clinical implications in breast cancer by base resolution profiling", EPIGENOMICS, vol. 7, no. 7, 18 May 2015 (2015-05-18), pages 1099-1110, XP093113534, United Kingdom ISSN: 1750-1911, DOI: 10.2217/epi.15.43 * cf abstract; fig. 4, 5; section "Breast cancer data from public database" on p. 1100; section "Methylation pattern changes in solid breast cancer & normal tissues" from p. 1104; "TCGA paired tumor & normal samples with both methylation & mRNA-seq data" section from p. 1105 * | 1-15 | |

TECHNICAL FIELDS SEARCHED (IPC)

C12Q

-/--

The present search report has been drawn up for all claims

| Place of search | Date of completion of the search | Examiner |
|---|---|---|
| Munich | 8 January 2024 | Fischer, V |

EPO FORM 1503 03.82 (P04C01)

Europäisches
Patentamt

European
Patent Office

Office européen
des brevets

# EUROPEAN SEARCH REPORT

**Application Number**

EP 23 38 2740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TANG WALFRED W C ET AL: "A Unique Gene Regulatory Network Resets the Human Germline Epigenome for Development", CELL, ELSEVIER, AMSTERDAM NL, vol. 161, no. 6, 4 June 2015 (2015-06-04), pages 1453-1467, XP029171297, ISSN: 0092-8674, DOI: 10.1016/J.CELL.2015.04.053 * cf fig. 5B; p. 1461, first column, third par.; p.1466, second column, second par. * | 1-15 | |
| X | KANANEN LAURA ET AL: "Ageing-associated changes in DNA methylation in X and Y chromosomes", EPIGENETICS & CHROMATIN, vol. 14, no. 1, 2 July 2021 (2021-07-02), XP093113393, London, UK ISSN: 1756-8935, DOI: 10.1186/s13072-021-00407-6 Retrieved from the Internet: URL:https://link.springer.com/article/10.1 186/s13072-021-00407-6/fulltext.html> * cf abstract; fig. 1; par. bridging p. 7-8 * | 7,10,11, 15 | |

**TECHNICAL FIELDS SEARCHED (IPC)**

-/--

The present search report has been drawn up for all claims

| Place of search | Date of completion of the search | Examiner |
|---|---|---|
| Munich | 8 January 2024 | Fischer, V |

EPO FORM 1503 03.82 (P04C01)

Europäisches
Patentamt

European
Patent Office

Office européen
des brevets

# EUROPEAN SEARCH REPORT

Application Number

**EP 23 38 2740**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI SHUXIA ET AL: "Exploratory analysis of age and sex dependent DNA methylation patterns on the X-chromosome in whole blood samples", GENOME MEDICINE, vol. 12, no. 1, 28 April 2020 (2020-04-28), XP093113332, ISSN: 1756-994X, DOI: 10.1186/s13073-020-00736-3 Retrieved from the Internet: URL:https://link.springer.com/article/10.1 186/s13073-020-00736-3/fulltext.html> * cf abstract; "DNA methylation data" section from p. 2; fig. 1; par. bridging p. 8-9, second column; p.9, par. bridging first and second column * | 7,10,11, 15 | |
| X | MCCARTNEY DANIEL L. ET AL: "An epigenome-wide association study of sex-specific chronological ageing", GENOME MEDICINE, vol. 12, no. 1, 31 December 2019 (2019-12-31), XP093113421, ISSN: 1756-994X, DOI: 10.1186/s13073-019-0693-z Retrieved from the Internet: URL:http://link.springer.com/article/10.11 86/s13073-019-0693-z/fulltext.html> * cf abstract; "Methods" section from p.2; par. bridging first and second column on p. 4 and p. 4, second column, second par; fig. 2 * | 7,10,11, 15 | **TECHNICAL FIELDS SEARCHED (IPC)** |

-/--

The present search report has been drawn up for all claims

| Place of search | Date of completion of the search | Examiner |
|---|---|---|
| Munich | 8 January 2024 | Fischer, V |

EPO FORM 1503 03.82 (P04C01)

| | Europäisches Patentamt European Patent Office Office européen des brevets | **EUROPEAN SEARCH REPORT** | Application Number EP 23 38 2740 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RONAN CHALIGN? ET AL: "X-chromosome inactivation in development and cancer", FEBS LETTERS, ELSEVIER, AMSTERDAM, NL, vol. 588, no. 15, 14 June 2014 (2014-06-14), pages 2514-2522, XP071254249, ISSN: 0014-5793, DOI: 10.1016/J.FEBSLET.2014.06.023 * cf abstract * | 1-15 | |
| A | WO 2018/072705 A1 (UNIV HONG KONG CHINESE [CN]) 26 April 2018 (2018-04-26) * cf par. 94, 100, 107-;fig. 4A, 4B, 7, 8 * | 1-15 | |

| | | | TECHNICAL FIELDS SEARCHED (IPC) |

The present search report has been drawn up for all claims

| Place of search | Date of completion of the search | Examiner |
|---|---|---|
| Munich | 8 January 2024 | Fischer, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2740

This annex lists the patent family members relating to the patent documents cited in  the above-mentioned European search report.
The members are as contained in the European Patent Office EDP file on
The European Patent Office is in no way liable for these particulars which are merely given for the purpose of information.

08-01-2024

| Patent document cited in search report | Publication date | Patent family member(s) | | Publication date |
|---|---|---|---|---|
| WO 2018072705 A1 | 26-04-2018 | CN | 109890984 A | 14-06-2019 |
| | | EP | 3529377 A1 | 28-08-2019 |
| | | EP | 4209598 A1 | 12-07-2023 |
| | | IL | 265769 A | 30-06-2019 |
| | | SG | 11201903346Q A | 30-05-2019 |
| | | US | 2018105807 A1 | 19-04-2018 |
| | | US | 2023047963 A1 | 16-02-2023 |
| | | WO | 2018072705 A1 | 26-04-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MORRIS TJ et al.** Analysis pipelines and packages for Infinium HumanMethylation450 BeadChip (450k) data. *Methods*, 15 January 2015, vol. 72, 3-8
- **BURTIS C. A et al.** Statistical Treatment of Reference Values. 2008
- **CARRERAS-GALLO, A et al.** The early-life exposome modulates the effect of polymorphic inversions on DNA methylation. *Commun. Biol*, 2022, vol. 51 (5), 1-13
- **M. E. LEVINE et al.** An epigenetic biomarker of aging for lifespan and healthspan. *Aging (Albany. NY)*, 2018, vol. 10
- **N. CARRERAS-GALLO et al.** The early-life exposome modulates the effect of polymorphic inversions on DNA methylation. *Commun. Biol*, 2022, vol. 51 (5), 1-13
- **BURTIS C. A et al.** *Statistical Treatment of Reference Values*, 2008